# EUROPEAN PATENT APPLICATION

(11) **EP 4 653 531 A1**
(43) Date of publication of application: **26.11.2025**
(21) Application number: 24744939.0
(22) Date of filing: 19.01.2024
(51) Int. Cl.: C12N 15/113, C07K 14/71

(54) **ANTISENSE OLIGOMERS TARGETING GFRAL AND USE THEREOF**

(30) Priority: 20.01.2023 KR 20230008908
(71) Applicant: Korea Advanced Institute of Science and Technology, Daejeon 34141 (KR); Thor Therapeutics Inc., Daejeon 34134 (KR); The Industry & Academic Cooperation in Chungnam National University (IAC), Daejeon 34134 (KR)
(72) Inventor: KIM, Jinkuk, Daejeon 34141 (KR); PARK, Minsung, Daejeon 34141 (KR); SHONG, Minho, Daejeon 34963 (KR); CHANG, Joon Young, Daejeon 35055 (KR); JU, Sang-Hyeon, Daejeon 35326 (KR); HONG, Hyunjung, Daejeon 34660 (KR); YI, Hyon-Seung, Daejeon 34962 (KR); LEE, Min Hee, Daejeon 34080 (KR)
(74) Representative: Mewburn Ellis LLP
(86) International application number: PCT/KR2024/000999
(87) International publication number: WO 2024/155160

(57) **Abstract**

The present invention relates to: GFRAL-specific antisense oligomers targeting the GDNF family receptor alpha like (GFRAL) gene; and a pharmaceutical composition for preventing or treating obesity, diabetes, appetite loss, or cachexia, the pharmaceutical composition including the antisense oligomers. The antisense oligomers according to the present invention were found to effectively inhibit GFRAL expression in the body and exhibit amelioration effects in cancer cachexia animal models. Thus, the application of the antisense oligomers as an agent for treating obesity or cachexia through GFRAL expression inhibition is highly promising.

## Description

### TECHNICAL FIELD

The present invention relates to a GFRAL-specific antisense oligomer targeting the GFRAL (GDNF family receptor alpha like) gene and the use thereof, and more specifically, to a GFRAL-specific antisense oligomer comprising a specific nucleotide sequence and a pharmaceutical composition for preventing or treating a disease associated with the activation of GFRAL due to an increase in GDF15 comprising the same.

### BACKGROUND ART

Cachexia is an irreversible disease condition characterized by muscle loss due to complex metabolic abnormalities, often accompanied by a decrease in adipose tissue, and not recoverable even with adequate calorie supplementation. Cachexia is accompanied by loss of appetite, weight loss, loss of adipose tissue and muscle, and depression, and causes low quality of life and decreased life expectancy. Cachexia is commonly caused by chronic diseases (such as chronic respiratory disease, heart failure, renal failure, and chronic infection) and cancer. Cachexia caused by chronic diseases including cancer is not treated by administration of appetite-stimulating drugs, exercise, or anti-inflammatory drugs other than correction of the cause (Baracos et al., Nat Rev Dis Primers, Jan 18;4:17105, 2018). Therefore, cachexia remains as an unmet medical need because there is no therapeutic agent for suppressing progression and facilitating recovery from cachexia.

Cachexia, which occurs especially in cancer patients, may occur in all types of cancer, including lung cancer, pancreatic cancer, and gastroesophageal cancer (Baracos et al., Nat Rev Dis Primers, Jan 18;4:17105, 2018). Cachexia occurs in more than 50% of cancer patients, and cachexia is the direct cause of death in 20% of cancer patients. The diagnostic criteria for cachexia in cancer patients are defined as a weight loss of 5% or more in 6 months, or a weight loss of 2% or more in patients with a BMI of less than 20 kg/m², or a weight loss of 2% or more accompanied by sarcopenia (Feaonetal et al., Lancet Oncol, May;12(5) :489-95, 2011).

GDF15 (growth differentiating factor-15) is increased in the blood of patients with chronic diseases (chronic respiratory disease, heart failure, renal failure, chronic infection, etc.) and cancer accompanied by cachexia, and is involved in the development of cachexia. In normal people, GDF15 has low serum concentrations (0.2 - 1.2 ng/dl), but in patients with chronic diseases and cancer, it increases by more than 10 to 1,000 fold, causing cachexia findings such as appetite suppression, nausea, vomiting, and fat and muscle loss. Therefore, serum GDF15 has diagnostic utility because it increases with the severity of the disease causing cachexia (Breit et al., Annu Rev Physiol. 2021 Feb 10;83:127-151). Since GDF15, which is increased in cancer, is directly related to the development of cancer cachexia, neutralizing antibodies that inhibit GDF15 activity have an effect of ameliorating cachexia by reducing weight gain and loss of muscle and fat tissue in a mouse model of cancer cachexia (Lerner et al., Journal of cachexia, sarcopenia and muscle 2016; 7: 467-482).

The receptor for GDF15 is GFRAL (GDNF family receptor alpha like), which is specifically expressed in the AP (area postrema) and NTS (nucleus tractus solitarius) regions of the hindbrain, and activation of the receptor for GFRAL by GDF15 induces cancer cachexia (Mullican et al., Nature 2017, Hsu et. al., Nature 2017). After GDF15 binds to GFRAL, it activates GFRAL neurons through Ret signaling, resulting in appetite suppression and weight loss (Goldman A et al., Nature 2017).

Research on the signaling process of the GDF15/GFRAL/Ret complex is attracting attention in order to ameliorate and suppress the symptoms of cancer cachexia. GDF15 dimer binds to domain 2 of GFRAL and activates cell signaling through the CLD1, CLD2, and CLD3 binding surfaces of Ret. Ret is known to activate the downstream signaling PI3K/AKT, PLC/PKC, and MEK/ERK by phosphorylation (Mullican et al., Trends Endocrinol Metab. 2018 Aug;29(8):560-570).

Effective therapies for correcting the cause of cachexia in cancer patients have not yet been developed and thus the development of effective therapies is urgently needed. Accordingly, the present inventors have elucidated the roles and effects of antisense oligonucleotides on GFRAL in the hindbrain, thereby completing the present invention.

The above information disclosed in this Background section is provided only for enhancement of understanding of the background of the invention, and therefore it may include information that does not form the prior art that is already known in this country to a person of ordinary skill in the art.

### SUMMARY OF THE INVENTION

It is one object of the present invention to provide an oligomer that inhibits the expression of the human GFRAL (GDNF family receptor alpha like) gene.

It is another object of the present invention to provide a pharmaceutical composition for preventing or treating a disease related to activation of GFRAL due to an increase in GDF15 comprising the oligomer, and a method of preventing or treating the disease.

It is still another object of the present invention to provide the use of the oligomer for the prevention or treatment of a disease related to activation of GFRAL due to an increase in GDF15 and the use of the oligomer for the manufacture of a medicament for preventing or treating a disease related to activation of GFRAL due to an increase in GDF15.

In order to accomplish the above objects, the present invention provides an oligomer capable of hybridizing with at least 13 consecutive nucleobases in an whole pre-mRNA of human GFRAL (GDNF family receptor alpha like) gene represented by SEQ ID NO: 1 through Watson-Crick base pairing A:T or G:C or wobble base pairing G:U, I:A, I:C or I:U and having a length of 13 to 35 nt.

The present invention also provides a pharmaceutical composition for preventing or treating a disease related to activation of GFRAL due to an increase in GDF15 comprising the oligomer.

The present invention also provides a method of preventing or treating a disease associated with the activation of GFRAL due to an increase in GDF15 using the oligomer.

The present invention also provides the use of the oligomer for the prevention or treatment of a disease related to activation of GFRAL due to an increase in GDF15 and the use of the oligomer for the manufacture of a medicament for preventing or treating a disease related to activation of GFRAL due to an increase in GDF15.

### BRIEF DESCRIPTION OF THE DRAWINGS

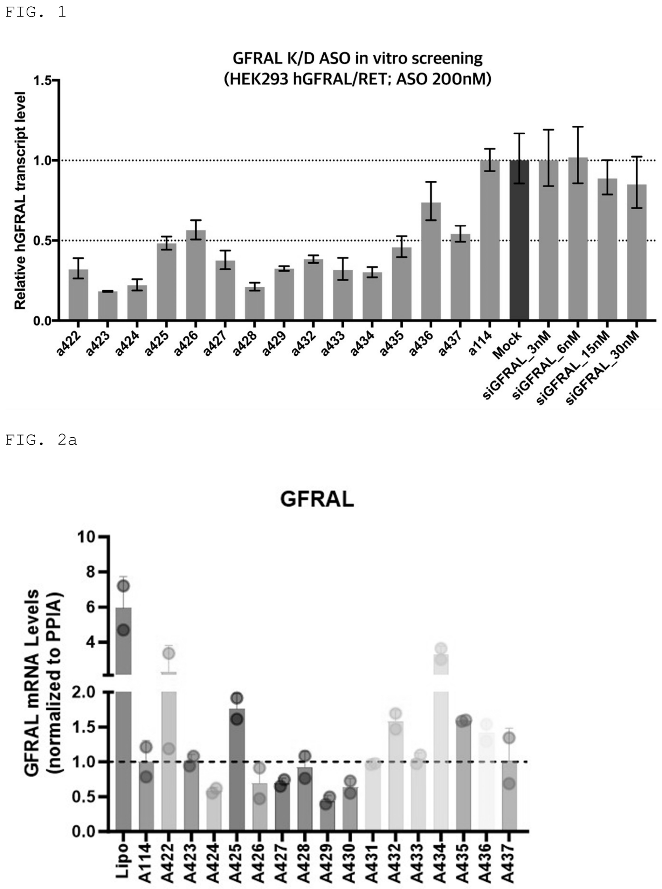

FIG. 1 is a graph showing the results of a test to determine the possibility of suppressing GFRAL mRNA expression levels when HEK293 GFRAL and RET cDNA overexpressing cells were transfected with antisense oligonucleotide candidates at a concentration of 200 nM according to the present invention for 24 hours.

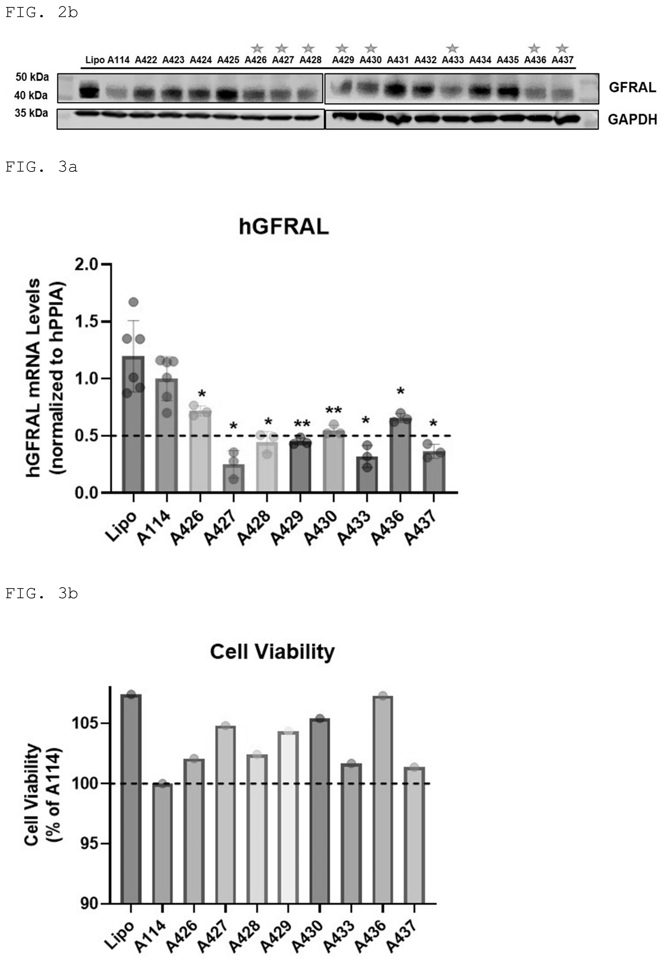

FIG. 2a is a graph showing the results of a test to determine the possibility of suppressing GFRAL mRNA expression level when HEK293 GFRAL and RET cDNA overexpressing cells were transfected with 16 antisense oligonucleotide candidates according to the present invention at a concentration of 100 nM for 24 hours. FIG. 2b is a graph showing the results of a test to determine the possibility of suppressing GFRAL protein expression level when HEK293 GFRAL and RET cDNA overexpressing cells were transfected with 16 antisense oligonucleotide candidates according to the present invention for 48 hours.
FIG. 3a is a graph showing the results of a test to determine the possibility of suppressing GFRAL mRNA expression when HEK293 GFRAL and RET cDNA overexpressing cells were transfected with 8 antisense oligonucleotide candidates at a concentration of 50 nM for 24 hours. FIG. 3b is a graph showing the results of a test to determine cytotoxicity when HEK293 GFRAL and RET cDNA overexpressing cells were transfected at a concentration of 50 nM with 8 antisense oligonucleotide candidates for 48 hours.

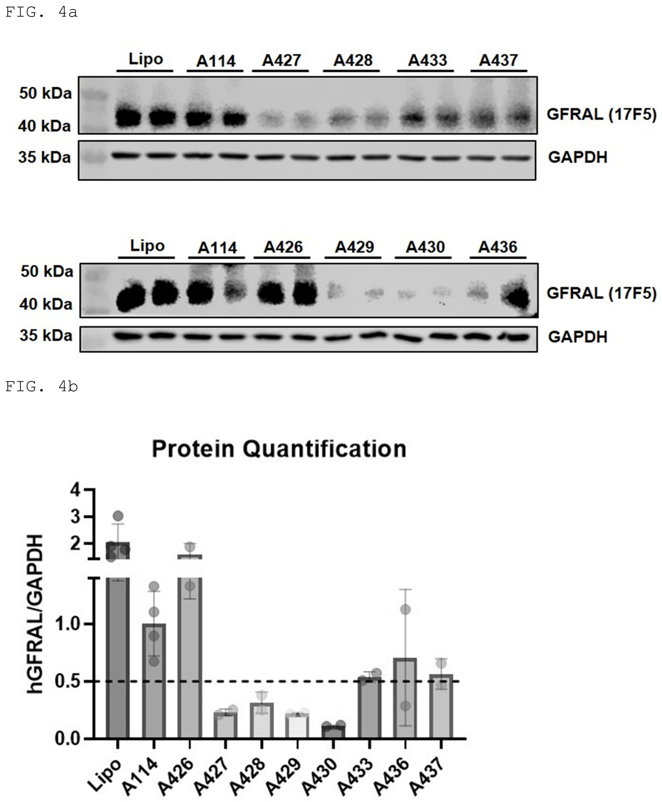

FIG. 4a shows the results of a test to determine the possibility of suppressing GFRAL protein expression level when HEK293 GFRAL and RET cDNA overexpressing cells were transfected with antisense oligonucleotide candidates according to the present invention at a concentration of 50 nM for 48 hours. FIG. 4b is a graph showing the quantified GFRAL protein expression level of FIG. 4a.

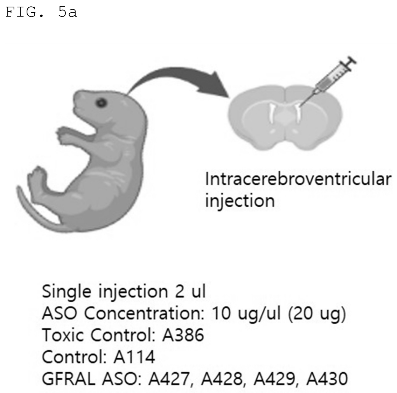

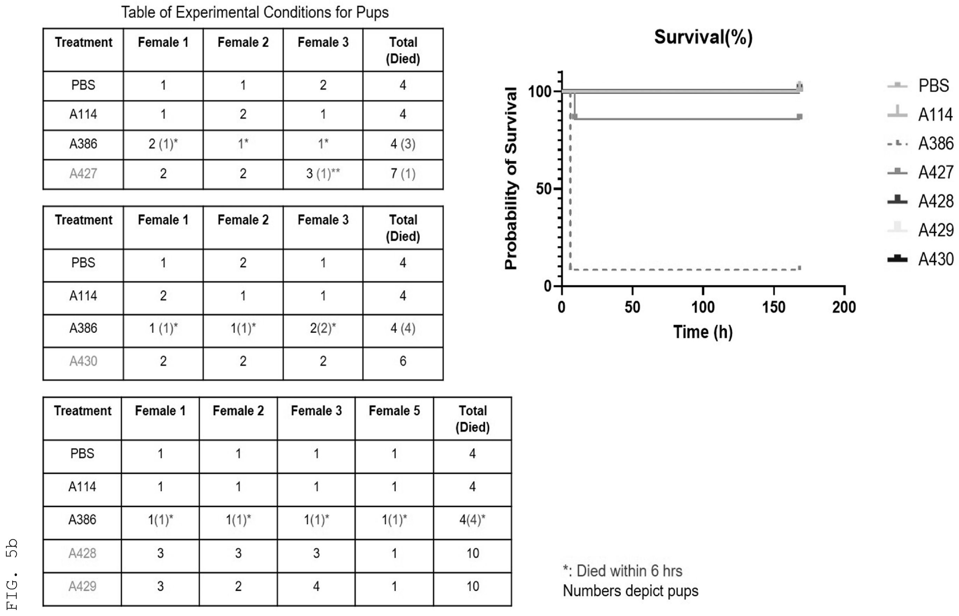

FIG. 5a is a schematic diagram illustrating the in vivo toxicity test of antisense oligonucleotide candidates according to the present invention. FIG. 5b shows the survival rate up to 7 days after administration of the toxicity negative control group, the toxicity positive control group, and four antisense oligonucleotide candidates to experimental animals.

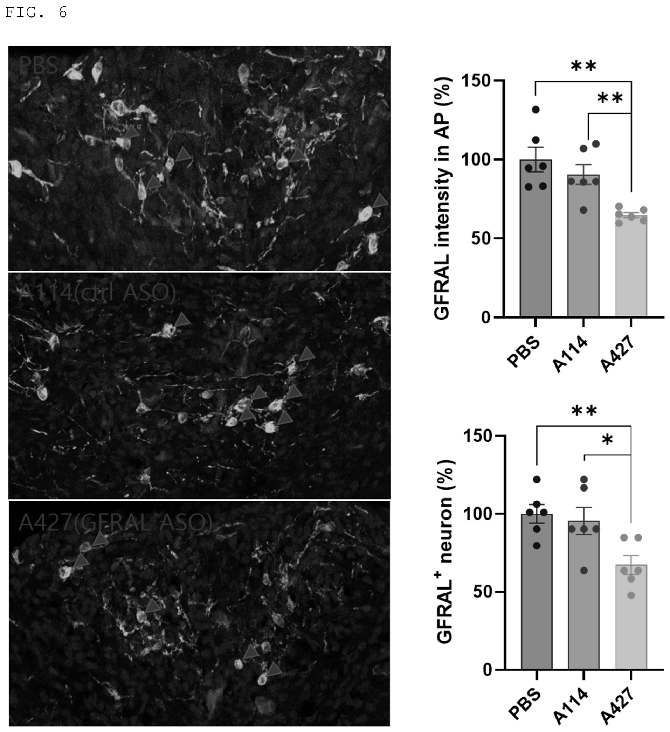

FIG. 6 shows the GFRAL protein expression determined by staining using purified antibodies 1 week after in vivo administration of the antisense oligonucleotide candidates according to the present invention. The arrows in the figure for each administration group indicate GFRAL positive neurons. The fluorescence intensity of the area postrema (AP) and the number of GFRAL positive neurons in the AP were measured and graphed.

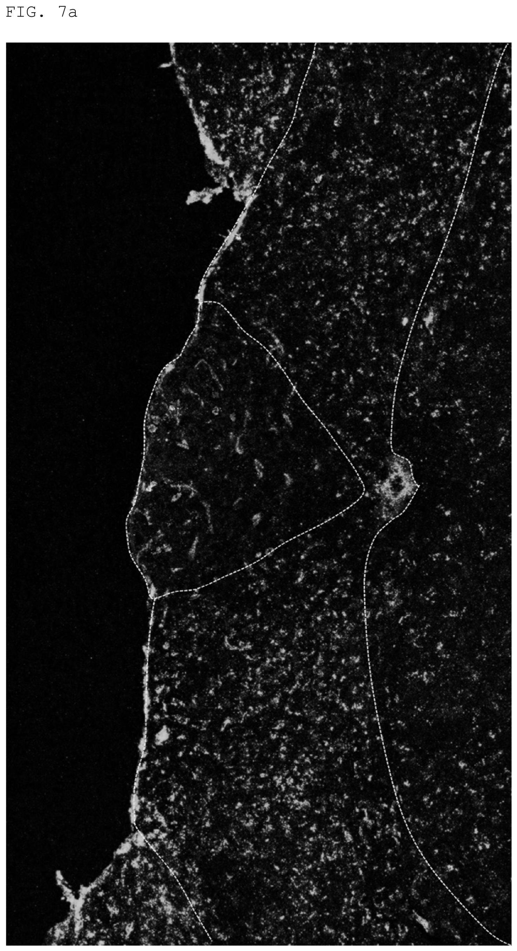

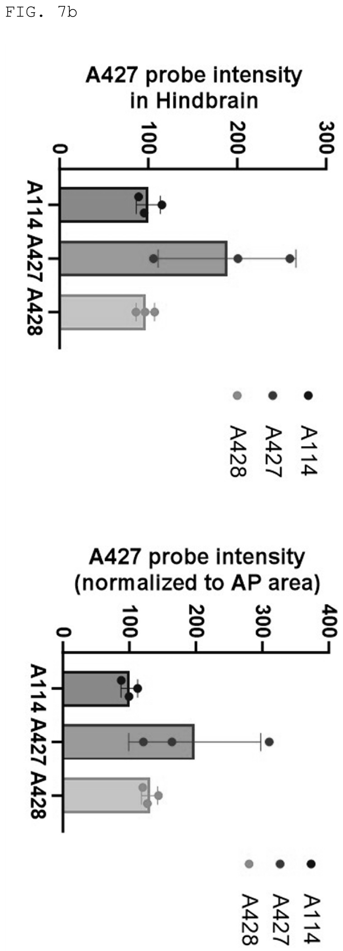

FIG. 7a shows the ASO distribution in the brain measured using A427 probe staining 2 weeks after in vivo administration of the antisense oligonucleotide candidates according to the present invention. FIG. 7b is a graph showing quantified ASO distribution in the brain.

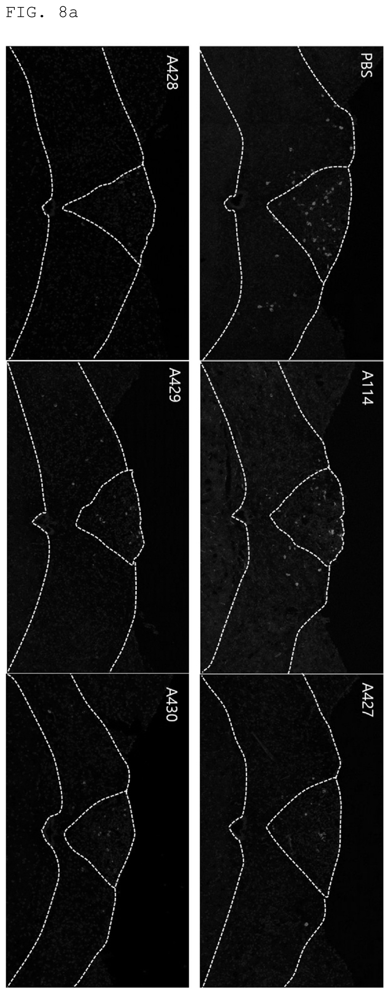

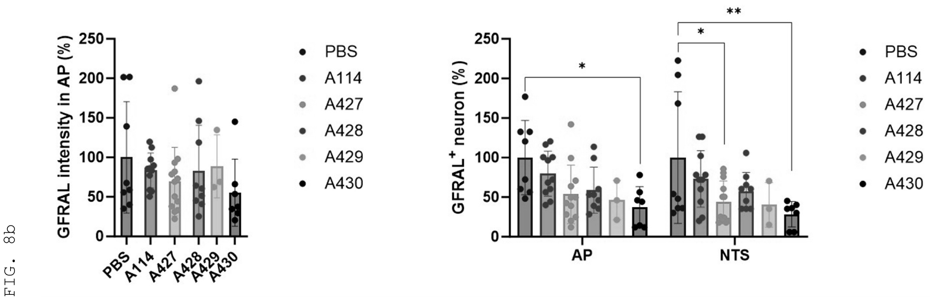

FIG. 8a shows the GFRAL gene expression measured using staining after 2 weeks of in vivo administration of antisense oligonucleotide candidates according to the present invention. FIG. 8b shows the fluorescence intensity measured in the area postrema (AP) and the number of GFRAL positive neurons in the area postrema (AP) and nucleus tractus solitarius (NTS).

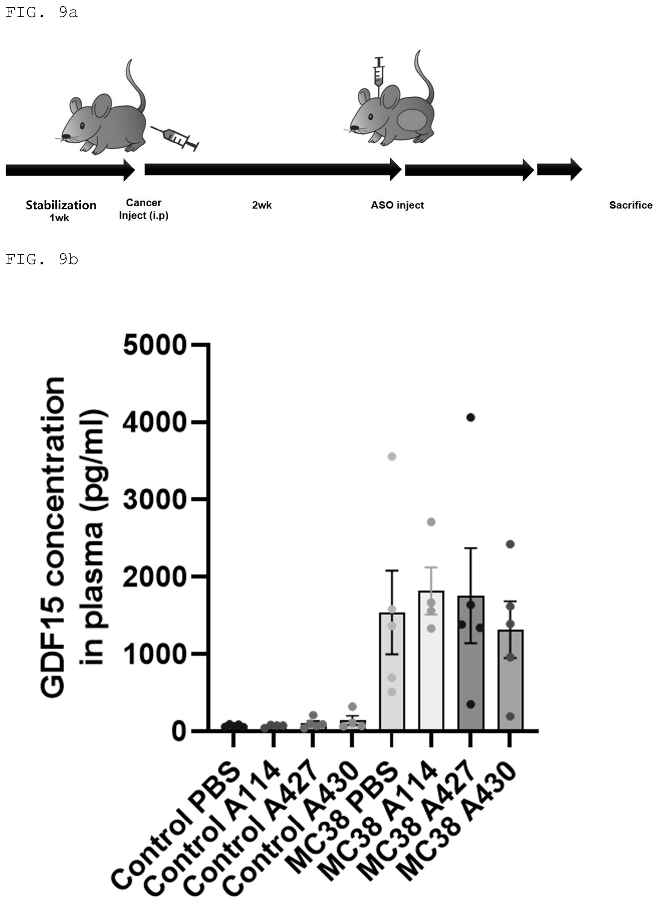

FIG. 9a is a schematic diagram illustrating an animal experiment schedule to verify the cancer cachexia treatment effect of antisense oligonucleotides according to the present invention, including the experimental protocol for constructing a cancer cachexia animal model and administering antisense oligonucleotides thereto. FIG. 9b is a graph showing the measured concentration of GDF15 in serum isolated from the experimental animal.

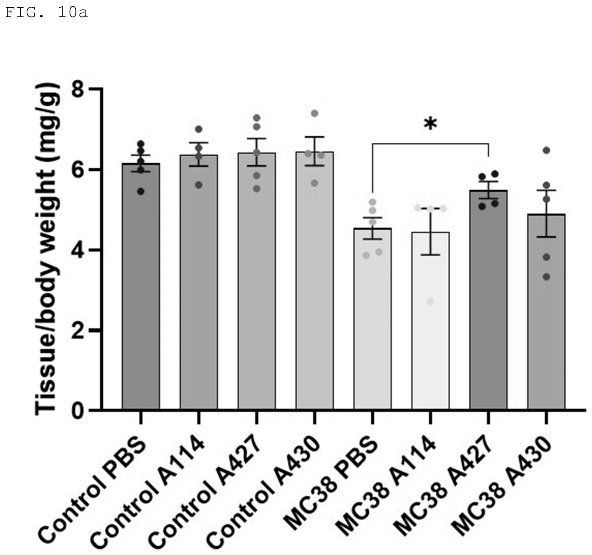

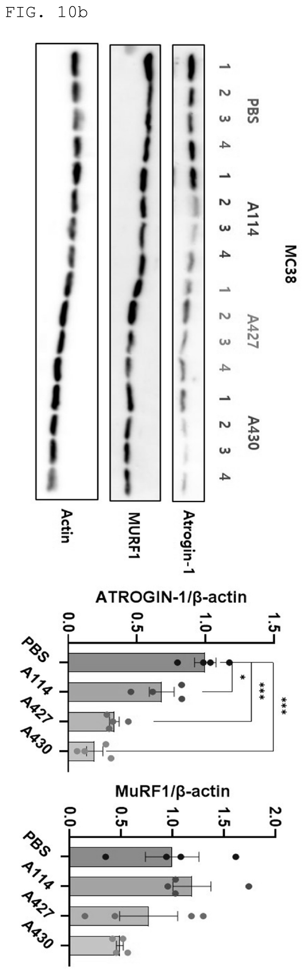

FIG. 10a shows the weight of muscle tissue measured 1 week after administration of antisense oligonucleotides to the experiment animal model. FIG. 10b shows the protein expressions of Atrogin-1 and MuRF1 in the tissues determined by western blot and is a graph showing a quantified weight.

### DETAILED DESCRIPTION OF THE INVENTION AND PREFERRED EMBODIMENTS OF THE INVENTION

Unless defined otherwise, all technical and scientific terms used herein have the same meanings as those appreciated by those skilled in the field to which the present invention pertains. In general, the nomenclature used herein is well-known in the art and is ordinarily used.

As used herein, the terms "GFRAL", "GDNF family receptor alpha like", "GDNF family receptor alpha type", "growth differentiation factor 15 receptor" or "GFRAL protein" and similar terms, unless otherwise stated, refer to a polypeptide ("polypeptide" is used interchangeably with "protein" herein) from a vertebrate source, including mammals such as primates (e.g., humans, cynomolgus monkeys (cyno)), dogs and rodents (e.g., mice and rats), or any native GFRAL, and in certain embodiments, the terms include related GFRAL polypeptides, including SNP variants thereof. GFRAL is also known in the art as "C6orf144", "Chromosome 6 Open reading Frame 144", "BA360D14.1", "IVFI9356", and "UNQ9356".

Anti-sense oligonucleotide (ASO) controls the transfer of information from genes to proteins by changing the intermediate metabolism of mRNA through single-stranded RNA or DNA. In other words, a nucleotide sequence that is sufficiently complementary and hybridizes specifically is selected so that the desired expression of the target protein is suppressed. Since the binding of ASO is sequence-specific to the target gene, it does not affect the expression of other genes other than the target gene. Therefore, ASO is not only a useful tool for analyzing the role of a specific protein in vivo, but also has the potential to be used as a gene therapy for specific diseases (FASEBJ. 9, 1288-1296, 1995).

Antisense DNA binds to the target mRNA to form an RNA/DNA double helix, and this RNA/DNA double helix structure is degraded when attacked by RNase H (RNase H; a type of ribonuclease that specifically degrades the mRNA in which a RNA/DNA hybrid double helix is formed) present in vivo. Antisense RNA forms an RNA/RNA double helix and the target mRNA is degraded when attacked by RNase L. RNase L is a ribonuclease that preferentially degrades single-stranded RNA around the double-stranded RNA strand (Pharmacol. Toxicol. 32, 329-376, 1992).

In one aspect, the present invention is directed to an oligomer capable of hybridizing with at least 13 consecutive nucleobases in an whole pre-mRNA of human GFRAL (GDNF family receptor alpha like) gene represented by SEQ ID NO: 1 through Watson-Crick base pairing A:T or G:C, or wobble base pairing G:U, I:A, I:C or I:U and having a length of 13 to 35 nt.

The antisense oligomer (antisense oligonucleotide) according to the present invention inhibits the expression of a gene encoding human GFRAL, specifically mRNA (pre-mRNA or mature mRNA). The antisense oligomer (antisense oligonucleotide) according to the present invention comprises a sequence complementary to an mRNA encoding human GFRAL.

The term "antisense activity" means any detectable or measurable activity that contributes to the hybridization of an antisense compound to the target nucleic acid thereof. In certain embodiments, the antisense activity exhibits a decrease in the amount or expression of a target nucleic acid or a protein encoded by such target nucleic acid. The antisense activity according to the present invention acts on a target nucleic acid encoding GFRAL to reduce the amount or expression of the encoded GFRAL protein.

The terms "targeting" and "targeted" specifically mean hybridization to a target nucleic acid to induce a desired effect.

The terms "target nucleic acid", "target RNA" and "target RNA transcript" all refer to a nucleic acid that can be targeted by an antisense oligomer.

The present invention provides an oligomer capable of performing hybridization to a target nucleic acid through hydrogen bonding.

The term "inhibition" means a decrease in the level of a target nucleic acid or a target protein in the presence of an antisense compound complementary to the target nucleic acid compared to the level of the target nucleic acid or the level of the target protein in the absence of the antisense oligomer.

The term "anti-sense oligomer" includes a single-stranded oligonucleotide having a nucleotide sequence that enables hybridization to a corresponding portion or segment of a target nucleic acid.

In the present invention, the oligomer may be capable of hybridizing to at least 13 consecutive nucleotides in the whole pre-mRNA of the human GFRAL gene and have a length of 13 to 35 nt.

The whole pre-mRNA of the human GFRAL gene may be a nucleic acid sequence represented by SEQ ID NO: 1, wherein the SEQ ID NO: 1 is GenBank Homo sapiens chromosome 6, GRCh38.p14 Primary Assembly NC_000006.12 REGION: 55327469..55402493.

The oligomer according to the present invention is capable of hybridizing with at least 9, at least 10, at least 11, at least 12, at least 13, at least 14, at least 15, at least 16, at least 17, or at least 18 consecutive nucleobases of the nucleic acid sequence.

The conditions used to achieve a stringent specific level in hybridization vary depending on the nature of the nucleic acid to be hybridized. For example, the length of the nucleic acid portion to be hybridized, the degree of homology, the nucleotide sequence composition (e.g., GC/AT composition ratio), and the nucleic acid type (e.g., RNA or DNA) are considered in selecting the hybridization conditions. An additional consideration is whether or not the nucleic acid is immobilized, for example, on a filter.

Examples of very stringent conditions include: 2X SSC/0.1% SDS at room temperature (hybridization conditions); 0.2X SSC/0.1% SDS at room temperature (low stringent conditions); 0.2X SSC/0.1% SDS at 42°C (moderate stringent conditions); and 0.1X SSC at 68°C (high stringent conditions). The washing process may be performed using one of these conditions, for example, the high stringent conditions, or each of the conditions, and all or part of the conditions described above may be repeated in the order described above for 10 to 15 minutes each. However, as described above, optimal conditions vary depending on the particular hybridization reaction involved and may be determined experimentally. In general, high stringent conditions are used for hybridization of important probes.

At least one of the base pairs forming the hybridization may include a wobble pair G:U, I:A, I:C or I:U.

In the present invention, the oligomer may be capable of hybridizing with at least 13 consecutive nucleobases comprised in any one nucleic acid sequence selected from the group consisting of SEQ ID NO: 3 to SEQ ID NO: 18, and have a length of 13 to 35 nt.

In one embodiment of the present invention, the oligomer may be an antisense oligomer comprising 15 to 25 linked nucleosides, may be capable of hybridizing with at least 9, at least 10, at least 11, at least 12, at least 13, at least 14, at least 15, at least 16, at least 17, or at least 18 consecutive nucleobases selected from the group consisting of SEQ ID NO: 3 to SEQ ID NO: 18, and may have a length of 13 to 35 nt.

Specifically, the oligomer may comprise an oligomer capable of hybridizing with at least 19 or 20 consecutive nucleobases selected from the group consisting of SEQ ID NO: 3 to SEQ ID NO: 18, and having a length of 13 to 35 nt.

In the present invention, the oligomer may be capable of hybridizing with at least 13 consecutive nucleotide bases in one nucleic acid sequence selected from the group consisting of SEQ ID NO: 8 to SEQ ID NO: 11, SEQ ID NO: 14, SEQ ID NO: 17, and SEQ ID NO: 18, and have a length of 13 to 35 nt.

In the present invention, the oligomer may comprise an oligomer capable of hybridizing with at least 13, at least 14, at least 15, at least 16, at least 17, or at least 18 consecutive nucleotide bases among nucleic acid sequences selected from the group consisting of SEQ ID NO: 8 to SEQ ID NO: 11, and having a length of 13 to 35 nt.

In the present invention, the oligomer may have a length of 13 to 35 nt, or a length of 16 to 25 nt, but is not limited thereto. Specifically, the oligomer may have a length of 16 nt, 17 nt, 18 nt, 19 nt, 20 nt, 21 nt, 22 nt, 23 nt, 24 nt or 25 nt.

At least one of the base pairs forming the hybridization may include a wobble pair G:U, I:A, I:C or I:U.

In the present invention, the oligomer may be capable of hybridizing with at least 13, at least 14, at least 15, at least 16, at least 17 or at least 18 consecutive nucleobases in the nucleic acid sequence of SEQ ID NO: 8 or SEQ ID NO: 11 and have a length of 13 to 35 nt, and at least one of the base pairs forming the hybridization may include a wobble pair G:U, I:A, I:C or I:U.

The term "consecutive nucleobases" means nucleobases that are immediately adjacent to each other. "Internucleoside linkage" refers to a chemical bond between nucleosides. The term "linked nucleosides" refers to adjacent nucleosides that are linked together.

The term "nucleic acid" refers to a molecule comprising monomeric nucleotides. Nucleic acids include ribonucleic acids (RNA), deoxyribonucleic acids (DNA), single-stranded nucleic acids, double-stranded nucleic acids, small interfering ribonucleic acids (siRNA), and microRNAs (miRNA). Nucleic acids may also include combinations of these elements within a single molecule.

The term "nucleobase" means a heterocyclic moiety capable of pairing with a base of another nucleic acid.

The term "nucleobase sequence" means a sequence of consecutive nucleobases independent of any sugar, linkage, or nucleobase modification.

The term "nucleoside" means a nucleobase linked to a sugar and the term "nucleotide" means a nucleoside having a phosphate group covalently linked to the sugar moiety of the nucleoside.

The term "oligomer" means a polymer of linked monomer subunits capable of hybridizing to a region of a nucleic acid molecule.

The term "oligonucleotide" means a polymer of linked nucleosides, each of which may be modified or unmodified independently of the others.

The term "single-stranded oligonucleotide" means an oligonucleotide that does not hybridize to a complementary strand.

At least one of the base pairs forming the hybridization may include a wobble pair G:U, I:A, I:C or I:U. The antisense oligomer according to the present invention may, for example, comprise a sequence having at least 90% sequence homology with a sequence selected from the group consisting of SEQ ID NO: 19 to SEQ ID NO: 34.

Specifically, the antisense oligomer may comprise a sequence having at least 91%, at least 92%, at least 93%, at least 94%, at least 95%, at least 96%, at least 97%, at least 98%, at least 99%, or 100% sequence homology, with a sequence selected from the group consisting of SEQ ID NO: 19 to SEQ ID NO: 34.

The antisense oligomer according to the present invention may, for example, comprise a sequence having at least 90% sequence homology with at least one sequence selected from the group consisting of SEQ ID NO: 24 to SEQ ID NO: 27, SEQ ID NO: 30, SEQ ID NO: 33, and SEQ ID NO: 34.

Specifically, the antisense oligomer may comprise a sequence having at least 91%, at least 92%, at least 93%, at least 94%, at least 95%, at least 96%, at least 97%, at least 98%, at least 99%, or 100% sequence homology, with at least one sequence selected from the group consisting of SEQ ID NO: 24 to SEQ ID NO: 27, SEQ ID NO: 30, SEQ ID NO: 33 and SEQ ID NO: 34. The antisense oligomer according to the present invention may comprise one of sequences selected from the group consisting of SEQ ID NO: 24 to SEQ ID NO: 27, SEQ ID NO: 30, SEQ ID NO: 33 and SEQ ID NO: 34.

The antisense oligomer according to the present invention may comprise, for example, a sequence having at least 90% sequence homology with a sequence of SEQ ID NO: 24 or SEQ ID NO: 27.

Specifically, the antisense oligomer may comprise a sequence having at least 91%, at least 92%, at least 93%, at least 94%, at least 95%, at least 96%, at least 97%, at least 98%, at least 99%, or 100% sequence homology with a sequence of SEQ ID NO: 24 or SEQ ID NO: 27. The antisense oligomer according to the present invention may comprise, for example, a sequence having SEQ ID NO: 24 or SEQ ID NO: 27.

As used herein, the term "homology" means the percent homology between polynucleotide moieties. The term "identity" means the degree to which sequences are functionally or structurally identical based on the polynucleotide sequences through a comparison window. Sequence homology may be determined by comparing sequences using standard software, for example, a program called "BLASTN" or "BLASTX", which was developed based on BLAST (Proc. Natl. Acad. Sci. USA, 90, 5873-5877, 1993).

In an embodiment of the present invention, the antisense oligomer according to the present invention may comprise 20 linked nucleosides.

As used herein, the complementary sequence comprises some base deletions and incomplete complementarity corresponding to the degree of suppression of expression of mRNA encoding GFRAL.

In one embodiment of the present invention, the antisense oligonucleotide is capable of obtaining the desired effect on regulating the expression of GFRAL by selecting at least one target site in the nucleic acid sequence of the gene encoding GFRAL, selecting an oligonucleotide sufficiently complementary to the target site, and hybridizing sufficiently specifically the oligonucleotide with the target site.

As used herein, the term "hybridization" means a hydrogen bond, which may be a Watson-Crick, Hoogsteen or reverse Hoogsteen hydrogen bond between complementary nucleosides or nucleotide bases. For example, adenine and thymine are complementary nucleic acid bases that form hydrogen bonds and are paired.

As used herein, the term "hybridizable", "complementary", or "substantially complementary" means that a nucleic acid (e.g., RNA or DNA) includes a sequence of nucleotides that non-covalently binds to another nucleic acid in a sequence-specific, antiparallel manner (i.e., the nucleic acid specifically binds to the complementary nucleic acid), i.e., by forming a pair of adenine (A) and thymine (T), a pair of adenine (A) and uracil (U), and a pair of guanine (G) and cytosine (C), or by performing "annealing" or "hybridization" under appropriate in vitro and/or in vivo conditions of temperature and solution ionic strength.

In one embodiment of the present invention, the hybridization occurs between an antisense oligonucleotide disclosed herein and a nucleic acid sequence of a gene encoding GFRAL. The most common mechanism of hybridization involves hydrogen bonding between complementary nucleic acid bases of the nucleic acid molecules.

Hybridization may occur under a variety of conditions. Stringent conditions are sequence-dependent and are determined by the nature and composition of the nucleic acid molecule hybridized. Methods of determining whether a sequence can specifically hybridize with a target nucleic acid are well known in the art.

As used herein, the term "complementary" refers to the property by which two nucleotides are capable of precisely pairing. For example, when two different nucleic acids of oligonucleotides or the nucleotide sequences of oligonucleotides are oriented in the 5' to 3' direction, and a certain portion of the nucleotide sequence of one nucleic acid or oligonucleotide is aligned in the opposite direction and it non-covalently binds with a certain portion of the other nucleic acid or oligonucleotide, that is, forms a pair of adenine (A) and thymine (T), a pair of adenine (A) and uracil (U), and a pair of guanine (G) and cytosine (C), the two nucleic acids or oligonucleotides are referred to as "complementary".

Accordingly, the terms "specifically hybridizable" and "complementary" may be used to indicate a sufficient degree of complementarity or precise pairing that allows stable specific binding to occur between an oligonucleotide and a DNA or RNA target. It is known in the art that the sequence of an antisense oligonucleotide need not be 100% complementary to the sequence of a target nucleic acid to be specifically hybridized.

The antisense oligonucleotide is interpreted as being capable of specifically hybridizing with a target DNA or RNA to inhibit the normal function of the target DNA or RNA, and having a sufficient degree of complementarity to prevent nonspecific binding of the antisense oligonucleotide to non-target sequences under conditions where specific binding is desired, i.e., under physiological conditions in an in vivo assay or treatment, or under assay conditions in an in vitro assay.

That is, when the antisense oligonucleotide can specifically hybridize with the target nucleic acid, non-complementary nucleic acid bases between the antisense oligonucleotide and the target nucleic acid may be used. Furthermore, the antisense oligonucleotide can hybridize with one or more nucleic acid portions such that the intervening or adjacent portions are not involved in hybridization (e.g., a loop structure, a mismatch, or a hairpin structure).

As used herein, the term "fully complementary" means that each nucleic acid base of the antisense oligonucleotide can form an exact base pair with the corresponding nucleic acid base of the target nucleic acid.

In one embodiment of the present invention, the non-complementary nucleic acid base may be located at the 5' end or the 3' end of the antisense oligonucleotide. Alternatively, the non-complementary nucleic acid base or nucleic acid bases may be located inside the antisense oligonucleotide. When two or more non-complementary nucleic acid bases are present, they may be adjacent (i.e., bound) or non-adjacent. In one embodiment of the present invention, the non-complementary nucleic acid base may be located at the wing portion of the gapmer antisense oligonucleotide.

In one embodiment of the present invention, the antisense oligonucleotide of the present invention may comprise those that are complementary to a portion of a nucleic acid sequence of a gene encoding GFRAL. As used herein, the term "portion" means a predetermined number of nucleic acid bases that are adjacent (i.e., bound) within a region or part of a target nucleic acid. The portion also means a predetermined number of adjacent nucleic acid bases of the antisense oligonucleotide. In one embodiment, the antisense oligonucleotide may be complementary to at least 8 nucleic acid bases of the target portion, may be complementary to at least 12 nucleic acid bases of the target portion, or may be complementary to at least 15 nucleic acid bases of the target portion. Antisense oligonucleotides that are complementary to at least 9, 10, 11, 12, 13, 14, 15, 16, 17, 18, 19, or 20 nucleic acid base of the target portion, or to nucleic acid base parts within a range defined by two of these values, also fall within the range defined above.

As used herein, the term "nucleotide" means a unit molecule constituting a nucleic acid formed by bonding of a nucleobase, a sugar moiety, and a phosphate group, and the nucleotide may encompass all unmodified or modified nucleobases, sugar moieties, and/or phosphate groups, such as nucleotide analogs, modified nucleotides, non-natural nucleotides, and non-standard nucleotides.

As used herein, the term "nucleoside" refers to a glycosylamine, which is considered as a part of a nucleotide excluding a phosphate group, and means a unit molecule comprising a nucleobase and a sugar moiety. The nucleoside may encompass all nucleosides in which nucleobase G or sugar moiety is modified or unmodified, like nucleotides.

As used herein, the term "oligonucleotide" refers to a low-molecular-weight polymer or polymer of ribonucleic acid (RNA) or deoxyribonucleic acid (DNA) or the analogue thereof. The oligonucleotide generally comprises oligonucleotides comprising covalent bonds between nucleobases, sugars, and nucleosides (backbones) present in living organisms, and modified or substituted oligonucleotides including nucleotide analogues, modified nucleotides, non-natural nucleotides, and non-standard nucleotides which act similar to such oligonucleotides. Such modified or substituted oligonucleotides have properties such as enhanced cellular uptake, enhanced nucleic acid target affinity, and increased stability in the presence of a nuclease, compared to unmodified or unsubstituted oligonucleotides.

As used herein, the term "antisense oligonucleotide (ASO)" encompasses an oligonucleotide capable of hybridizing with a target nucleic acid sequence by hydrogen bonding. The antisense oligonucleotide comprises, but is not limited to, oligonucleotides, oligonucleotide analogs, oligonucleotide mimetics, siRNA, single-stranded siRNA (ss siRNA), short hairpin RNA (shRNA), micro RNA mimics, ribozymes, external guide sequence oligonucleotides, and other oligonucleotides that hybridize with a target nucleic acid sequence to regulate the expression thereof, and the antisense oligonucleotides comprise single-stranded and double-stranded oligonucleotides.

In one embodiment of the present invention, the antisense oligonucleotide has a nucleic acid sequence that comprises a reverse complementarity to a target portion of a target nucleic acid sequence to be targeted in the 5' to 3' direction. Preferably, the antisense oligonucleotide complementarily binds to a nucleic acid sequence of a gene encoding GFRAL. The gene encoding GFRAL may be selected from mRNA and pre-mRNA including introns, exons, and untranslated regions as a nucleic acid that is the target of the antisense oligonucleotide.

In the present invention, the oligomer may comprise a chemical modification of a modified internucleoside linker, a modified nucleobase, or a modified nucleoside.

Specifically, the modified nucleobase may be 5'-methyl-cytosine.

Specifically, the internucleoside linkage may be a phosphorothioate, boranophosphate, or methyl phosphonate linkage.

The internucleoside linkage according to the present invention may be a linkage of sooosssssssssssooos. s may be a phosphorothioate internucleoside linkage and o may be a phosphodiester internucleoside linkage.

The nucleoside may include the following modified sugars:

A modification of substitution at the 2' carbon position of the sugar structure with 2'-O-methyl (2'-O-Me), 2'-O-methoxyethyl (2'MOE), 2'-O-methoxyethyl-5'-methyl, 2'-O-aminoethyl, 5'-methyl, 2'-O-propyl, 2'-methylthioethyl, or 2'-fluoro.

In the nucleoside, the internucleoside linkage may, for example, include: a phosphorothioate, boranophosphate, or methyl phosphonate linkage; and may include modifications of substitution at the 2' carbon position of the sugar structure with 2'-O-methyl (2'-O-Me), 2'-O-methoxyethyl (2'MOE), 2'-O-methoxyethyl-5'-methyl, 2'-O-aminoethyl, 5'-methyl, 2'-O-propyl, 2'-methylthioethyl, or 2'-fluoro.

The nucleoside may, for example, include a modification selected from the group consisting of the following modifications: modifications of sugar moiety, for example, modifications at the 2' carbon position of the sugar structure in the nucleotide, specifically modifications to 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro; modifications of the nucleotide linkage to phosphorothioate, boranophosphate, or methyl phosphonate; modifications to peptide nucleic acid

(PNA), locked nucleic acid (LNA), or unlocked nucleic acid (UNA); and a phosphate group.

The term "2'-O-methoxyethyl" (also 2'-MOE and 2'-O(CH2)2-OCH3) means an O-methoxy-ethyl modification at the 2' position of the furosyl ring.

The term "2'-O-methoxyethyl nucleotide" means a nucleotide comprising a 2'-O-methoxyethyl modified sugar moiety.

The term "modified sugar" refers to a substitution or change from a natural sugar.

The term "5-methylcytosine" refers to a cytosine modified with a methyl group attached at the 5' position. 5-methylcytosine is a modified nucleobase.

The term "modified internucleoside linkage" refers to a substitution or any change from a naturally occurring internucleoside linkage.

The term "modified nucleobase" means any nucleobase other than adenine, cytosine, guanine, thymidine, or uracil. The term "modified nucleobase" means the purine bases adenine (A) and guanine (G), and the pyrimidine bases thymine (T), cytosine (C), and uracil (U).

The term "modified nucleotide" means, independently, a nucleotide having a modified sugar moiety, a modified internucleoside linkage, or a modified nucleobase. The term "modified nucleoside" means, independently, a nucleoside having a modified sugar moiety or a modified nucleobase.

The term "modified oligonucleotide" means an oligomer comprising at least one modified nucleotide.

The antisense oligomer according to the present invention may include a modification to 2'-O-methoxyethyl (2'-MOE), 2'-O-methoxyethyl-5'-methyl, 5'-methyl or phosphorothioate linkage.

### Modification of the sugar moiety

In one embodiment of the present invention, the modified nucleoside may be a modified nucleoside comprising a non-bicyclic modified sugar moiety and/or a bicyclic or tricyclic sugar moiety, and/or a sugar moiety modified with a sugar surrogate or sugar mimetic.

In the present invention, the modified nucleoside is, for example, a sugar moiety into which one or more substituents selected from 2'-O-alkyl such as 2'-O-methyl, 2'-O-alkoxy such as 2'-O-methoxy, 2'-O-alkoxyalkyl such as 2'-O-methoxyethyl, 2'-amino, 2'-allyl, 2'-fluoro, 2'-arabino-fluoro, 2'-O-N-substituted acetamide such as 2'-OCH₂C(=O)-NHCH₃ (NMA), 2'-O-benzyl and 2'-O-methyl-4-pyridine, 4'-O-methyl, 5'-methyl, 5'-vinyl and 5'-methoxy are introduced, but is not limited thereto.

The antisense oligomer according to the present invention may optionally comprise one or more modified nucleosides having substituted or modified sugar moieties. Modification of the sugar moieties imparts nuclease stability, binding affinity or other advantageous biological properties to the oligomer. The (pento)furanosyl sugar ring of the natural nucleoside may be modified in various ways, including, but not limited to: addition of a substituent (particularly at the 2' position); cross-linkage of two different ring atoms forming a bicyclic nucleic acid (BNA); and substitution of an atom or group such as -S-, -N(R)- or - C(R1) (R2) at the ring oxygen at the 4' position. Modified sugar moieties include, but are not limited to: substituted sugars, particularly, 2'-substituted sugars having 2'-F, 2'-OCH₂(2'-OMe) or 2'-O(CH₂)₂-OCH₃ (2'-O-methoxyethyl or 2'-MOE) substituents; and bicyclic modified sugars (BNAs) having 4'-(CH₂)n-O-2' (n=1 or n=2) bridges. Methods of preparing such modified sugars are known in the art. The base portion of the nucleoside including the modified sugar moiety may be maintained to hybridize with the target nucleic acid.

In the present invention, the modified nucleoside includes, at the 2' position, F; O-, S-, or N-alkyl; O-, S-, or N-alkenyl; O-, S-, or N-alkynyl; O-alkyl-O-alkyl; O-alkyl-O-alkyl-N (dialkyl); or O-alkyl-carboxylamide (wherein alkyl, alkenyl and alkynyl are substituted or unsubstituted C₁ to C₁₀ alkyl or C₂ to C₁₀ alkenyl and alkynyl). Particularly preferred are O[(CH₂)nO]mCH₃, O(CH₂)nOCH₃, O(CH₂)nNH₂, O(CH₂)nCH₃, O(CH₂)nONH₂, O(CH₂)n0(CH₂)nN[(CH₂)mCH₃]₂, O(CH₂)nC(=O)-NHCH₃ and O(CH₂)n0N[(CH₂)mCH₃]₂ (wherein n and m are 0 to about 10).

Preferably, the modification may include 2'-O-CH₂CH₂OCH₃, which is also known as 2'-methoxyethoxy (2'-O-(2-methoxyethyl) or 2'-MOE (Martin et al., HeIv. Chim. Acta, 1995, 78, 486-504), i.e., an alkoxyalkoxy group, and the modification may include 2'-dimethylaminooxyethoxy (i.e., a (CH₂)₂ON(CH₃)₂ group, also known as 2'-DMAOE), and 2'-dimethylaminoethoxyethoxy [i.e., a 2'-O(CH₂)2O(CH₂)₂-N(CH₃)₂ group, also known as 2'-O-dimethylaminoethoxyethyl or 2'-DMAEOE].

The bicyclic or tricyclic sugar moiety may be selected from the group consisting of locked nucleic acid (LNA), constrained ethyl bicyclic nucleic acid (cEt), 2'-O,4'-C-ethylene-bridged nucleic acid (ENA), and tricyclo-DNA, but is not limited thereto.

In one specific embodiment, the modified nucleoside may include a sugar surrogate having a six-membered ring or acyclic moiety. The sugar surrogate may be selected from the group consisting of a morpholino ring, such as a phosphorodiamidate morpholino oligomer (PMO), a cyclohexenyl ring, a cyclohexyl ring, and a tetrahydropyranyl ring, for example, hexitol, anitol, mannitol, and fluorohexitol, but not limited thereto. Various other bicyclic and tricyclic sugar substituted ring systems that may be used to modify the nucleosides introduced into the antisense oligomers according to the present invention are known in the art. The activity of such ring systems may be enhanced through various substitution processes.

In addition, the sugar surrogate may be an acyclic moiety such as an unlocked nucleic acid (UNA) or a peptide nucleic acid (PNA), but is not limited thereto.

Peptide nucleic acid (PNA) is a type of nucleic acid analogue in which nucleobases are linked by peptide bonds instead of phosphate bonds, and the phosphodiester bond is replaced by a peptide bond, and has nucleobases such as adenine, thymine, guanine, and cytosine, so that it can specifically cause a hybridization reaction with nucleic acids. PNA is not found in nature, but is artificially synthesized by chemical methods, and may form a double strand through a hybridization reaction with a nucleic acid of a complementary nucleotide sequence. In addition, PNA is not only chemically stable because it is electrically neutral, but also is biologically stable because it is not degraded by a nuclease or protease. Although the N-aminoethylglycine skeleton is the most widely used PNA, PNA having a modified skeleton may also be used as is known in the art (P.E. Nielsen and M. Egholm "An Introduction to PNA" in P.E. Nielsen (Ed.) "Peptide Nucleic Acids: Protocols and Applications" 2nd Ed. Page 9 (Horizon Bioscience, 2004)).

Unlocked nucleic acid (UNA) is a modified nucleoside that does not have the C2'-C3' bond of ribose. Due to its open chain structure, unlocked nucleic acid (UNA) adjusts the flexibility of oligonucleotides without restricting the steric configuration. It is known that, when UNA is included in an oligomer, the Tm value may be lowered by about 5°C to 10°C and the off-target may be reduced.

### Modification of nucleobase

In the present invention, the modified nucleoside includes at least one modified nucleobase selected from the group consisting of pseudouridine, 2'-thiouridine, N6'-methyladenosine, 5'-methylcytidine, 5'-fluoro-2-deoxyuridine, N-ethylpiperidine 7'-EAA triazol modified adenine, N-ethylpiperidine 6'-triazol modified adenine, 6'-phenylpyrrolocytosine, 2',4'-difluorotoluylribonuleoside and 5'-nitroindole, but is not limited thereto.

Unmodified or natural nucleic acid bases mean the purine bases, namely, adenine (A) and guanine (G), and the pyrimidine bases, namely, thymine (T), cytosine (C), and uracil (U).

The modified nucleoside may also include nucleobase modifications or substitutions. The nucleobase modifications or substitutions are structurally distinct but functionally interchangeable with naturally occurring or synthetically unmodified nucleobases. The naturally occurring nucleobases and the modified nucleobases may participate in hydrogen bonding. Such nucleobase modifications impart nuclease stability, binding affinity, or other favorable biological properties to the antisense oligomer. For example, certain nucleobase substitutions, such as 5-methylcytosine substitutions, are known to increase nucleic acid duplex stability by 0.6-1.2°C and may be particularly useful in enhancing the binding affinity of the antisense oligomer to the target nucleic acid.

For example, the modified nucleobases include 5'-hydroxymethyl cytosine, xanthine, hypoxanthine, 2'-aminoadenine, 6'-methyl and other alkyl derivatives of adenine and guanine, 2'-propyl and other alkyl derivatives of adenine and guanine, 2'-thiouracil, 2'-thiothymine and 2'-thiocytosine, 5'-halouracil and cytosine, other alkynyl derivatives of 5'-propynyl (-C≡C-CH3) uracil and cytosine and pyrimidine bases, 6'-azouracil, cytosine and thymine, 5'-uracil (pseudo-uracil), 4'-thiouracil, 8'-halo, 8'-amino, 8'-thiol, 8'-thioalkyl, 8'-hydroxy and other 8'-substituted adenines and guanines, 5'-halo (especially 5'-bromo), 5'-trifluoromethyl and other 5'-substituted uracil and cytosine, 7'-methylguanine and 7'-methyladenine, 2'-F-adenine, 2'-amino-adenine, 8'-azaguanine and 8'-azaadenine, 7'-deazaguanine and 7'-deazaadenine and 3'-deazaguanine and 3-deazaadenine, tricyclic pyrimidines such as phenoxazine cytidine (lH-pyrimido[5,4-b][1,4]benzoxazin-2(3H)-one), phenothiazine cytidine (lH-pyrimido[5,4-b][1,4]benzothiazin-2(3H)-one), and G clamps, for example, substituted phenoxazine cytidines such as 9-(2-aminoethoxy)-H-pyrimido[5,4-b][1,4]benzoxazin-2(3H)-one), carbazole cytidine (2H-pyrimido[4,5-b]indol-2-one), and pyridoindole cytidine (H-pyrido[3',2':4,5]pyrrolo[2,3-d]pyrimidin-2-one).

The modified nucleobases include nucleobases disclosed in U.S. Patent No. 3,687,808, the literature [The Concise Encyclopedia Of Polymer Science And Engineering, pages 858-859, Kroschwitz, J. I., ed. John Wiley & Sons, 1990,Englisch et al., Angewandte Chemie, International Edition, 1991, 30, 613], and the literature [Sanghvi, Y. S., Chapter 15, Antisense Research and Applications, pages 289-302, Crooke, S. T. and Lebleu, B. ed., CRC Press, 1993].

### Modified internucleoside linker

In the present invention, in the antisense oligomer, the modified internucleoside linker may include at least one modified internucleoside linker selected from the group consisting of phosphorothioate, phosphorodithioate, phosphotriester, phosphoramidate, mesyl phosphoramidate, methylphosphonate, methoxypropyl-phosphonate, and boranophosphate.

As is known in the art, the nucleoside is a combination of a nucleobase and a sugar moiety. The nucleotide further includes a phosphate group covalently bonded to the sugar moiety of the nucleoside. In a nucleotide comprising a pentofuranosyl sugar, the phosphate group may be bonded to the 2', 3' or 5' hydroxyl group of the linked sugar. In the formation of an oligonucleotide, the phosphate group covalently bonds to adjacent nucleosides to form a linear polymer compound. In turn, each terminal of the linear polymer structure also bonds to form a circular structure, but an open linear structure is generally preferred. In the oligonucleotide structure, the phosphate group typically forms the internucleoside backbone of the oligonucleotide, and the naturally occurring bonds and backbones of RNA and DNA are 3' to 5' phosphodiester bonds. The antisense oligomer according to one embodiment may comprise one or more modified internucleoside bonds in addition to the naturally occurring internucleoside bonds, which are often selected over antisense oligomers including naturally occurring internucleoside bonds due to the properties thereof such as enhanced cellular uptake, enhanced target nucleic acid affinity, and increased stability in the presence of nucleases.

A specific example of a preferred antisense oligomer that may be used in the present invention is an oligonucleotide comprising a modified backbone or an unnatural internucleoside linkage. As defined above, an oligonucleotide having a modified backbone comprises a nucleotide containing a phosphorus atom in the backbone and a nucleotide that does not contain a phosphorus atom in the backbone. In addition, as used in the art, a modified oligonucleotide that does not contain a phosphorus atom in the internucleoside backbone is also interpreted as an oligonucleotide herein.

In an antisense oligomer according to one embodiment, the modified internucleoside linkage may comprise a linkage between nucleosides that contain a phosphorus as well as a linkage between nucleosides that do not contain phosphorus. Examples of the representative phosphate-containing internucleoside linkage may include, but are not limited to, phosphorothioates, chiral phosphorothioates, phosphorodithioates, phosphotriesters, aminoalkylphosphotriesters, methyl and other alkyl phosphonates and phosphinates including 3'-alkylene phosphonates, 5'-alkylene phosphonates and chiral phosphonates, phosphinates, 3'-amino phosphoramidates and aminoalkylphosphoramidates having normal 3'-5' linkage and 2'-5' linkage analogues thereof, phosphoramidates including mesyl phosphoramidate, thionophosphoramidate, thionoalkylphosphonates, thionoalkylphosphotriesters, selenophosphates and boranophosphates, and compounds having opposite polarity wherein at least one internucleotide linkage is a 3'-3', 5'-5' or 2'-2' linkage.

In the present invention, the modified oligonucleotide comprises a gap segment comprising linked deoxynucleosides, a 5' wing segment comprising linked nucleosides, and a 3' wing segment comprising linked nucleosides, wherein the gap segment is located between the 5' wing segment and the 3' wing segment, and the nucleoside of each wing segment may comprise a modified sugar moiety or sugar surrogate, but is not limited thereto.

In one embodiment of the present invention, the modified oligonucleotide includes: a gap segment comprising 8 to 10 linked deoxynucleosides; a 5' wing segment comprising 3 to 5 linked nucleosides; and a 3' wing segment comprising 3 to 5 linked nucleosides, and each nucleoside of each wing segment may include a modified sugar moiety, but is not limited thereto.

In a gapmer, an internal region having a number of nucleotides that support RNase H cleavage is located between external regions having a number of nucleosides that are chemically different from the nucleosides of the internal region. In the antisense oligonucleotide having a gapmer motif, the gap segment may support cleavage of the target nucleic acid, while the wing segment may comprise a modified oligonucleotide comprising modified nucleosides for improved stability, affinity, and exonuclease resistance.

If desired, the gap segment may also comprise a modified oligonucleotide. The modified oligonucleotide may comprise one or more modifications selected from one or more modified internucleoside linkers, one or more modified nucleosides comprising a modified sugar moiety, and one or more modified nucleosides comprising a modified nucleobase, wherein each modification is as described above.

In one embodiment of the present invention, the antisense oligomer may preferably comprise a nucleotide sequence represented by SEQ ID NO: 19 to SEQ ID NO: 34, and may comprise a chemical modification of Table, 2 but is not limited to.

In the present invention, the oligomer may most preferably comprise a structure represented by the following Formula (I) or Formula (II):
G*GTATA*8*8*8*8*7*5*8*5*7*5*AGAAC*C Formula (I);
C*ACAA*5*5*5*7*6*6*5*5*6*5*TCTG*G Formula (II),
wherein A represents 2'MOE-A, C represents 2'MOE-5'-methyl-C, G represents 2'MOE-G, T represents 2'MOE-T, 5 represents DNA-A, 6 represents DNA-5'-methyl-C, 7 represents DNA-G, 8 represents DNA-T, * represents PS (phosphorothioate), and 2'MOE represents 2'-O-methoxyethyl.

In the present invention, various formulations have been developed to facilitate the use of oligomers, for example, by minimizing degradation, facilitating delivery and/or absorption, or imparting other beneficial properties to the oligonucleotide in the formulation, which can deliver the oligonucleotide to the target or cellular environment.

In the present invention, the antisense oligomer for reducing the expression of GFRAL can be suitably formulated so that, when administered to a subject either systemically or directly under the environment of the target cell, a sufficient portion of the oligomer enters the cell to reduce the expression of GFRAL. In one embodiment, the antisense oligomer may be formulated in the form of a buffer solution, for example, a phosphate buffered saline solution, a liposome, a micelle structure, or a capsid. In addition, the antisense oligomer may also be formulated in water or an aqueous solution (for example, pH-adjusted water) or a basic buffered aqueous solution (for example, PBS).

In the present invention, the formulation of the oligomer having a cationic lipid may be used to facilitate the introduction of the oligomer into the cell. For example, cationic lipids, such as lipofectamine, cationic glycerol derivatives, and polycationic molecules (e.g., polylysine) may be utilized, and suitable lipids include oligofectamine, lipofectamine (Life Technologies), NC388 (Ribozyme Pharmaceuticals, Inc.), and FuGene 6 (Roche), all of which may be used in accordance with the manufacturer's protocol. Such formulations may include lipid nanoparticles.

In addition, the formulation may comprise an excipient. The excipient may include a liposome, a lipid, a lipid complex, a microsphere, a microparticle, a nanosphere, or a nanoparticle, or may be otherwise formulated for administration to a cell, tissue, organ, or body of a subject in need thereof (see, e.g., Remington: The Science and Practice of Pharmacy, 22nd edition, Pharmaceutical Press, 2013). The excipient imparts improved stability, improved absorption, improved solubility, and/or therapeutic enhancement of the active ingredient to the composition. In addition, the excipient may be a buffer (e.g., sodium citrate, sodium phosphate, tris(II) base, or sodium hydroxide) or a vehicle (e.g., a buffered solution, petrolatum, dimethyl sulfoxide, or mineral oil).

In one embodiment, the oligomer may be lyophilized to extend the shelf life thereof and then prepared into a solution prior to use. Accordingly, the excipient in the composition comprising the oligomer according to the present invention may be a lyoprotectant (e.g., mannitol, lactose, polyethylene glycol, or polyvinyl pyrrolidone), or a disintegration temperature regulator (e.g., dextran, Ficoll, or gelatin).

The pharmaceutical composition suitable for injection may comprise a sterile aqueous solution (if water-soluble) or dispersion and a sterile powder for the extemporaneous preparation of sterile injectable solutions or dispersions. For intravenous or subcutaneous administration, suitable carriers may include saline, bacteriostatic water, Cremophor EL.TM. (BASF), or phosphate buffered saline (PBS). In addition, the carrier may be a solvent or dispersion medium comprising, for example, water, ethanol, a polyol (e.g., glycerol, propylene glycol, or liquid polyethylene glycol), and suitable mixtures thereof. In many cases, the composition preferably comprises isotonic agents, for example, sugars, polyalcohols (e.g., mannitol, sorbitol), and sodium chloride. The sterile injectable solution may be prepared by incorporating the oligonucleotide in the required amount in a selected solvent along with one of the ingredients described above or a combination thereof, as desired, followed by sterile filter sterilization. The pharmaceutical composition may comprise at least about 0.1% of a therapeutic agent (e.g., an antisense oligonucleotide for reducing the expression of WFDC2), but the percentage of active ingredient is preferably from about 1% to about 80% with respect to the total weight or volume of the composition. Factors such as solubility, bioavailability, biological half-life, route of administration, and product shelf life, as well as other pharmacological considerations will be taken into account in the preparation of the formulation.

In another aspect, the present invention is directed to a pharmaceutical composition for preventing or treating a disease related to activation of GFRAL due to an increase in GDF15 comprising the oligomer.

In still another aspect, the present invention is directed to a method of preventing or treating a disease related to activation of GFRAL due to an increase in GDF15 comprising administering the oligomer to a subject.

In yet another aspect, the present invention is directed to the use of the oligomer for the prevention or treatment of a disease related to activation of GFRAL due to an increase in GDF15.

In further aspect, the present invention is directed to the use of the oligomer for the manufacture of a medicament for preventing or treating a disease related to activation of GFRAL due to an increase in GDF15.

As used herein, the term "prevention" means any action of inhibiting or delaying the progression of a disease associated with the activation of GFRAL due to an increase in GDF15 by administering the composition of the present invention, and as used herein, the term "treatment" means inhibiting the development of a disease associated with the activation of GFRAL due to an increase in GDF15, or alleviating or eliminating symptoms of the disease.

In the present invention, the disease associated with the activation of GFRAL due to an increase in GDF15 may be obesity, diabetes, anorexia, or cachexia, but is not limited thereto.

Specifically, the disease may be cancer cachexia.

Cachexia is a debilitating metabolic syndrome associated with a number of diseases, including cancer, AIDS, chronic heart failure (also known as congestive heart failure), chronic obstructive pulmonary disease (COPD), chronic kidney disease, tuberculosis, sepsis, and other forms of systemic inflammation. In particular, cachexia may be a wasting disorder associated with involuntary weight loss and may be associated with systemic inflammation and/or acute inflammatory responses. In addition to loss of muscle mass, loss of fat mass is often a major clinical feature of cachexia. Sarcopenia caused by a loss of muscle mass may result in functional impairments, including loss of strength, increased tendency to fall, and loss of autonomy. Respiratory function may also be impaired, resulting in decreased lung capacity. Sarcopenia is usually a disease of the elderly, but the development of sarcopenia may be associated with muscle wasting and malnutrition, and may occur concurrently with cachexia. Cachexia may progress through stages precachexia, cachexia, and refractory cachexia.

GDF15 is expressed at increased levels in serum in a number of malignant cancers (particularly, invasive brain cancer, melanoma, lung cancer, gastrointestinal tumors, colon cancer, pancreatic cancer, prostate cancer and breast cancer). Likewise, chemoresistance may be correlated with high GDF15 expression and a correlation between high GDF15 expression levels and cancer prognosis has also been reported. GDF15, which is a member of the TGF-β superfamily, has been reported to be a mediator of cachexia induced by various diseases. GFRAL is a high affinity receptor for GDF15 and thus the antisense oligomer according to the present invention may be used for the prevention or treatment of GDF15-induced cancer cachexia.

As used herein, the terms "cancer" and "tumor" are used interchangeably and refer to or mean a physiological condition of mammals characterized by uncontrolled cell growth/proliferation.

In the present invention, the pharmaceutical composition comprises a therapeutically effective amount of the antisense oligomer.

As used herein, the term "pharmaceutically acceptable carrier" refers to a substance that may be added to an active ingredient to help formulate or stabilize a preparation and does not cause significant harmful toxic effects to the patient. The pharmaceutically acceptable carrier may be commonly used in formulations and include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil and the like.

The pharmaceutical composition may further comprise, in addition to the ingredients described above, a lubricant, a wetting agent, a sweetening agent, a flavoring agent, an emulsifier, a suspending agent, a preservative, or the like. Suitable pharmaceutically acceptable carriers and formulations are described in detail in Remington's Pharmaceutical Sciences (19th ed., 1995).

As used herein, the term "administration" means introducing the pharmaceutical composition of the present invention into a patient by any appropriate method, and the pharmaceutical composition of the present invention may be administered orally or parenterally, for example, by infusion, intravenous injection, intramuscular injection, subcutaneous injection, intraperitoneal injection, intrarectal administration, topical administration, intranasal injection, or the like, but is not limited thereto.

The suitable dose of the pharmaceutical composition according to the present invention may vary depending on factors such as the formulation method, administration method, and age, body weight, gender, pathological conditions, diet, administration time, administration route, excretion rate, and responsiveness of the patient. A dose effective for the desired treatment or prevention may be easily determined and prescribed by a skilled physician. As used herein, the term "pharmaceutically effective amount" means an amount sufficient to prevent or treat obesity, diabetes, anorexia, or cachexia.

The pharmaceutical composition of the present invention may be used in combination with a conventional therapeutic agent. That is, the antisense oligomer according to the present invention and the pharmaceutical composition comprising the same may be administered simultaneously, sequentially or in reverse order along with a therapeutic agent such as a conventional anticancer drug, and may be administered in a combination of appropriate effective amounts within the scope of those skilled in the art.

Hereinafter, the present invention will be described in more detail with reference to examples. However, it will be obvious to those skilled in the art that these examples are provided only for illustration of the present invention and should not be construed as limiting the scope of the present invention.

### Example 1: Development of GFRAL target antisense oligonucleotides

In consideration of target specificity and stability, 16 GFRAL mRNA target sequences (SEQ ID NOs: 3 to 18) were developed from the GFRAL gene (pre-mRNA sequence) and GFRAL mature-RNA sequences (SEQ ID NOs: 1 to 2), and 16 antisense sequences (SEQ ID NOs: 19 to 34) were developed for these sequences (Table 1).

In addition, 16 antisense oligonucleotides were developed by applying chemical modification to the 16 complementary binding sequences in Table 1 (Table 2). As an antisense oligonucleotide structure, a "gapmer" was used, which comprises a "gap" portion comprising 10 nt internal DNA nucleotides, and a "wing" portion comprising 4-6 nt 2'-MOE nucleotides each having a 2'-MOE (2'-Methoxyethyl) modified backbone at the 5' end and the 3'-end, which is known to be stable and effective. In the gapmer, a phosphorothioate bond (PS bond) was used instead of the conventional phosphodiester bond (PO bond) for the portion connecting two DNA nucleosides or as the bond connecting the DNA nucleoside to the 2'MOE nucleoside, and PS bonds were used only as bonds located at the last two ends of the sequence of the wing portion composed of 2'-MOE nucleotides, and PO bonds were used as the remainder. Meanwhile, all cytosines (C) were replaced with methylated cytosines, and all uracils (U) in the RNA portion were replaced with thymine (T), which is intended to increase the stability of the antisense oligonucleotide and reduce the host immune response.

For example, A427 has the sequence and chemical modification of G*GTATA*8*8*8*8*7*5*8*5*7*5*AGAAC*C (A=2'MOE-A, C=2'MOE-5'-methyl-C, G=2'MOE-G, T=2'MOE-T, 5=DNA-A, 6=DNA-5'-methyl-C, 7=DNA-G, 8=DNA-T, *=PS).
SEQ ID NO: 1 (GenBank Homo sapiens chromosome 6, GRCh38.p14 Primary Assembly NC 000006.12 REGION: 55327469..55402493) 
TTATTCTGGACAGTTACTCTTAAGAAAGTTGTCAGAAGAAACGCATCTGCCTTTTT
TTCCAGGTGAACTGCCGTGAGTTGTCCAGCATGATAGTGTTTATTTTCTTGGGTAAGTGAA
TGGTGCTTCTGGTTTATCTGAATTATTCATAATACTAATTCTTTGTAAACTGAATACCATC
ACACTTAAGCTTAACAGGGGCTTATAATATGAAGTACCAAGTAAATTACAAATTTATGCTT
TAACATGGTTTATTCTTCTTGCAAAAATGTACACTCACTTTTACTCACTTATTTCTAACTC
TATAGATAATGCAGCATGTGAAAATTGTATTAATCTCTCAGCTTGTTTCTGTTTTATTGTA
TAGAATGACCCAATGACTTTGAGTTACTTATTAGGATTAATGGAATTCACACATGTGTTAG
AACCTTTTAGAAATAAAAATAAAACCTTCCAGTATCTATTTACAAGTTATAAGATGAAAAA
TTTCAAACTGTTATCTATTTTATTTATTTTGCTTTGAAATTAAACAAAAATAAGATTAATT
 
TAAAACATTCAATTTACCTACAGTGTTAATATTTGCTAACTAGAAGTAATTTAGTGAAGTA

AAACCACTTAACACCATATCAGAATGCTTCATATTTTCATTAATTTTTATGACCTAGAACT

CAAGTGTGGACTTACTTCTCTTGTATTGGATCCGATGAATGTATTATAAGATGTTGATGAA

AAATAATTTATTTTTATTTAATGTAGAATCCATGAATCTATTTGCTAGATGTGTGACTGGA

GAATATTTTATATCTTCTTTAGCATTTGGTAGAATCTATAAACTGAATTCAGAAAAGGAAT

AATTGTAACTGTAGCTAGATTGTAATTTTAAAAATATGGCATTTTATGTTTAAGCTAATAT

ATACTTGAATAAAGGCATAATTATGGGCCAGAAAGCAAAGAAGTTTACTTTAAATTGTATC

GAACATAAAAGGAGAGTCATATCAAGTCAAGGAAACAAGTTAAGAAGGAGAGAAATCCCTT

ATATTTTCAGTTTTTCTTTTGTTTAAGTAATATTTTTCCATTGTTCCATAATTTTGCTTTT

TGTAATTATTCCTAAAATATTTTCTTATTTTTATAAAGATTGTTAATATTTCTTTAGTTCT

TTTTTGTGATAAAGAGAAATATAGGAAACTTTAATTTTTATAGATCAGGCAGCAATATAAT

GAAGCTCACAAAATACATTACAGACTCATTATGACCTCAGTTGAAAAGGCAATGGCCTCTT

ATTTTCATCTTTTACTTCTAAATTAACTTGACACCAGGTTGCATGAAGGAGGGAAATAACT

TCTTAACATTGTTAGAACTAAAATTATACTATTTGATATGTTGTACCAACATTTCCCATAT

TATATTTTTTAGAATGCTTGTTTGACATTATCTAAATAGGTCTTATAATGAGGAAAATGTC

TGTTGGTAAAGAAATGTGGAAAACAATGAATTTTAAAATCTCAGAACATTTAGTAGGATAA

TAAGCCCCATACATCTTTAAAACACTGATATAATTTGCAGCATTATCCATCTTATTAATTT

AAAAATAGAAGTTATTTTTCATTGAGTATTACAGGAAAATTCTTGAAAAATCCTAGGTTAA

ATACAAAACCACAAGTCATAGTTTGGAATGAATAAGACAGATTTTGGAGAGGTAATACATT

CTTGATTCTTGTGGACCAAATCATTGAGGTGGTTTTCTTAGTTGAGTCAATATTTTGTTGA

GTAGGAAGTGTGGAAAATCCTATAAAATAATCTATTAAGAAGCTTGTTCAACCAAAAACTG

AAAATGTGAAAGGAAGTAAAACATTAAAAACTGAAAATGTGAAAGGAAGTAAAACATATTT

GGGGAAGACAACATGTAGTATTTCTAAGTGATTATTACTCTGAAGTACTGATGAAGTTTTG

ATGATATATCTGGGATATTATCACTTATAAGATTCTTGAAGGGGCTTTTCACAAGATATAA

TGATTAATGGCAAATAAAATACCATTTTCTTATAAGTTGAGTTTATGACTTCAAATTTCTA
 
TTTCTGGGTCTTTGGAAAACTGGATTTGACCAAGTAAATTTGTGCTAGGATCAAAAGAGTC

CTCTTTTTCTTTCTTTGGGCTAGCCAAATAAGGAGTACAAGGGCTTGGCTAGATGAGGTTG

TCCATGAACACATGAGAAAACAGAAAGCTGATATGTCCATTTCCACTCCAGCCCTCAGCAA

GAAAAATGTTACCAAGCTACACCCATGGGCCTAATCTCCAAATGCAGTGACCCAGCAAGTA

GTACACATGGTCCAATCAGTTGTCTTGGATTCTTCTTATAAGATGAGATTTGCAAAGAGGT

AAGATTTAAAAAAAAAAGAGAGATTTCAAGCCCTTACTTGAAGCCTATCACTCTACATTGT

CACAGGTGATTCATAATTAACAACAGCATTTAAAAATTCAAATAATTTGGCTCTTATTTAG

TTATAGCTTGACTCTCTTGATGACAGTTACTACAACTGTCTCTGTAACACAATTTCAATCC

CTGTATTTTTTTCTATTAATTGATTTGGCTAGTGTCACTTCATATGTTTGCAGAATCAATT

TGACTAACTTGCGTAACATATTAAATTAACATTGCTATTTTCTTGATTGTTTTTAGAAAAC

TTGCTGGATTTCTCCTCTAGCAAAAGTAACTTGTAAATATCTGGAAGGTATTATTTTTTAG

ATGTTATCAGTTATTTATATATCTTATATATAAACATCTAATAACTTGGGTATAAGCCAAT

ATTCATTTATTGAATACCTATACTGCATCACTGTGTTTGGTGACAAAAATACAGTTATAAA

TTATCCTTAATGTCTGTGGGTAAAGCTGTACAATCCAGTGGTGGAGACAGGCACATGAATA

TATACACTACAGAACAGTAAAATCTGTAATTGCGGGATCCCCACAGTTCTAGTAAATGGAT

GGAAGATGTCAAATTCAATTTGAGTGTGGAAAAAGGGGAGTATAGAATTCAAGTAAAGTTT

CCAGAAAATAGCTAATGATTGTGTTCAGTTTTAAACAACAAAAAGGGTTATTCCAGAAGAA

AAAGAAAGAAAATACTTATCAGGGATCAGACAGTAGGTATGCAGAGGTAGAAGACAGCATG

GATCATTCGGGAAACGATAAATGCTTGAGACCTTCCAGATTGCACATGGTGAGGAATATGA

GAGCCCTGATATGAGAAATAGTTTAAAGGATATAAAACAGAAAAATGGCAAGACTAGAATA

GTACATCAGGTAGATTAGGGGTTACAAAGAACCTATCTGAATAAGGTTCTGAATAAGATTC

AAACTGGAGGCAGACTGACTAATAAGTTAAACAAATGCTCAAAGTGAAAGTCAATAAATTT

AGCAAGAATGAATATGGGAGAGTGAAGTTGAGGATGGAAAATAGTCTACCACAAAACCAAG

ATTTCTAGTTTGAGTTCTCATGTTAATTGTGGCATTGTGAATGAAGTAGTGGTCCACAGTG

GAACAATGATGATGAAGATCCTGTGAAAGTGTTTCTGAAACATTCAAGTAGAGATAACAAT
 
TACGTACCTATGACAGGAACGCTGGAAATACTGAGATTAAATTATACATTTGAGAATCATC
AGACTAACATTTTAGTTCAAACCATAAGAGTACATGGAATTTTCTGCAGAGAATGTATAAA
ATGAAAATAGAAAAGGTCTGAGAAGAGAACCTAAGCTAAAATTATACTTTACTAGAAGAAT
AAAATGATCCTGGGAATAATTTTAGCTATGGTCAGAAAATACAAAGTGGATAAATATCAAG
AGAACAGAAGGGAGGGCTGGCAAAGGAAGAGTTTCAAAAACAAAAAAGTCAACAAAATGGT
CAATGGTGAGATAAAGTTTAAAACCATTAAGTTAGATTTTAAAGGGGTAATTAGAGACTTT
TTTAAGAGCAGTTTTAATGGAATAATAGTAATAAAAATAAATTATAAGTTGAGGAATAAAT
AGATGATGAGACGTCAAAATGAGCAAATTTAAAATATTCATTTAAGAATCTATAAAAAGAA
ATACAGATCTACAGCTAGAGAGGAGGTTAAGGTTATTCCCACCATCAATTATTTCACTACA
TGTTATTTTCCATAATTCTGCTCTTTATCATTAATAACTTAGATTTGAAGAAAATGGATGC
CAAATTTATATTACAACCTTGTTTTTGTTGTTGTTATTCAAGCTATGGGGTTAAGCTTGGA
AAATGAATACACTTCCCAAACCAATAATTGCACATATTTAAGAGAGCAATGCTTACGTGAT
GCAAATGGATGTAAACATGCTTGGAGAGTAATGGAAGATGCCTGCAATGATTCAGGTAAAC
AAGTTGCTAAAAATACACTCAAATGATTTATTTTTACTAAGATAAAAACAGGATTTGATAT
TTTGTCATTATCCTAAGAACTAAGTTTTTTTCTATGTATCTTATTTAATCTTGACCCCCAT
CGATTCACTTTTAATCATGTATTAGCAAATTTAATTACAAAAGGATTTTCTTAATTACTAA
AGAGCTTTAATTACTGAGGGCAGCATTATTCAAGATTTCAGATTATTTTCTTACCTCTCTC
ACCATGTATAATGCTACAATTTTATGCATTCTTCTGAGAACCATGGCACTATTTTTTTTCA
AATAAAAGGTTATTTTGATTACATATGTCAATGATAAAATAGAAAAAATAAGATTTGTTAT
AATAATTGTTGTGCATTTGTCCATGCACAATGTACAATGTATATTCTTGAAACATATATGC
GGTATTTCCAATTCTATGTCAAGAACAAGTAAAGCTATAGTTTTGTTTTTAACTTGGCCTA
CCTTCCAAACATTTGTGGGGAGGTTCAAGAGTTGAGAATGTGAAGATTGGAGAGTGACTGG
TATTTTCTTTCTTTTTTCTTTTTTTTTTTATTTTGAGACTGAATCTTGGTCTGTCGCCAGG
CTGGAGTGTAGCTGCGTGATCTCGGCTCACTGCAACCTCCGCCTCCCGGGTTCAAACGATT
CTTTCTCCTGCCTCAGCCTCCAGAGTAGCTGGGACTACAGGCGCATGCCACCATGCCCAGC
 
TAATTTTTGTATTTTTAGTAGAGACGGGATTTCACCACGTTGGCCAGGATGGTCTCAATTT
CTTGGCCTCGTGATCTGCCTGCCTCGGCCTCCCAAAGTGCTGGGATTGCAGGCGTGCACCA
CCGTGCCTGGCCTATATTTCCTTAAAAAGAAAAAATATATATATTTTGAATATTCAAGTAG
CTAAATAAGCATTGAATGAGAACATTATAGTAAGACTCCTTTCATTAAAAGTAAATAGAAT
AGTATAAACTACAAATAGAATAAAGTAAATAGAATAAGTAAATAGAATAAACTGTCTATGT
GGTAGAACATAGTCAATTTATACAGTAGAACATTGTTCTACAGCATAATCTATACAGATTA
CAGTATAATCTATCTACAGTATAATCTAAAAATAATCTATTTATTTTGATGATTAAACTTC
TTGCTACATTTTATAAGGCAATTTAAAATATGTTGTTAAAATATTATATTTAGGTGTCACA
AATTGTACCTTATTTAATTTCAGTATACAAATAGTCTTTTGCAGTAAATTGCAATAATATT
TGCATAATTATTTTATGTATGAAGCTGAAATATGCGTATTCTCAAATGTACAAATGAAATA
AACTAAGGTAGTTTTATTCAGAATAATAAATAAAAGAAAATGTTCAGGTAGAGGATTACAT
ATTTTTAAATTAATGTAGAAAATGAAATATTGGTAATTATGAGACTTATTCAATAATATTT
ATTGAGTGCTATGTGCCAGGACCACATAATTTTTAATGATAAATTTAGAGCTACCTGCACA
TATCTTTCTATAGTCTGAGAATGATATGAAAATAAAAGATCAGATTATGACAAAATAGCTA
ATAAAATAGATAAGCTGACAAAATGCTAAGATTAGAGAGCATTCTGGTCACCTCCTGTAAG
AGTAGCCTTATTTTTACTGAAAAGCAAGAAATGATATTCTAAGTAGGAAAAAGCTATTTTG
AAATGCAGAAAGTTTTAATATAGAAAGTACATTATGGAAAATATTCAATGAAAATACAGGT
TTTTCATAAAATGTCACTTATATGGATATCTTGCAGCAACCCCAGATAATCTTACCATATT
AATTAATTATTCAGGTTAATATGTTAAAAGTACTTTGGGGAGGAAGAATCCAAAATTATAA
TCAGATTAATATCTATAGTGTTATGTGTTTGATTGTGAGATCCAGGTGACCCCTGCAAGAT
GAGGAATTCATCATACTGTAACCTGAGTATCCAGTACTTAGTGGAAAGCAATTTCCAATTT
AAAGAGTGTCTTTGCACTGATGACTTCTATTGTACTGTGAACAAACTGCTTGGAAAAAAAT
GTATCAATAAATCAGGTAATATTTTGTTTTAAGAAATGTTTATAGTCATGAAAAAGAAAGC
AACAATTTCAAAATTAACCACATCTATGTAATGTGATTAATGTTTTTTGTAATTTGATTAA
TTCTTTTCAAAGCTTTATTATTTTAAAAATTTTGTTGGTATATAGTAGGTGTATCTATTTG
 
AGGGGTACATGAGGTGTTTTGATACAGTTCTTGATGTACCTTGGGGTTCTGTGAGCCATAG

ACCTCAGTAAGTGCACGACATACTTCTTTCATTACTAACCACAATTCTAAGCTTCATTCGA

GTCAGTGGAATTATAGATTCACTCATTCAATTCATATATTTGTGGAGTTTATTGTGGCCTT

GCACAAAACCTAGATGATAGAAGGCAAATAAGATATTATCTTTGTTGTTAAGGAACTCAGT

GTCTCCTGGGAGAAATCAAAATGTAACTAATTGCTAGAGTTATACTTGCCTTTGCATAGGG

ACATTTAATCAGGCTAATACACTGGAGTAGGGGTGATCAGTTAGCCTGCATGTACCCTTAA

CTGTACAACAAAGACATTCCAAGAGATGTGCGAGATCAAACAGTTCTAAAGTCAACAGTTT

CCAGTCTTCAATGTCCCTATATACTCCGAAAAGTAATCAGCAGAGGAATGCACCTGTTATC

ACAGAGTGCTCAATTCTTCTTTCACAATCAACCTTCTCTCACTTTACAAGAGGTAGATTTA

CCTCTCATCTACACGGAATGGTCTCATCGTCCAATCCAACCCATTCAACCATCCGTTGTGG

GCTCATTGGACAAATTTCTGTTCCATAATTTTGCCATTTCCAGAATATCAACAAATGAAGT

TATGCAACATGTAGCCGTTTTAATTCAGTTTATTTTGTTTAACATAATACATTAGAGATTC

ATTAATTTTTCTCATGTGTAAGTTTATTTTTCTTGAAGAGTAGTATTTCATTGTGTGAGTA

TATCACAGGTTGGTTCCATTCATTATCGAAAGTAAATTGCATTATTTCCAGGTTTTCAGGG

ATTAAAAATAAGGCTGCTATAAATATTCACATACAAGATTCAGACAAACATAAGCTTTACT

TCACTTATGAAATTGTGGGGTCACATGGTAAGTATATGCTTAATGTTACAAAAAGCTTCTA

AACTGTAATTCAGATTTAGTGTAATCATCAATGTATAAAAGTTCCTGTTGCTTCATATTCC

CAGCAACACATTTTTTTGCCATTCTAATATATAGTGGTACTATATTGTAGTTTTAATTTGC

ACTTAATTATAATGTTAAGCATATTTTTATATGTTGTGTGCCATCTATCTGTCTATCTTAT

TTGGTAAAGTATTTCTTCAAAGTTTTGTCCATTTTAAAATTGGTGTTTGGTTATTGCATTG

AGTTTTGAGAGCTGTTTCTGCAAGTTGAATAGATTTTTTTTAATCAGAATATGTGTTTTGT

ATTTTTTTAGTCTGTATCATTTAAGAGTGTCTTAAAAAACAGAATTTTAAAAATTTTGGTG

AAATTGAGTTTATCATCTTTTCTTTCATAGATCATACTTTTGATGTTGCATCTAATAAATA

TTTAATTCACATTAACAAAGATTTTCTCATAAGATCTAGAAATGATGTAGCTTTAGGTTAT

ATATTTAGGCCTATGTTACATTATGAATTAATTTTTGTATCCAGTACAAGATATAAGTCAG
 
ATTTTCTTCCCTGCAAATATCTAATTGTTCCACCCACATTTGTGGAAAATAATATAATTTT

ACTTTTCTCAAAAGTCATTTGGCCATATGTTGGTGGCTCTATTTCTAGACTTTCTATTCTG

TTCCATTGAGTTAATTCTGTACTTTTGCAAATATTTTATAGTCTTACTATAGCTTCACAGA

AAGTCTTGAAATCAAGTAGTATGATTCCATTAACTGTTACTTGAAAAATAATTTTGGCTAT

TCTATTTCTTTGTCTTTATTTTATTTTATTTTATTTTATTTTATTTTATTTTATTTTTGTT

GTGTTGCTGTAGTTGTTTTTGAAACAGGGTCTTATTCTGCCACCCAGGCTGGAGCATAGTG

GCCCAATCTTCACTCAGTGCAGCCTCTACCTCCTGGGTTCAAGTGATTCTCTTGCCAAAGC

CTCCCAAGTAGTTGGGATTACAGGCATGTGCCACCACACCAGCTAAGTTTTGTATTTTTAG

TAGAAACGGGTTTTCGCCATGTTGGCCAGGCTGGTCTGCAGCTCCTGGGCTGAATTGATCT

GCCTGCCTCAGCTTCCAAAAGTGCTGGGATTACAGGTGTGAGCCACCGGGCCGGGCCACTT

CTTTGTCTTTCCATAAACATTTTAGAATCAGCTTGTCAATGTCTGCAAAGAATCCTGCTGA

CATTTTGACTGAACTTACCTTGAAATTATAAACCAATTTACTATGTCGAGTGTTCAGATTT

ATTAACATGATATATTTCTTCATTTATTTATAACTTCATTGATGTAGTTCATCAGTATTTG

ATAGTTTTCAGTATATAGTTCTTGCACAACTTTTTAGGCTTCTACAAACACACTTCAGGTT

TTGGTGCTGTTACAAATGGTAATTTATTTTACATCTTAACTTTCTATTGATTATAGTGATT

ATATAGAAATACAATTAATTTTTCTATATGGACTTTATAGCCTATGACTTGCTAAATGCAA

TTATTCTATTTTTTTGTAGAATCTGCAGGAAATTCTAAATAAATATTCTTGTCATCTATAA

TAGAGTTTCATTTTCCCCTTTCCAATCTTTAGTCTTTCATTTATTTTCCCTGCTTTATTAC

CCCAACTAGAGCTTTCAATATATTAAATAGAATTGTTGAAAGTGGACATCTTACCTTTTCA

CCTGTCTTAGGAGAAAGGCATTCAGTTTTGTACCATAAAGAGTGTGTGTGTGTGTTCTTTG

CAGGTTCTCTTTATCAAGTGGAGGAAATTCCTTATTTTTGGTTTTCTTCGTTTTTAAAATC

ATAAATGTATAAATAAATGCTGAATTCTGCAAATACTTTTTTAGCCTCTATTACTATTAAT

TACTTCAAACATTTATCTTTTCCTTTTTTGGGGAGTATTTCTATTTTTCTGGCCATTTTAA

AATATATAGTAAATTAGTGTTAACTATAGTCTCCCTACTGTACTATCAAGTACTAGAACTT

ATTCCTTCTATGTTAATGTATATTTGTATCCGTTAACCAACTTCTCTCCATTTCTCCTCCC
 
CCGTGTCCTGCCAAGCTTGATCACATTTTTTCCTTTAGTTTTTTATATGGCGAAATTCATT

ATTTGATTTTTTACTGTTGTTCCAGCCCTTCATTTCAAGGCTAAATTCTGTTCAATCATGG

TGTATTATTCTTTTTGTATTTCTGGATTCAACTGCTAATATTTTGTTTAAAAAACTATGTT

CATAAAGTATATTGGCATGTTGATTTGTTTTCTTATAATGTCTTTGGTTTGGTATCTAAGT

TATGCTGGCCTGTAAAATCAACTGGGAAGTATTTCCTCCTCTTCAATATTCTGGAAAAGCA

CAAAAAGAATTGGTGTCATTCCTTCCTTACATGATCTATTTCATTAACCAGTAAAGATATC

TGGCCCATGCTTCATTAAAAAGATTTGAAAATATAAATTCAATGTGTTAACTTGTTGTAAG

ACTATTCTTGTTATCTATTTCTTCATGAGTGAGCTTTGGTAATTTGTGTCTTCCATTTGCT

GAATTTATGTTGGATTTTTATCCTTAAAGTTTTCAGTTTACTGTTTCTTTTTGGTTTCTAT

CTCCCTACAAAATTGCTCCTAGTATTTCCTAATTGTCCTTTTTAATATCTGTAGCAGTTGC

AGTGATGTCCTTTTTATGTTTACATATTGATAATTTGTATAATTTGTGTCCTTTCAATTTT

ACTATTCATTGTTTCTTTATGTGTATGTCTGATTTGATTTTAATGTGTTCTCCATTTTCTT

GCTTCCTAACATAAAACCTTGAGCCACTGATTTGAGATTTTATTCTTTCCTCATATAAGCA

TTTAATACTATGAATTTCCTTCCAAGTACTGATTTAGCTATAGCCACACATTCTGATATGT

AGTTTTAATTTAAATTTATGTCAAAATATTTTCTCCTTTTCTTTGTGACTTCCACTTTGTT

ATGATTTATTTTATTATTATTATTATATTTTGAGGCAGAGTCTCTCTCTGTCGCCCAGGCT

GGAGTGCAGTGGAGTGATCTCAGCTCACTGCAAACTCCTTCTCCTGGGTTCAAGCTATTCC

TCTGCCTCAGCCCCTGAGTAGCTGGGATTACAGGTGCTTGCCACCACACCTGGCTGATTTT

TGTATTTTTAGTAGAGACATGGTTTTGCCATGTTGTCCAGGCTGGTCTTGAGCTCCTGACC

TCAGGTGATCTGCCAGCATCAAACTTCCAAAGTGCTGGGATAACAGGCATGAGCCACCATG

CCGAGCCAGAAGGTTATTTAATTTCCTAATATCTGAGATTTCTCAGATATCTTCATTATTG

ATTTCTAGTTTAATTTCAGTATCATAATATAACATATTTTGGATGATCTCAATTCTCAATT

CTTAATTAAAATAATAATTGCCAAATATCACCTATACAATGTGATACAGTCTACACACATA

ATAAGGACACACACATACACACACAGACAAAGTACTAGAAGCCATAGAGTAAAAAATAATG

GTGACTCAGAAGACTTCACAGAATTGTTAGACTTGGGCCTTTTAGGGTAAGTTGGTTTCAT
 
TAGGTAGGCAAAACAAAATGGATAATTTAAGCAGAAGAAACAGAATGAAGTGGGGAGCATT
TGGGGAGCAACAATTCCCTTGTTAAGCTGGATACTAGAATGGAAAAGTATTTAACACTGGA
AAGAAATCAGCTATTTATGAAAAGGAGGAAGGCTGAACTTACATATCTTTCTAAAGAGATT
AGACTTTAAACCGTTGCTATGAGGAGCTATTACATATTTTAAATAGAGGAATGTATCATCA
GTTTCTCAAAAGTTATCACAGGCAATAACGTAGAGAGGATGGAATGGGAGGCAAAGAAAGA
TGCAGGTCAAGAATATCAGTTAAAAAGCCATCAAAATAATTTAAGAGAGAGGTAATGCATG
CCTGAACTAAGGCACCGGTCCTGAGAATTATAATAAAATATATACTTAAGGTATATTTATG
AATAATAATCAATAGGAATTGGTGACCAGTTGGATATGAGGAATTTAAAATGTATTAAGCG
TAATCTCCAGGTGGCTGGCTTGGGACAAGGGGATTGCTATACATTGAGCCAAGAAATGAAA
AATTGTACAGGACTGGGGACGAAAGTGATGAGTTGTATTCTGAAAACATTTCATCTGAGGC
ATGCTTTGGAATATTCGAGTTAGGAATGTTGGTATTTTTTTTCTAAATTTGCAATGATAAA
AAAATAGTCTAAAAATGTAAAGGAGCATGCCTCATCAAGCTGGCATTGGGAAGCAGGGATC
TTGAGGAGAGAAATCTAAAACAGAGATATAGAGGTTTATGAGTAGATAAAAAACATAAAGG
AGAGTAAGACTGCCTGGGAAAAACACAAAGGGAGGAGAAAGATATTTTCAGAAGAATTTTA
TGACCTATTTGAAGAAACCAAAAAGAAGTTTTTTAAATAGGAAAAGAAACTACAGAGAGTG
GAATTTTTGAAGCCAATGAAAGAGAGGACATGAAGAAAATTGTCAAAATTCTGAAAAGTCA
AAAAAGATAGGGACTGATAATGGCATTAATTTGCTACTTAGTAGATATTACTAGTTTTAGA
TTAATTAGGCTGAAATGTTAGCATGCTGAAATGCTTATTAAAATATTTAGTGAGTTTTTTC
AAGATGGTAAGACATCTGAACTTAATAGCCTAAATTGCCACTTTTAGCATATCAAATTTTG
ACAAAATATTGTATATCAACAGAACTATACATATTTAGAGCAGTTAAGTTGAAATCAGTTT
ACATACTAAAACTATTTTTTCCACAATAGAAATTTATTAAATGCTGTTATGTGGAAATATT
GATGAATCAGGCATACAAAAACCTTGCACTCGATGAGAGTTTCATATGGACAAGACCTAAA
GGAGAAAGCCTAAATATTCCCGATGGACTTCGTAAAGGACCTGACCTGGATTTTGTGTTTA
CAGAAAGCATTCAGCTGATTTATTATAATTCATGACAAAGAATTATAAAGAAATTGATAAT
TTAAATATGAGAAAGAACATGATAATTTAAATCTTACCATTCCTTCTATGTCAACTGCAGC
 
ATCCTCCACAGACCTTCTAATCATCACTTCAACAAAATGGTTTTCTCTCCATGCTTTGCAT

TCCCAAAATATTCTCCAGAGTACACAGACATGCACTTTTCTATTCTCAAGAAAATCAGTTT

TGTTATTTCTTTTAAAAAATATGCTAAAGCACCCAGCAGAATACCTGACGCTTAGCAAATT

ATTAGTAAATATATAACGTAAATATTTTTCATTAACTAAATATTGAGCATCTCCATGATGT

ATCCATTAATAAAATATTTATGTGTAAACCCTGGAGAATATAAAGAGCATCATCAAATGAA

ATGAGCAAACAAAAGTGGTTGGCAGCAGTTCCAAGATGGCAGAATAGGAACAGCTCCAGTC

TACAGCTCCCAGCCTGAGTGATGCAGAAGACGAGTGATTTCCACATTTCCAACTGAGGTAC

CAGGTTCATCTCACTGGGGCTTGTCAGACAGTGGGGGCAGGATAGTGGGTGCAGCCTACTG

AGCGTGAGCTGAAGCAGGGTGAGGCATTGCCTCACCCGGGAAGCACAAGGGGTCAGGGAAT

TCCCTTTACTAGCCAAGGGAAGTGGTGACGGATGGCACCTGGAAAATCGGGTCACTCTCAC

CCTAATACTGCACTTTTCCAGTGGTCTTAGCAAATGGCACACCAGGAGATTATATCCCGTG

CCTGGCTTGGAGGGTCCCACGCCCATGGAGCCTCGCTCATTCCTAGCGCAGCAGTCTGAGA

TCAAACTGCAAGGCAGCAGCGAGGCTGAGGGAGGGACGCCTGTCATTGCTGAGGCTTGAGA

AGGTAGACAAAGCAGCTGGGAAACTCAAACTGGGTGGAGCCCATCGCAGCTCAAGGAGGCC

TGCTTGCATCTGTAGACTCCACCTCTGTGGGCAGGGCATAGCCGAACAAAAGACAGCAGAA

ACCTCTGCAGACTTAAATGTCCCTGTTTGACAGTTTTGAAGAGAATAGTGGTTCTCGCAGC

ACAGAGTTTGAAATCTAAGAATGAACATACTGCCTTCTCAAGTGGGTCCCTGACCCCCAAG

TAGCCTAACTGGGAGGCACCCTCCAGTAGGGGCAGACTGACACCTCACACAGCTGGGTACC

CCTGTGAGACGAAGCTTCCAGAGGAACGATCAGACAACAACATTTGCTGTTCAGCAATATT

TGCTGTTCTGCAACCTCTGCTTCTGACACCCAGTCAAACAGGGTCTGGAGTGGACCTCCAG

CAAAGTCCAACAGACCTGCAGCTGAGGGTCCTGACTGTTAGAAGGAAAACTAACAAACAGA

AAGGACATCCACACCAAAACCCCATCTATACATCACCATCATGAAAGACCAAAGGTAGATA

AAACCACAAAGATGGGGAAAAAACAGAGCAGAAAAGCTGAGAATTCTAAAAATCAGAGCAA

CTCTCCCCCTCCAAAGGAATGCAACACCTCGCCAGCAATGGAACAAAGCTGGATGGAGAAT

GACTTTGACGAGTTCAGAGAATAAGGTTTCAGATGATCAAACTTCTCTGAGCTAAAGGAGG
 
ATGTTCGAACCCATTGAAAAGAAGCTAAAAACCTTGAAAAAAGATTAGACAATTGGTTAAC

TAGAATAAACAGTATAGAGAAGTCCTTAAATGAGCTGATGGAGCTGAAAACCATGGCACGA

GAACTACATGATGAATGCACAAGCTTCAGTAGCCGATTCGATCAACTGGAAGAAAGAGTAT

CAGTGATTGAAGATTAAACAAATGAAAGGAAGCGAGAAGAGAAGTTTAGAGAAAAAAAGAG

TAAAAAGAAATGAACAAAGCCTCCAAGAAATATGGGACTATATGAAAAGACCAAATCTACA

TCTGATTGGTGTACCTGAAAGTGATGGGGAAAATGGAACCAAGTTGGAAAACACTCTGCAG

GATACTATCCAGGAGAACTTCCCCAATCTAGCAAGGCAGACCAACATTCAAATTCAGGAAA

TACAGAGAATGCCACAAAGATATTCCTTGAGAAGAGCAACTCCAAGACACATAATTGTCAG

ATTCACCAAAGTTGAAATGAAGGAAAAAATGTTAAGGGCAGCCAGAGAGAAAGGTTGGGTT

ACCCACAAAGGGAGGCCCAGCAGACTAACAGCAGATCTCTCGGCAGAAACTCTACAAGCCA

GAAGAGAGTGGGGACCAATATTCAACATTCTTAAAGAAAAGAATTTTCAAACCAGAATTTC

ATATCCAGCCAAACTAAGCTTCATAAGTGAAGGAGAAATAAAATACTTTACAGAAAAGCAA

ATGCTGAGAGATTTTGTCACCACTAGGCCTGCCCTAAAAGAGCTCCTGAAGGAAGCACTAA

ACATGGAAAGGGACAACCGGTACCCAGCCACTGAAAAACTCATGCCAAATTGTAAAGACCA

TCGATGTTAGGAAGAAACTGCATCAACCAACGAGCAAAATAACCAGCTAACATCATAATGA

CAGGATCAAATTCACACATAACAATATTAACCTTAAATGTAAATGGGCTAAATGCTCCAAT

TAAAAGACACAGACTGGCAAATTGGATAAAGAGTCAAGACCCATCAGTGTGCTGTATTCAG

GAGACCCATCTCACGTGCAGAGACACACATAGGCTCAAAATAAAAGGATGGAGGAAGATCT

ACCAAGCAAATGGAAAACAAAAAAAGGCAGGGGTTGCAATCCTAGTCTCTGATAAAACAGA

CTTTAAAACAACAAAGATCAAAAGAGAGAAAGAAGGCCATTACATAATGGTAAAGGGATCA

ATTGAACAAGAAGAGCTAACTATCCTAAATATATATGCACCCAATACAGGAGCACCCAGAT

TCATAAAGCAAGTCACTAGAGACCTACAAAGAGACTTAGACTCCCACTCAATAATAATGGG

AGATTTTAACACCCCACTGTCAACATTAGACAGATCAATGAGACAGGAAGGTAACAAGGAT

ATCCAGGAATTGAACTCAGCTCTGCACCAAGCAGACCTAATAGACATCTACAGAACTCTCC

ACCCGAAATCAACAGAATATACATTCTTCTCAGCACCATATCACACTTATTCGAAAATTGA
 
CCACATAGTTGGAAGTAAAGCACTCCTTGGCAAACGTAAAAGAACACAAATTATAACAAAC
TGTCTCTCAGACCACGGTGCAATCAAACTAGAACTCAGGATTAAGAAACTCACTCCAAACC
GCTCAATTACATGGAAACTGAACAACCTGCTCGTGAATGACTGCTGGGTACATAATTAAAT
GAAGGCAGAAATAAAGATGTTCTTTGAAAACAATGAGAACAAAGTCACAACATACCAGAAT
CTCTAGGACACATTTAAAGCAGTGTGTAGAGGGAAATTTATAGCACTAAATGCCCACAAAA
GAAAGCAGGAAAGATCTAAAATTGACACCCTAACATCACAATTAAAAGAACTAGAGAAGCA
AGAGCAAACACATTCAAAAGCTAGCAGAAGGCAAGAAATAACTAAAATCAGAGCAGAACTG
AAGGAAATAGAGACACAAAAAACCCTTCAAAAAATCAATGAATCCAGTAACTGGTTTTTTG
AAAAGATCAACAAAATTGACAGACTGCTAACAAGACTAATAAAGAAGAAAAAAGAGAAGAA
TCAAATAGAAGCAATAAAAAATGATAAAGGGGATATCACCATTTAATACAGAAATACATCC
CACAGAAATACAAGCTACCATCAGAGAATAAACACCTCTACACAAATAAACCAGAAAATCA
ACAAGAAATGGATAAATTCCTGGATGCATACACTCTACCAAGACTAAACCAGGAAAAAGTT
GAATCCCTGCATAGACCAATAACAAGCTCTGAAATTAAGGCAGTAATTAATAGCCTACCAA
CCAAAAAAAAAGTCCAGGACCAGATGGATTCACAGTTGAATTCTAGCAGAAATACAAACAG
GAGCTGGTACCATTCCTTCTGAAACTATTCCAATCAATAGAAAAAGAGGGAATGCTCCCTA
ACTCATTTTATGAGACCAGCATCAACCTGATAACAAAGCCTGGCAAAGACACAACAAAAAA
AGAGAATTTTAGACCAATATCCCTGATGAACATTGATGCAAAAATCCTCAATAAAATACTG
GCAAACCGAATCCAGCAGCACATCAAAAAGCTTATCCACCATGATCAAGTGGGCTTCATCC
CTGGGATTCAAGGCTGGTTCAAAGCATACGCAAATCAATAAACATAACCCAGCATATAAAC
AGAACCAAAGACAAAAACCACATGATCATCTCAATAGATGCAGAAAAGGCCTTTGACAAGA
TTAAACAACGCTTCATGCTAAAAACTCTCAATAAATTAGGTATTGATGGGATGTATCTCAA
AATAATAAGAGCTATTTATGACAAACCCACAGTCAATATCATACTGGATGGGCAAAAACTG
GAAGCATTCCCTTTGAAAACTGGCACAAGACAGGGATGCCCTCTCTCACCACTCCTATTCA
ACATAGTGTTGGAAGTTCTGGCCAGCGCAATCAGGCAGGAGAAAGAAATAAAGGGTATTCA
ATTAGGAAAAGAGAAAGTCAAATTGTCCCTGTTTGCAGATGACATGACTGTATATTTAGAA
 
AATCCCATCATCTCAGCCCAAAATCTCCTTAAGCTGATAAGCAACTTCAGCAAAGTCTCAG

GATACAAAATCAATGTGCAAAAATCACAAGCATTCCTATACACCAATAATAGACAAACAGA

GAGCCAAATCATGAGTGAACTCCCATTCACAATTGTTTCAAAGAGAATAAAATACTTAGGA

ATCCAACTTACAAGGGATGTGAAGGACCTCTTCAAGGAGGACTACAAACCACTGCTGAACA

GAATAAAAGAGGACACAAACAAATCGAAGAGCATTCCATGTTCATGGATAGGAAGAACCAA

TATCATGAAAATGGCCATACTGCCCAAGGTAATTTATAGATTCAATGCCATCCCCATCAAG

CTACCAATGACTTCCTTCACAGAATTGGAAAAAACTACTTTAAAAAGTTCATATGGAACCA

AAAAAGGGCCCGCATTGCCAAGACAATCCTAAGCCAAAAGAACAAAGCTGGAGGCATCTTG

CTACCTGACTTCAAACTATACTACAAGGCAGTAGTAACCAAAACAGCATGGTACTGGTACC

AAAACAGAGATATAGACTAATGGAACAGAACAGAGCCCTCAGAAATAATACCACACATCTA

CAACCATCTGATCTTTGACAAACCTGACAAAAACATGAAATCGGGAAAGGATTCCCTGTTG

AATAACTGGTGCTGGGAAAACTGGCTAGCCATATGTGGAAAGCTGAAACTGGATTCCTTCC

TTACACCTTATACAAAAATTAACTCAAGATGGATTAAAGACTTACATGTTAGACCTACAAC

CATAAAAACCCTAGAAGAAAACCTAGGCAATACCATTCAGGAGATAGGCACGGGCAAGGAC

TTCATGTCTAAAATACCAAAAACAATGGCAACAAAAGCCAAAATTGACAAATGGGATCTAA

TTAAACTAAAGAGCTTCTGTACAGCAAAAGAAACTACCATCAGAGTGAACAGGCAACCTAC

AGAATGGGAGAAAATTTTTGCAATCTACCCATCTGACAAAGGGGTACTACCCAGAATCTAC

AAAGAACTCAAACAAATTTACAAGAAAAAAACAACCCCATCAAAAAGTGAGTGAAGGATAT

GAACAGACACTTCTCAAAAGAAGACATTTATGCAGCCAACTGACACATGAAAAAATGCTCA

CTATCACTGGCCATCAGAGAAATGCAAATCAAAACCACAATGAGATACCATCTCACACCAG

TTAGAATGGCGATCATTAAAAAATCAGGAAACAACAGGTGCTGGAGAGGATGTGGAGAAAT

AGGAACACTTTTACACTGTTGGTGGGACTGTAAACTAGTTCAGCCATTGTGGAAGACAGTG

TGGCGATTCCTCAGGGATCTAGAACTAGAAATACCATTTGACCCAACCATCCCATTACCAG

GTATATACCCAAAGGATTACAAATCATGCTGCTATAAAGACACATGCACACGTATGTTTAC

TGCAACACTATTCACAATAGCAAAGACTTGGAACCAACCCAAATGTCCATCAATGATAGAC
 
TTGATTAAGGAAATGTGGCACATATACACCATGAAATACTATGCAGCCATAAAAAAGGATG
AGTTCATGTCCTTTGTAGGGACATGGATGAAGCTGGAAACCATCATTCTCAGCAAACTATC
CCAAAGACAAAAAAACCAAACACCGCATATTGTCACTCATAGGTGGGAATTGAACAATGAG
AACACTTGGACACAGGGAGGGGAACATCACACACCGGGGCCTGTCGTGGGGTGGTAGGAGG
GGGGAGGGATAGCATTAGGAGATATACCTAATGTAAATGATGAGTTAATGGATGCAGCACA
CCAACATGGCGCATGTATACATATGTAACCAACCTGCACGTTGTGCACATGTACCCTAGAA
CTTAAAGTATAAAAGTAAAAGAAAAAAAAAGTTGACCATATGTTGAGTCAAAGCAAATATC
AACAAATTTCAAGACACTGAAATAATTAGAAACATGTAATTTTATCTTGGTGGAAATAACC
TAGATATCAGTACAAAAAATAAACAAGAAATCCCCCCCCCCCAAAAAAAAGAAAAAACAAA
GTAAGTGATGAAGAAAAATATCACCAAAAGAAATTGAACATTAACATTATGTGGTTCTATA
ACCTGTAATATTGGTGGTAATTAATTTTTTAATTTGTTGGGATGCTAGTTAAGTTGATAGT
GTGACTTTTCTGTGAGAAAACAAAACAGTATAAATGAGCAGATAAACCACTCAGTAAAATT
TTATAGGGTTGGAGTTTTATAGAGAATCACGGGAAAGTAAAAGAAGAAAGGACGAAAAGAC
AATAACAAGAAACTGTTCCTTTATCTGAATGGGTGAGTGATATAGGCTGGAAAGAGTCTAT
GAATCCAGGAGATGATGCTAAGATTGGGAGTGAATAAAAAGATGAAAGTTCTGGAGGTATA
TGTGCACATGAAATAATCAAGAATGTGAAGCTTTGGAAGCGAGTAAGATAAGTAATATTTG
CAATTGAAAGAGAGAGAACTTCATGTCAATGTCAGGTCTAGAAGTCGATCATTGGTGTGGC
ATAGTTGGAGTGTCGGTGAAGGTCATAAGAGCTATGGTTAAGGAATTCTGAATTAAGGGTG
GAAGATGGTTTTGTCCCAAAAGACATAGAGATAACTTAGCACAATGACAAGATCTGAGGTA
GAGGAGAATATTGAGACAGTACCTGAGTCGATGCTGAATATCAGGGCAAGACTTTGAGGTT
AATATTTGACAAAAGAAAAGAGGGAGGCACAACCCGACAGCATAAAATTCAAAGAAGGAGA
AATGTTTTATAAGGATGAATAATTAATATTTTGGAAGCTACAAAGCTGGAAAAGTAGATAT
AAACACATTCTAATGATATCGTGGTATGATCTATATGGGAAATGGGTAGGATTCACTAGCA
AGAGCTACAGGGAAGTGAGGCTAGAGAAAATCTAGGCTTCCCAGATATTACACTTTTCTAT
TTTTCTTCTTGCTTCCATGGGTGATTCTTCTCAGTCTCTTTTGAGCATCTTCTTTTGCTTT
 
TGCTTTAAATGTTGCCACATACCCTCTAAAAAAGAAATCTCTGTTGCATTTAATTCAACTA
GTCATTTTATAGAAACATGTTTGTCTACTAGTTTATTGTAAGCATATTTCTTATCAGATTA
TAAAATATAAATCAAAATATTTACATATACAAGAGTTTAGTTTCTGATATTATCAATGTTA
AGAAAAAGAAGATGTATGGAAGTACTTCTGATTCTAATATTAGATATTAGAGCATATTTTA
GGGATTGTTGGATATTTTCTGATGAACTGTATAGGATTTTATAACTCACATGTAGGTCTTA
TTGGTGAATACTTTTATTTGTTTCCTGAACATATTGACCTTCAGCTCCTCTTTCAGTAAAA
GCCACTGTTTAAAATATCCTCTGGGAAAAAATGAGTGTGTGTGTGTGTGTGTGTGTATGTG
TAAATGCTAGTTTTATATTTAATGTGTAAATGTTAATTTCATACAAAATTAGCATTTAATG
TATAAGTATTAAAATGTAATACCTTCTATGAAGCACTTTATTTTCAGACACTAAAAATAAC
TGTTTTGTTGTGTTGGTAACAAGTACTCATATAGTAAGTAAATTAGGCTGCTCTGGCTACC
ATAACGAAATACTACGGGCTGGGTGGCTTAAAAAGCAAAAATTGTATTTTTCCCCAGTTCC
GGATTACGAAAAATCTCAGATAAAGGTCCCCAGAATCTTTTCCTGGCTAGGACTCTTCTTT
TACTAGCTTGTAGATAGCCAGCCACATTTTCCCTGCGTTCTCAGGACCTTTCTTCAGTGAG
TACTCCAGGGGAGAGAGACAGCTAGCTCCCTGAAGTCTTCTGACATGGACACTAATCCTAT
TCGATCAGGGCCCCACTCTTATGACCTCTTTTAACCTAAAGACACCATCACCCCATAACTG
CACACGGAGATTATGGCTCCAACTTATGAATGTAGGGGTAATGAGAAAAACATTCAGTCAA
TAACAGTGAGTGAAGCTGTTGTTTATTTAGGTCTACACAGTAGTTTCTTGAATATATTTTA
GTCAGTGGCACAGCCTTACCAATCATTCCCAATGCAGGGGTACTAATCCATAAAAGAATGT
CCAGCCTAGACTTTTGTACCTGTTACAAAAGCTATATGACTCCCTGGGATCTGTTTGTTGG
GATTTTATTAGATATGGAATAGTAGCACAGAAGTTGGAACCTCCAGATCTTCCTCAGAGAG
GCGGGATGATGGGTGGGATCAAGAAGAAAGAGTGGTTCTCAAGCTGAGACTTGATCAATTA
AAAAAATGAAAAACAATGCATAATGAGAATAGCTTGGACAATATAAGGAGTCTGGAAGGCC
ATGACACACCTGGGGGTAGAAGTAAGTTTCCTGTGATAGTACCATCTTATATGTGCACTAG
ACTAGCAGGCAGGAAATATGAACAAACAGCTCAGATTAGGACATAATCATGAAGGGACATT
TATGCCATAACAAAACTTATTATCTTAATATCCAATGAAAAAATAATAATTTTCATTTCAA
 
AATGAAAATCTTATCATCTGATTCCCCTGCCACTTTATGGATACATTAGTGCTCCTTATAG
TTTCTTTTGGCTTTGTTTTATATGTCTGTATTTCCTTCTGGGCTGTGGGATGCTCGATGAC
AGGGACTGCAGATTTGCATCCTCAGTTTGTCACCTGGTACATAGTAGGGAACCAACCAATG
TTCCTTTAAAGATTTTGGAATTAGATGAACACCGAGTTCATTTTCGTTGATGAGATTTTTC
AATACTATAATTATAATTTCTAAGAACTCTTGGAGGATAAGATAATCAGAGGTGAAACTGG
TTTTAAAAATTGCATTTCATACTAAAGAAGACCCTTCTGAGAAGGGTTTTTATGCTGGGCA
GGGTGGGAGGTAGTGTTCAGAAAATATTCCTTCTGTTTTTTTTTTTTTTTTCCCCAGTCAG
CATCCAATGTTTGTTTACTCCGTGCACACAGTCTAATCACTAGCATTTATTTAAGTCTTCA
AGTAACTGAATCCTCAGAGCAGGGCTGTATAGCTCACTTGAATGATATTTCAGAGGCTTGT
TTTCTTCATCTATACCACATTAATTTTGAATATCAAGGGCTAAATACATGTTACATAACCT
TGTATGTACTTTTGCTTGAATATATGTGGACTTTACTCATTTATAAATGTGGCCCACGTTT
TCCTAGGAAATTCAGAAAATTGTTCAATAATGAAATAATTTCTTTGTTTTCCTTCTAGATA
ACGTGAAAGAGGATAAATTCAAATGGAATCTAACTACACGTTCCCATCATGGTAATGTTTT
TAAGTTATTATTTTGATCTGCATTGCAAATACCTGATAATATAAAATGCAGTTACCAGGCT
TCTTAAAGATAGGCCAATACAGAACAAATCATGTGGTTTAACAGTTCTAATATAATAAAAT
GCAGCAAAATCATTTCATTTCTTTTACTAATGATGCATCAAAGCAACTACTTTGCCCATCC
ATTATCAATTTATACATGCGCTAAGAAATACTTAATAATTAAAACCAAATACTAGGTTGGG
CACAGTGGCTCACAGCTGTAGTCCCAGTACTGTGGGAGGCTGAGGTAGGTGGATCATTTGA
TCCCAGGAGTTTGAGACCAACCTGGACAACATGGCAAAACCCTGTCTCTACAAAAACAAAA
CAAAACAAAAACAAAAACAAAAGTCAAAACTTAGCGGGTCATGTAGGCTTGTGTCTGTGAT
CCCAGCTCATTGGGAGGCTGAGGCAGGAGGATCACTTGAGCCCAGGAGTTGGAGGTTGCAG
TGAGCTGGGATCATGCCACTATACAACAAGACCCTGTCTCAAAAATCAAACAAACAAAAAT
CCAAATAGTAGATAGTGATTACATTTAATAGGTATTTGTGATCATATAAGTTATTTGGCAC
CAACTGTTTTTGTGTATACTTGTGTATCTGTAGGAGTATAAACATTTATATATGCTAGAAT
AAATATGTGGAGCCTTTATTCAAGATTCCCCTCACAAAAATGTGCAACAGAACAAGAATCA
 
TTTTGCCTTGAGCCAATGAGCTAACCATAAGCTACATAGTTCAAATGCAATTTCTGAAGAT
TTATCGTTTACATTTGAAAACCTTGGACTGGGAGATTTTTCCTTCAGCTCAATTTTGGCCC
AGCACGCTTCCCATCACTCTAGATCACAATGTGTATCCTGTATAGATGAAAGATGATTCCA
ATAAGTCTACGATTTAGGTCTCAGTAAATTCATACTTACTCTAAAAGATGTAATGTTTGCC
AAAAATAGGACATTGGTACATCATTGTATTTTGCTCATAGACTTCTCACAGTGTTTTATAG
TTTCTGTATGTACAGCTTTGTGTTTACTTTTCCACGACCTGGGCATATCATTGCTTTCAGG
ATTCAAAGGGATGTGGTCCTGTTTGGAAGTGGCAGAGGCATGTGTAGGGGATGTGGTCTGT
AATGCACAGTTGGCCTCTTACCTTAAAGCTTGCTCAGCAAATGGAAATCCGTGTGATCTGA
AACAGTGCCAAGCAGCCATACGGTTCTTCTATCAAAATATACCTTTTAACATTGCCCAGAT
GTTGGCTTTTTGTGACTGTGCTCAATCTGATATACCTTGTCAGCAGTCCAAAGAAGCTCTT
CACAGCAAGACATGTGCAGTGAACATGGTTCCACCCCCTACTTGCCTCAGTGTAATTCGCA
GCTGCCAAAATGATGAATTATGCAGGTGGGTAAAAACACTCATACCTTACTTTCTTATTTC
GGCACCTTATTTGTTATAGTCCAGTCCTAACACTTGATCTCATAACATTAGCTAGAGTTCC
CACCATCATCCAACATAGTGTAAATAAAACCAGACAATTAATATTTTTTTGAATCTGTGGT
ACATATGCAGTATCATCTGAAATTCAAATGCAGAGTAGATTTTTGAAATAAAAAATCAGCA
TGTCAAAATATGGTAGCCATGAAATAATATTATAAAAATTATGTATTCAAGTTAGACTAAT
GAATTGGTTAATAGACCTGACTTACATATCAGCTTTTTAATTTTTTTTTTGTTGTTTGTTT
AATTATGTAAGGCTGTTACAAATTTATTATTTTGGCAAGGAGACTCTTCAAACTTTGAAAT
AAATTCTTTCTGTCTTAGGCTGGAGATCAGGAGGAAGAAGGTAGCATAAATACTCACATGA
GTGGTGCAAAGTCAGAGATAACCAGGTCCACACACATTTGTGTCTTTTCACAATGTCAGGC
TTTAATACTGTTCAATCACAAAAGCCACAAGCTACATGGAGTTCCCAAGGAGGCAGTTCTC
CTTAGTACTTCTCATTCACTTTGTTTTCAGAGCCATGGGTACACAGGCTCAAGCCACTCCA
CAAATCAGTCAATATTGCAAACCATACATAATAATATACTTAATATATAAGGATTATAGGT
TAAACATTCCACAACAAAGTAACATTTAACATTAAGAGAAAAGGGATAGAGAAAAGGGTTA
ATGAAAAAGTCCAGGAAGAGTGACAAAAACAAAGATTCTTCTGGTCTTATCTGGGCTGTCC
 
ATTGATCTTGCAAGGAAGAGTCTGATGTGGGGAGAATTGTTGGCAGCAGATGCTGGGTGCT

TATGAGCGACAGCAAGATGGTGTCAGGTAAGATGGCCATTTTGAGCTGCTGAAGTCCTACT

CTCTTTATGGCCACAGAGTCCTCTTGAGAGGACCGATAGTGGAAGAGTATGCTTGTTTATG

TCCTTATTTGGTTGGATGCAGTATTGTTTATTTAGCAAAATATTTTGTCCCTGTTGGAAAA

GTACCGTATGAAATCTAAGATGGAGTCTTTTTTGGAGTTACTTATGTCAAGGATGCTCTAT

ACAAGTAGGATTTGGACATCCCTAGCACACATGTATGCTATGACCATAGAAATGTATTCAT

TCTCCTCTTTCTAACCTCACACCTTTAATTATTGGGGGAGTAATGTTAGTCGTTTATTTAT

TAATTCAAAAAAAATTTATCAAGCACCTACTACTACTCTAGGCAATGGCGAATAAGGAAAA

CAAATGCCTATGGATCTTATAGTCCAGAGAGAAGACAGATATTAAATTAAAAATGTATGTG

TTGTTTTAAGGCAAACAACACTAGGCAAACAGTAGATACTGAGAGAGGAGATATTAAGCAA

ACTGACCAGCCAGTGCTTAGGAAAACATGTATGTGGCATTTTAGAAAAGCAGTGAGGGTAG

AGTGTGCAAAGGGGAGAGAAATAGGAGCGAGATAAGAGAGTTAATTGAGTGGGTGTGAACA

GGCCTATATCATATGAGGGCTTGAAGGACACTGCCTAGGGCTTCGGACATGTAGGTGGCTT

AGTGTGTGATTTGTCATATTTGTCCCCTTGTTTCAACTACTGGCAAAAAAACACAAAAAGT

AATGATATCAACTTCCTGAAGGTTCTTAGTAAAATTCTCTCTTTTAGCTTTTTAACTATGT

CTACTCTTATTTTCAGTCACTCTAACTGGCAAAGTAGTTCCAGATCTTGTAAACATTTGTT

AAAAAGAACTGCTTCCCTATAGCACACATTCTATGAAATAAGTGGTAAAGAGCTAAGCAGA

GTCCTAGAGCATTGACATATTTCGTCAACTTATAGGATATCAAGGTTCTTAAAATTGTATT

CAAGAATTTTTCATGCAAATGTGTCAGTTATTTTAAGGCTTATTATTTATTTTTCTTGAGT

GAATAAGGCATTGACAAAATACTTTTTATAATGCAATTTAGGTTTTTCAAACTCATTTATC

ATCTCAAAAATCCTTATTAGAGAAATACTACATTTCATTGATTCTAAGATGCAATTTCTTT

TTACACATTTTAACATCTCTGACATATTTTAAATAAATTTTTAAGGTCAATAACAAAGACA

TTTGTCATAATTTAAGTTCCAGCAGGTGTTTTTTGTTGTTATTGTTGTTGTACATAAAATA

ATGGTATCTTCTGAAATCAATGAAATATGAAATCAATGAAATATCAAACACATGTGATTAC

ACTACTTTTCTCACCTCAAATGAACTAAAACTATTACTATTCTGCCTTTCTAACTATCATA
 
TTCCTACATCAACAATGAAACCTAATAATATTTTAACCAGCCAGGGCCAGGGGCACCATGG

TTTCAATAGCAAAATCACTATAAAAATATCAGATTTATTCACAGATGGGTACCCAGCTTTG

CCTGATTCCAGTGCCAGGGCTTTCGATATGCTATCTAATTATCTGGAAGCCATTAGCATTC

AACCTTCAAAGCATGGTATTTCTGAGCATCCACATCAAAGAAAATATTTTAATAGCAAGTG

GTGGCTCTGCTGCTATAGTCCTTGAGAATTCCTGTGATAGACTCTGAAGCACACACTCTTA

CACTAACCTCCCCTTGGTATCAACACCTTCTCAGATGGACTGGCACAGAGTTGATGATTTT

CCTCAACAAGGACCCCCAGCTTTAACCACCTTTTCAAAATGTTTGGTGTGGGGTCTGGAGT

GGGATAAGCACTCAAAAAACGTCATCTCCCTTCCTCTCACTTTTGAATATTGTACACTTAA

TTGTTATATTTTAGTATACATATATGTATTTCTTCTCATGTAATTTATTTCTTAGATTAGT

TCAAGTGTAAGGACTGTGTGTCTTCAATTTCCTTTCACTATGTGCAAATAGTCCCTTAATC

AGTACTTTATAGAGGTGATGGAAATGTTATATAATATTAGTAGTCAATGTGAAAAGACAAA

TCCTATCATTTTCCTGAGCACATTTTGCCTTAGGGAGATTTCCAGCTTTTATATTAAATTA

TTGTTAATGTAATTCAGATGAAAAACAAATCTCCAAATGGAAAACCACTTCTTCAATGAGA

ATATTAAGCCCTTTATGATACTTATCTTTTTTGCAGAAGAGCCTGTACATAGGCAAAACAG

GGCACTTCAGTTTTAAAATACAATTTCTGTCCCTGTTCCAAATTTTTCAATTCACGATGGG

CTTTGAAATTCCTTTATTTTTTAACTGCCAAAGAGAACAATTTAAGGGCCATTTCTCAATT

ACCTTTCTCTAGTACCGACACCCTCAGGGAGGTGAGATTATGTCTTGCCAATTTCACAAAA

GTCTGGGTATTTACAGAAAATTATAATACATTATATTTTTATATATTAACTTAAAAAAGTA

ATGACAAGGTATTTGCTTAGGAAATATTCTAAATACTTTTAAATATGTATGCATTATTTAA

TTGATTTAAAACAGATGACATCATATTATATACTTCTCTATATATTGTTTTTGTTTTTTTT

CCCTAAATGTGGATTAAAGATCTTTCCATAATTGCATATAAGAAAATTCACACATCAGGGT

CTGTTAGGGAGTCGGGGGCGAGGGGAGGGAACTTAGAGGACAGGTCAATAGGTGCAGCAAA

CAACCATGGCTCATGCGTACCTATGTAATAAACCTGCACGTTCTGCACATCTATCTCATTT

TTCTTAGAATAAATTAAAAAAAAGAAAATTCTCAAATGTTTGAATGGCTATAGAGTATGCC

ATTGCATGGATGTAGCTTAATTAATTTGCTTAGTCTTTTTTGGACATTTAGACATATTCCA
 
AGTATCTTATTTTATTTGTAGCTATTGTAAATGGGACTACTTTGAAGATTTGTTTTTCAGA

TTGCTTGCTGTTAGCATATAGAAATGCTACTGATTTTTGTATGTTAATTTTATATCTTGCA

ACTTTACTGAATTTGTTGATCAGTTCTAAGAGTTCCTTGGTGGAGTCTTTGGGTCTTTTCA

AATATAAAATTATATAATCTGCAAACAAATTTTTCTACCTTTCTAATTTGGATGCCCCCCT

TTTCTTTTCCTTCTCTTGTCTAATTGCTGGGCTAGGACTTCCAGCACTATGTTGAATAATA

GTGATGAAAGTAGGCATCCTTGTCTCATTACAGATCATAGAGGAAAAGCTTTCAGTTTTTC

CCCATTTTATATGATACTAGCTATTGGTCTGCTCTGTCATATATGGCATTTATTCTGTTGA

GGTATATTCCTTCTATATCCTGGTTTTGAGGCTTTTATCATGAAGGAACGTAGAGAACTTT

AGCAAATGCTTTTTCAGCATCAATTGAAAAGATCATGTTTTATGTCCTTCATTTTGTTGAT

ATGATGTATCAAACTGATTGATTTTCATATATTGAACCATCCTTGTATCACTAGGATAAAT

CCCACGTGGTCATGATGAAAAATCTTAATGTGTTGTTGCATTTGGTTTGCTAGGATTTTGT

TGAGGATTTTTGCATTAATGTTCTTCAGGAAAATTGTCCTGTAGTTTACTTTCCTTGGTAT

GTCTTGTCTGGTTTTGGTATTAGGGTGATACTGGCCTCAAAGAATGAGTTTGGAAGTATTC

CCTCCTCTATTGTTGGAAATATTTTTAGTAGAATTGGTATTAGTTCCTCTCTAAGTGTTTG

GTAAAATTCAGCATCATGTCCCAGGTTTTCCCTTGCTGGGAGACTTTCTATTATAGCTTCG

ATCTCATTACTTGTTATTGGTCTGTTCAGGATTTGGACTTCTTTATAGTTCAATCTTGGTA

GGTTGTGTCTACAAATTTATTCACTTATCCATTTTAGATTTTCTGATGTATTGGCATATAT

TTGCTCATAGTAGGCTTCAATGATCCTTCGAATTTCTGTGGCATCATTTGTAATATTTCCT

TTTTAATCTCTGATTTCATTTATTGAGGTCTTCTGTTTCTTTTTATTAGCCTAGCTGATGG

TTTGGAGATTTTATCTTTCCAAAAGACCAACTTTTCATTTTGTTAATCTTTTGAATTTTTT

TTGTTTCACTTTCATTTATTTCTGCTCTGAAGTTTATTTTCTACTATTAATTTTGGGTTCA

GTTTGCTCTTGCTTTTCTAGTTCTTTAAGATGCGTTGCTAAGTTGTTTATTTGAAGTTTTT

CTACTTTTTTGATGTAGGTGCTTATTTCTATAAACTTTTCTCTTAATACTGCTTTTTCCAT

ATCCCACAGGTTTTGGTATGTTGTTCTTCCATTTTCATTGTTTTAATACACCTCTAAATTT

TACTCTTAATTTCTTTATTTGCACACTGGACAATCAGGAGCATATTGTTTAATTTCCATGT
 
ATTTGTATAGTTTTCAAAGTTCCTCTTGTCATTGATTTCTAGTTTTATTCCATTGTCATCA

GAGAAAATACTTGATATGATTTCAAGTTTTTGAAAATTTTAAGACTTGCTTTTTAGCTAAC

ATATGGGTTACCATTGACAACGATCTGCGTGCTGAGGAGAATAATGTGTATTCTTCAGCTA

TTGTATGAAATGTCCTGTAAATATCTATTAGGTACATTTTATCTATAGTGCAGATTAAGAC

TGATGTTTCTTTGTTGATTTTCTGTCTGAATGATCTGTCCAATGCTGAAAGTGAGATGTTG

AAGTCTCTCTCTTTAGGTCTAATACTATTTGCTTTACATGTCTAGGTACTGCAGTGTTGGG

TGCATATATATTTACAATTGTTATATCTTCTTGCTGAGTTGAATCTGTTATCATTATATAA

TCACCTTCTTTATCTTTTTATAGTTTTTTTCTTGAAATCTATTTTGTCTAATTAAAGTATA

GCTACTCCTGCTTCTTTTTTTGGTTCCATTGGCATAGAATATCTTTTTCCATCCCTTTATT

TTTCAATCTATGTGTTTCTTTATAGATGATGTATGCTTCTTAGAGGTAACAGATCATTGGC

TCGTTTTATTTTTAATCCGTTTAATCCATTCAGCCATACTATGTCTTTTAATTGGAGACTT

TAGTCCATTTACATTTCAAATAATTTTGGAATCCAGTGTACCAAAGTAGTATTTTATTTTT

AATTAAATATTCTTTAGTTCATCATTTTGGCAAAATTTCATGGTGTTTTTCCTGAGTGAAA

ATTTATCTACTTATGTATAAGTAGATAAACCATGCAATTTAAACTTAGTAAAACATATTTA

AAATGAATAAATAGCATGTAGGTACAGATGTAGTCTCTATCTGAATGACAATCAATACTTC

CAAAACTTACATTTAGAAGATATCTTATATGTAACCCTTAACAGCATTGAAGGAATATGTT

TGTTCTTCTGACCTAATAATTACATGTTCAGAAAATTTGCAGTATTTCCTTTATTATAAAT

TTAATATACATGAATTAATCTTCATTATTATATTTAAAAGCTTATCCTTTAAGATGAGTTA

AATATTTAATTATTAAAAACAATGTGACTTTGGTTTTTTAACATAGCAGCATCCATGTGGA

CAAATAACTTCATGAAAATGTCATGTACTTATAGGGCTTTCATACATGTTTGATGTCAACC

TATTCAAGAAGAGTAAAAAGCAATTTTCGGTGACAAACATTAAATTGAAATAATTTTATTC

TTGCATTCATTAATAAAAATATATTCTTATTGTAAAAAAATTCAAAAATGAAATTAGTGTA

CATTATCCAGACTTTTTTTCAATTTTATTACAGCAATAATCAAATACATACAGAAATTTTA

TACCTCACAACATATTGTAAATTCTAATTAGTCTGTTTAGTCTCTTGAGAAAGTATAAAGC

GCCTCCCATCCTGTGGGATAACTGACTTGAAAAGTCAAATACCTTACTTGAAATCACTTGG
 
TGTGACACAGTGGCCAAAGGCTAGCCACAAAACTATATTTGGAATAAAAAATTGCCAATAA
ATCTCTGAAGCAGTTCATGCCTCAAATAGTCATACATTGGAATAAAATTAGTAATAAAGGA
AAATCCATCCGTGGAATCCTCAACTTAACTGCTGTCTTTCATCACAAAAAGGACAATAA
TTCACAATATTACTCTACAATTTCTTCCACACTACCTATGGTGATTTTTCAAATCACAAAT
TTACTTTTCAACTATGTACTGGCATCTCGAAGGCATTGTGTTCTATGTCTTTCATTAGGTG
AGGGGGGATCACATGTTAATGTGAAATAACACTATTCAAAACTAATTATTTTTCTTCACTC
TTTCTCTAGGAGGCACTATAGAACATTTCAGTCAAAATGCTGGCAGCGTGTGACTAGAAAG
TGCCATGAAGATGAGAATTGCATTAGCACCTTAAGCAAACAGGACCTCACTTGTTCAGGAA
GTGATGACTGCAAAGCTGCTTACATAGATATCCTTGGGACGGTCCTTCAAGTGCAATGTAC
CTGTAGGACCATTACACAAAGTGAGGAATCTTTGTGTAAGATTTTCCAGCACATGCTTCAT
AGAAAATCATGTTTCAGTAAGTTCCCCAAATAAAATTATCTGTCTATCTATCTATCTATCT
ATCATCTATCTATCTATCTATTTTGTTTTTGCCTATACAGTAAAAGAGGTAATCCAGCACA
GACATTTTTGTGTTTGGTATTGCATTTGAAAATTTGCTTTCTGATTATGTTCTGTGTATTT
GCTATTTTACTCACTTACTTGAAAATAGTACATTTTAAAAATTTATATGATAGGTCACTCT
TAATTATCATTTATTGATTTCAATATCAAAAGCATAGAAGGTTTATTCTATCAGATAAATG
GATATATCAAGTATCAATTGCTGCATAACAAACCATAAGTATCTATACTAAGTAACAAAAC
AATGGCTTAAAACGATAACCATTTATTTGCTCACAATTCTGCACATTGGACTTGGCTCAGC
TGGATGGCTCTTCCGATGGTCTTTTGGGGGGTCACTCATGTGCCTATAGTCATCTGGTATC
TTTAGGCTCGGCTGAGGCTGTTTGGTCCAAGGTGGTATTAGCAGACACCATTTTCTGGTGT
GGTGTGGTTCACTTCAATATGACATCTTCAGCAATATAGCCAAAAACTCTTATGTGGAAGC
AGAAGAGTTCCTAATGGCTAAACCCCATTGTGTAGCTGTCTAGTATCTGCTACTGTCACAT
TTGTAATTATTTCACTGAACAAAGCAAATCACAATGGCCAACCTCAGAGTCACTGTGGGAG
TGGACTACATAAGGATATGAATATTCAAGGTCAGATTAATCAAGGATCATTACTCTAACAA
CCTACTATGATTGGCTGTCCTGGCCTATGTACAATATATTCACTCTTCAATTTAGTTTTTC
TCCCCTTGGTCTTTAACATGTCTGCTGATCAACCTATAATGATGAAATGGAAAACATATTT
 
TCCGAAGTTATTGTTTAATTTAGGGACAAGAAAATTGATATTTAGGAATTTAAATAATTTG
TTCAGCATCTCAAAGTGAGCTAATGGTCAAGAAGGACTTTCATGAGTCTTAAGGAAACAAC
CTAAGGATGTGATCATGTAATAATTCAAAACCTCTCTATTATCCATCTTCAGGGTTAGACC
ATGCAACCTTCGACGTCCCCAAACTTCCCCAGGCCCCTACCCCTAGATCCCTAGCTGTAAC
TACCATATCTGCAGGTACTGTTTCTAGAAGGAAGTAATGGATATTTACTGTTTCATAGCAA
ACCAAATCTTTATATTTATTGAAAATGTTTGACAGACATAGATCTATATCTACATCTATAT
CTATATCTATATCTGTATCTATATCTATATACAGATGCATATTTACACATTTATATTTCTA
TTTTTCTTTACAGATACTTAGGAGATGCACATTAGCCAATGTGCTAAAAATTGTGATTTGT
TGTATATTATGTAATAGGCATACAGCTCAGAGTTTATGGTTTTGATTTCGACAGTGTTTTC
TGTGGAATATCCACAATTTCTATAATTAACACTACAGATATATTTCAACTGAAATCACGAT
ACAGAGAAGAATTGAAGTGTTATATAAGTCATATTCTCTAATTTTCCTCTCTTATTAACAT
TAGTTCTGTTGAAACTGAAAATACTTATAATGAATGGCTCATATGATTACAGCAGGAAAAT
ACCAAATAACCAATTTGGTATTAACCAAACTAAACTTATAGTAATATATACAGTTATATAA
TAATGGTTATTTATAGGTTCTTTCTAATATTACAAGGTATGAAGAAAAATGTTGATTCTAC
AGGTTAATAATGATTCAATTTATTTGGCAAAAATTCTCTAAAAATACAGGTCTAAACATCC
TAATTCTAGTTCTAAACATGTTTTTCTCAAATATAATAACAATTGTGTTCAATTTTCATAG
TGTTTGCTTACTATAACCTTTTCAGAGCAAAAAATTTGCAGAGGCACTAAAGATAGACATT
TAAAATTGTGTAAATATTAAAGTTCTGTATGAATGCTTTAAAGAATGAAAATTCAAAATGA
TTTCACTTTTCTTTACTCACCTAGGAAGAGGAAGTTTTTGCTCCAAATATTACCTACTAAG
ACTAGGAATTTCAAATGACAGGATTATAGAATTAAAGGAACCTCAAAGGCCTTTGGTCACA
GTTCTTGGTAGAATTGTCCCTTGGTATCCATGGGGAATTGGTTCCAGGACTCAAATGGACA
CCAAAATTTGTTCATGTTCAAGTCCCTGATATAAAATGGTGTAGTATTTGCATATAACCTA
TGTATATCCTCCCATATACTTTAAATAATATCTAGAATACTTATAATATCTAATACATGAT
TTACATATAAATGTCATGTAAATTGTAGTTATAATGTATCTTTTATATTCATATTATATTT
TATCATTTTGCTGTTTTTTTAAAATTATTTTTTTCTCTCAAATATTTTGATCCAAAGTTGG
 
TTGAGTCGAGGGGTGCAGAACTCACAGGTATGGAGTGATGACTATACTTGAATTAATAGGA
AAAATTCCAAACAAAACCAGAAACCAAATCCTTAGGCCTTGCTAATTCTAACAAACCTTTA
TCCATTGCCTCAAATATATGAAAGTAGCACAAGGACATTTGCTGAAGGAAGTATGTCTCTC
ATCATTGCCCCGACTTCTCACATCAGCCTAGCACTCTCATGAATTTTCAAAAGATCGGTCA
TTACAGTATGTTTCTAAAACCCTTTAGCTTCTGCTTTCTTTACTTGAAAGGGTCAGAACTG
AGTGGAACTGAGAGGACTGTGAAGGGAGGCTTTTCAGTTTGTCACATTTGCTTAATTAAAT
GCATTGTTTCTCAAACTCATTTGAGCTTAGGCCACAGTAAGTAATATATGTTTATATTAGC
ACTGTACTCTCATTTTTATGCAACATACTCTGTTTGTTAACAATTTTTATTGCTTTATTCT
ATTGTTCCCCAATGCTGACCACTACCCACTAAATTATTTTAAGACTTACTAACATGTTGTT
CACTGAAGTTTCAAAAACACTTTCTTGGAACAAAAATTTTTCTGGAAATAAATTTATGAGA
TTAAATAGCTTGCCCAGGGTTCTACATTGGGTGTGGTATAGTCATGACCCTGGCTGGTTGA
CTTGGAGCCCGCTATGTTCTCCTGTTTGTGAAATGATAATGAAAAAAAAAAAAACAGCAAC
AACAAAACAAAACAAAACAAAAAACTTATATCTGTTCCATATGCTCCAAAGTCCTATTAGT
CTGAACTTCATAGTTCTGTTCTGAGAACTAAGCTTGGAAAATGCACTGAAGAGCCCTTATC
TGGTCCTGTGTGCAGGACTAGACTCAGGCAGAAGAGCATCCTGAACAAACCATGTATTCTG
TCACCAACCTCCACTTACCTACTAACCTATGGATTCACAACAAACTTACTTGTCTGTGATA
ATCTGTAGCACAGAACAAATTTGTGGCCCTTAGAGCTAATAGGGAAATGAGAGCCATGGAA
GAGAGAGAAGTTGGTTAAGAGACAGAGGACCATCAACACGGGAAGGATTAAAGGATCCATT
ATTGTTTTCAGTCCATTATACCGAAGGAGTGAGAGGGTATACCTTAAGCTGACAGAAATGT
TCTTTTTGCATTTCCCAGAGCTTTTTTTTATTTAAATACCAGTTATATAGTCAACTAAAAG
AGATATGAAATACCACCATTCTGACCCAAAATATTCAGATCCTCCATGTGGATAATTAAAA
GAAAAAGGAGAAAGAAAAGGAGGAAGAGGAAAAGGAAGGGGCAGGAAGGGAGAGGGAAGAG
AAAACAGGAAGGAAGAGTAGGAGAAGAAGTAGGAGGAGAGAAAGGAGAGGAGGAGGAGAAG
GGGGAGGAGGGGAGGAAGAAGAGGAAACAGGAAGGAGGAAAAGAACAAGGAGAAGAAGTAG
GAGACAAAAGAGGGAAGGTAGAGGAGGAGGAGGAGAAAGAGGATTAAGAAAAAGCAGAAAA
 
CCTCTGTTTAGTAAATTCTACAAGAGATGGAAAACATAGCAATACAAAAAAAAAAGCATGT

GATACATGCTTAAACAAATTTAAAGAATACAGAAATAATCCCATTCTCAAGAGAGAGCCAA

TGTTCATAAGGTTGGTGTATATTCTTTTGAACTTTTTCTTTACTTGTAATACATACATACT

TTGAATACCTTGTATATACCCAAACATGTCTCCTTTACTCTGATACACACACACATTTTTT

AAGGCAATCAAACTATACTTATTGCTTTACAGGTTCCTTTTTAAACTTAAAAAATATATGG

TAGACCCATTTCCAAGTATTTACATACATGGTAGCTTAATTCTTTTTTTTTTTTTTAATTA

TACTTTAAGTTTTAGGGTACATGTGCACATTGTGCAGGTTAGTTACATATGTATACATGTG

CCATGCTGGTGCGCTGAACCCACTAAATCGTCATCTAGCATTAGGTATATCTCCCGATGCT

ATCCCTCCCCCCTCCCCCCACCCCACCTCAGTCCCCAGAGTGTGATATTCCCCTTCCTGTG

TCCATGTGATCTCACTGTTCAATTCCCACCTATGAGTGAGAATATGCGGTGTTTGGTTTTT

TGTTCTTGCGATAGTTTACTGAGAATGATGATTTCCAATTTCATCCATGTCCCTACAAAGG

ACATGAACTCATCCTTTTTTATGGCTGCATAGTATTCCATGGTGCATATGTGCCACATTTT

CTTAATCCAGTCTATCATTGTTGGACATTTGGGTTGGTTCCAAGTCTTTGCTATTGTGAAT

AATGTCGCAATAAACATACGTGTGCATGTGTCTTTATAGCAGCATGATTTATAGTCCTTTG

GGTATATACCCAGTAATGGGATGGCTGGGTCAAATGGTATTTCCAGTTCTAGATCCCTGAG

GAATCGCCACACTGACTTCCACAATGGTTGAACTAGTTTACTGTCCCACCAACAGTGTAAA

AGTGTTCCTATTTCTCCACATCCTCTCCAGTACCTGTTGTTTCCTGCCTTTTAAATGATTG

CCATTCTAAGTGGTGTGAGATGGTATCTCATTGTGGTTTTGATTTGCATTTCTCTGACGGC

CAGTGATGATGAGCATTTTTTCATGTGTTTTTTGGCTGCATAAATGTCTTCTTTTGAGAAG

TGTCTGTTCATGTCCTTCGCCCACTTTTTGTTGGGATTGTTTGTTTTTTTCTTGTAAATTT

GTTTTAGTTCATTGTAGATTCTGGATATTAGCCCTTTGTCGGATGAGTAGGTTGCGAAAAT

TTTCTCCCATTTTGTAGGTTGCCTGTTCACTCTGATGGTAGTTTCTTTTGCTGTGCAGAAG

CTCTTTAGTTTAATTAGATCCCATTTGTCAATTTTGTCTTTTGTTGCCATTGCTTTTGGTG

CTTTAGACATGAAGTCCTTGCCCATGCCTATGTCCTGAATGGTAATGCCTAGGTTTTCTTC

TAGGGTTTTTATGGTTTTAGGTCTAACGTTTAAGTCTTTAATCCATCTTGAATTGATTTTT
 
GTATAAGGTGTAAGGAAGGGATTCAGTTTCAGCTTTCTACATATGGCTAGCCAGTTTTCCC
AGCACCATTTAATAAATAGGGAATCCTTTCCCCATTGCTTGTTTTTCTCAGGTTTGTCAAA
GATCAGATAGTTGTAGATATGCGGCGTGATTTCTGAGGGCTCTGTTCTGTTCCATTGATCT
ATATCTCTGTTTTGGTACCAGTACCATGCTGTTTTGGTTACTGTAGCCTTGTAGTATAGTT
TGAAGTCAGGTAGCGAGATGCCTCCAGCTTTGTTCTTTTGGCTGAGGATTGACTTGGTGAT
GCGGGCTCTTTTTTGGTTCCATATGAACTTTAAAGTAGTTTTTTCCAATTCTGTGAAGAAA
GTCATTGGTAGCTTGATGGGGATGGCATTGAATCTGTAAATTACCTTGGGCAGTATGGCCA
TTTTCACAATATTGATTCTTCCTACCCATGAGCATGGAATGTTCTTCCATTTGTTTGTATC
CTCTTTTATTTCCTTGAGGAGTGGTTTGTAGTTCTCCTTGAAGAGGTCCTTCACATCCCTT
GTAAGTGGGATTCCTAGGTATTTTATTCTCTTTGAAGCAATTGTGAATGGGAGTTCACTCT
TGATTTGGCTCTCTGTTTGTCTGTTGTTGGTGTATAAGAATGCTTGTGATTTTTGTACATT
GATTTTGTATCCTGAGACTTTGCTGAAGTTGCTTATCAGCTTAAGAAGATTTTGGGCTGAG
ACAATGGGGTTTTCTAGATATACAATCATGTCATCTGCAAACAGGGACAATTTGACTTCCT
CTTTTCCTAATTGAATACCCTTTATTTCCTTCTCCTGCCTAATTGCCCTGGCCAGAACTTC
CAACACTATGTTGAATAGGAGTGGTGAGAGAGGGCATCCCTGTCTTGTGCCAGTTTTCAAA
GGGAATGCTTCCAGTTTTTGCCCATTCAGTATGATATTGGCTGTGGGTTTGTCATAGATAG
CTCTTATTATTTTGAAATATGTCCCATCAATACCTAATTTATTGAGAGTTTTTAGCATGAA
GGGTTGTTGAATTTTGTCAAAGGCTTTTTCTGCATCTATTGAGATAATCATGTGGTTTTTG
CCTTTGGCTCTGTTTATATGCTGGATTACATTTATTGATTTGTGTATATTGAACCAGCCTT
GCATCCCAGGGATGAAGCCCACTTGATCATGGTGGATAAGCTTTTGGATGTGCTGCTGGAT
TCATTTTGCCAGTATTTTATTGAGGTTTTTTGCATCAATGTTCATCAAGGATATTGGTCTA
AAATTCTCTTTTTTTGTTGTGTCCCTGCCTGGCTTTGGTATTAGAATGATGCTGGCCTCAT
AAAATGAGTTAGGGAGGATTCCCTCTTTTTCTATTGATTGGAATAGTTTCAGAAGGAATGG
TACCAGTTCCTCCTTGTACCTCTGGTAGAATTCGGCTGTGAATCCATCTGGTCCTTGACTC
TTTTTGGTTGGTAAGCTATTGATTATTGCCACAATTTCAGCTCCTGTTATTGGTCTATTCA
 
GAGATTCAACATCTTCCTGGTTTAGTCTTGGGAGAGTGTATGTGTCGAGGAATTTATCCAT
TTCTTCTAGATTTTCTAGTTTATTTGTGTAGAGGTGTTTGTAGTATTCTCTGATGATAGTT
TGTATTTCTATGGGATCGGTAGTGACATCCCCTTTATCATTTTTTATTGCATCTATTTGAT
TCTGCTCTTTTTTTTTCTTTATTAGTCTTGCTAGTGGTCTATCAATTTTGTTGATCCTTTC
AAAAAACCAGCTCCTGGATTCATTAATTTTTTGAAGGGTTTTTTGTGTCTCTATTTCCTTC
AGTTCTGCTCTGATTTTAGTTATTTCTTGCCTTCTGCTAGCTTTTGAATGTGTTTGCTCTT
GCTTTTCTAGTTCTTTTAATTGCGATGTTAGGGTGTCAGTTTTGGATCTTTCCTGCTTTCT
CTTGTGGGCATTTAGTCCTATAAATTTCCCTCTACACACTGCTTTGAATGTGTCCCAGAGA
TTCTGGTATGTTGTGTCTTTGTTCTGGTTGGTTTCAAAGAACATCTTTATTTCTGCCTTCA
TTTCGTTATGTACCCAGTAGTCATTCAGGAGCAGGTTGTTCAGTTTCCATGTAGTTGAGTG
GTTTTGAGTGAGATTCTTAATCCTGAGTTCTAGTTTGATTGCACTGTGGTCTGAGAGATAG
TTTGTTATAATTTCTGTTCTTTTACATTTGCTGAAGAGAGCTTTACTTCCAAGTATGTGGT
CAATTTTGGAATAGGTGTGGTGTGGTGCTGAAAAAAATGTATATTCTGTTGATTTGGGGTG
GAGAGTTCTGTAGATGTCTATTAGGTCCGCTTGGTGCAGAGCTGAGTTCAATTCCTGGGTA
TCCTTGTTGACTTTCTGTCTCGTTGATCTGTCTAATGTTGACAGTGGGGTGTTAAAGTCTC
CCATTATTAATGTGTGGGAGTCTAAGTCTCTTTGTAGGTCACTCAGGACTTGCTTTATGAA
TCTTGGTGCTCCTGTATTGGGTGCATATATATTTAGGATAGTTAGCTCTTCTTGTTGAATT
GATCCCTTTACCATTATGTAATGGCCTTCTTTCTCTCTTTTGATCTTTGTTGGTTTAAAGT
CTGTTTTATCAGAGACTAGGATTGCAACCCCTGCCTTTTTTTGTTTTCCATTTGCTTGGTA
GATCTTCCTCCATCCTTTTATTTTGAGCCTATGGGTGTCTCTGCACGTGAGATGGGTTTCC
TGAATACAGCACGCTGATGGGTCTTGACTCTTGATCCAATTTGCCAGTCTGTGTTTTTTAA
TTGGAGCATTTAGTCCATTTACATTTAAAGTTAATATTGTTATGTGTGAATTTGATCCTGT
CATTATGATGTTAGCTGGTTATTTTGCTCGTTAGTTGATGCAGTTTCTTCCTAGTCTCGAT
GGTCTTTACATTTTGGCATGATTTTGCAGCAGCTGGTACCGGTTGTTCCTTTCCATGTTTA
GTGCTTCCTTCAGGAACTCTTGTAAGGCAGGCCTGGTGGTGACAAAATCTCTCAGCATTTG
 
CTTGTCTGTAAAGTATTTTATTTCTTCTTCACTTATGAAGCTTAGTTTGGCTGGATATGAA
ATTCTGGGTTGAAAATTCTTTTCTTTAAGAATGTTGAATATTGGCCCCCACTCTCTTCTGG
CTTGTAGGGTTTCTGCCAAGAGATCCGCTCTTAGTCTGATGGCGTTCCCTTTGAGGGTAAC
CCGACCTTTCTCTCTGGCTGCCCTTAACATTTTTTCCTTCATTTCAACTTTGGTGAATCTG
ACAATTATGTGTCTTGGAGTTGCTCTTCTCGAGGAGTATCTTTGTGGCGTTCTCTGTATTT
CCTGAATCTGAACGTTGGCCTGCCTTCCTAGATTGGGGAAGTTCTCCTGGATAATATCCTG
CAGAGTGTTTTCCAACTTGGTTCCATTCTCCCCATCACTTTCAGGTACACCAATCAGATGT
AGATTTGGTCTTTTCACATAGTCCCATATTTCTTGGAGGCTTTGCTCATTTCTTTTTATTC
TTTTTTCTCTAAACTTCCCTTCTCACTTCATTTCATTCATTTCATCTTCCATCGCTGATAC
CCTTTCTTCTAGTTGATCGCATCAGCTCCTGAGGCTTCTGCATTCTTCACGTAGTTCTCGA
GCCTTGGTTTTCAGCTCCATCAGCTCCTTTAAGCACTTCTCTGTATTGGTTATTCTAGTTA
TACATTCTTCTAAATTTTTTTCAAAGTTTTCAACTTCTTTGCCTTTGGTTTGAATGTCCTC
CCGTAGCTCAGAGTAATTTGATCGTTTGAAGCCTTCTTCTCTCAGCTCGTCAAAGTCATTC
TCCGTCCAGCTTTGTTCCGTTGCTGGTGAGGAACTGCGTTCCTTTGGAGGAGGAGAGGCAC
TCTGCTTTTTAGAGTTTCCAGTTTTTCTGCTCTGTTTTTTCCCCATCTTTGTGGTTTTATC
TACTTTTGGTCTTTGATGATGGTGATGTACAGATGGGTTTTTGGTGTGGATGTCCTTTCTG
TTTGTTAGTTTTCCTTCTAACAGAGAGGACCCTCAGCTGCAGATCTGTTGGAGTACCCTGC
CCTGTGAGGTGTCAGTCTGCCCCTGCTGGGGGGTGCCTCCCAGTTAGGCTGCTTGGGGGTC
AGGGGTCAGGGACCCACCTGAGGAGGCAGTCTGCCCATTCTCAGATCTGCAGCTGCGTGCT
GGGGGAACCACTGCTCTCTTCAAAGCTGTCAGACAGGGACATTTAAGTCTGCAGAGGTTAC
TGCTGTCTTTTTGTTTGTCTGTGCCCTGACCCCGGAGGTGGAGCCTACAGAGGCAGGCAGG
CCTCCTTGAGCTGTGGTGGGCTCCACCCAGTTGGAGCTTCCCGGCTGCTTTGTTTACCTAA
GCAAGCCTGGGCAATGGCGGGCGCCCCTACCCCAGCCTGGCTGCTGCCTTGCAGTTTGATC
TCAGACTGCTGTGCTAGCAATCAGCGAGACTCCGTGGGCGTAGGACCCTCCGAGCCAGGTG
CTGGATAGAATCTCATGGTGCGCCGTTTTTTAAGCCCGTCGGAAAAGCGCAGTATTCGGGT
 
GGGAGTGACCCGATTTTCCAGGTGCCGTCCGTCACCCCTTTCTTTGACTCAGAAAGGGAAC
TCCCTGACCCCTTGCGCTTCCCAAGTGAGGCAATGCCTCGCCCTGCTTCGGCTCACGCAAG
GTGCGCGCACCCACTGACCTGCGCCTACTGTCTGGCACTCCCTAGTGAGATGAACCCGGTA
CCTCGGATGGAAATGCAGAAATCACCCATCTTCTGCGTCGCTCAGGCTCGGAGCTGTAGAC
CGGAGCTGTTCCTATTCGGCCATCTTGGCTCCTCCCTGCTTAATTCTTTTTAAAGGTCATA
TCATATCTTATAGTAGAGCTATATCATAATTGTTAACTAATCTCCTGTTAATGTGGATATT
TTGTTTGAATGCAATTGTTTTTACAGACATTGCTGCAATAAATGTTATTGCACGTATGTAC
TGGCATTTGAATAGAGTAAGTTTCAAATAGTGGAATTACTAGACCATAAGATTTGTGCATT
TTTAAATTTGGTAGACACTGAAAAAAAAACAGTTTGTTTCTGTTACATTTTAATGAGAAAG
TTTCAATATTTTTAATTTGTATTTTTATTATAAATAATATGGAACATCTTTTCAGTGACTT
GTTGGAGATATTCTGTTTCTTCTTACCTACTCCTGTACTGTGCTTAAAATATCCTGCTTCT
TTAGTCTCAGTAAATGACACTTTTGTTGCCTGTGTCAGCTGTTTGTGTGGATATGTGAAGA
CTCATTGATCTGCATACCTCCTTAATGATCCTGATCACAAAGTGATGACCTGGTGGGTCAT
AGCCTTGGGCTTGGAACTGGACCCTACTATGTGTTATCTGAGTGATGTAAAGAGCAAAGTT
TTAAAATCATTTTTTGAATATTCAACCATTCTTCCTCAACTGGATAATTTCCATTGCTTTT
TGAAAGGATAATTTTCACTACATGATTAATAGTCAGAAAACAAAATTTATAATATAAATAT
AATTATGAAAAATAATTATATTTATCATTCAGTTAAAGAAAAATAACATTGTCATGATTTT
TGAAGTTCTTTTAAGTGTCTCTCAACATCACATTCTTTTCTTTCCCAATACAGAAATTAGT
GTTGATTAATTCCTTGCTTCCCTTTCTAATTTTGCCACATTAAAAAAAATCCAATCAATAT
ATTGTTTAGTTTTTCATGTTTTGAGCATTATATAAATGGAATCACATTTTTTTGCCCCTTT
AGTATCCTGAAATGAAGCCATGTTGTGATGTAGAACTAGTGACTTATTTTTTACTGCTATG
TAATATTCTATTGCAAATACATCTATTTATTCTATAGCTGGCATATGTTAGGATTTTTCCA
TTTTAAAAAATTGTAAATAGTGTAGCTTGATCATCTTAGTACATTTTTCTTGATACACACA
TGCAAGATAATTCTCTAGGGAGCAGTTCTCAATCTTTGCTAAACGTGAGAATCATTGAGAG
ATTAAAAATTCTCATGTCCAATAAACCAATAAAATCACATTATATAATGGCAGGACGTGAC
 
AGTAGGACCAGCTTCATGGACATGTGGCCAATGTAGTTACATAGGCCCTGTGCTCAGAAGA

ATCCCAGATCTGGGGTTTAATGCTCTGCAGTCATTGTCGAAATTCTTAATAATTTTCTTTT

GCTTTTGTTTTTGAAATTCAATGAAATAATAAAGCACACAAGAGGTGGCTTGGAGCCTCAG

CACATGTGTGTTTCTGTGTCTTGCCATTTCGCATCTTCTTAGGGGCAAGCAAGTTCTCAGC

TGCCTGCTCCCCATCCTCTGATGCTGGGCCCTGCCCAACACCCCTTCACTTCGCCATCCTG

CAACTGCTGCCAGACATTTGTCTATTTTCTAAAAAGTGATATTGAGCCTTCCAATCCACAG

ATATATATTTTTTTCTTTCCTGTCTTATAATAAATTTTTGTTACTTTCACAATGAAAAGTA

ATTTCTAAATAAATTTTTTCATAACTTTTGCTATCTATAATTATTTCTAGGTATGGCCCAA

ATATTACAATTGCTATTGAAAGTCATATTTTCTCAGGATTATACTGTCAAACTCTTCATGG

TTGAACTATAGAAATTCATTTGATTTCTACACATAAATTCATGTGTAGCAAAGTTGTTAAA

CACTCATAGATTTTGACAATTTATGGAATTATTTTGTTCTTCCTTTTAGCTAACCATATAC

TCGTCAATAATCACAGTTTTGTTTCTTTTGTTCAATGCTCATGCTTTTTTTTAATCCTCCC

CACCACTTCCTATAACATCTAGGACCTACAGAAAAAAAGTGAAATATAAATGGTGATAGTG

AGCACCCTTGCCTTGTTCCTGATCTTGAAGAGAATTATCTCATCATTTACCATTAAGTCTG

ACATTTGTCACAAGTTTTTTGTAGATACTGCAGATTGGAATAAAGAAGTTATCTTCTATTC

ATGGAATGCAAATCACTTTTAAACAACAAATGAATGCTGAATTTTATTAAATGCTACTATA

CATTTGAAGATCTCATCTGAAATTTGTTCAGTACTTTTTAGTCATTGTCTTGTGGTAGAGT

GATTTGTCATGGTTCTAATATTGTTAGACTATCAGAAATAGAACTAAAGTATTCATTTAAA

CACATTCAAGAATCTCTATTTGTACAAAAAGGAAAAAAAACAGACACAAATAAACCCAACT

TATTCATTAGCACCATGTCTTTTCCCCTTTCACAGACAAATTTTCCAGAAGAGTTGCCTGG

GTTTATTGTTTCAATTTCCTTTTCTCTAACAGGTTGATCCACCCTTTCCAGTCTGGCTTCT

GGGTATAGATCCACTCACAAAAGCAGCTTTCCCATAGGTTATCAACAACATCCATTTCAGA

GGGCCTGGAGGTCCTTGTCTTATTTGGATTTTAAGCTACCTTCAAGAACAATTGATATACT

TTTCTTTCTTGATATACTCCCCTCACACCTTAGCTCCTATTCCTCTGATCACCCTGAATGC

TCCTCTTATTTCTACTTTGTAGGCTTATCCAATTGGCTAATTAATATTTGTGTTTCAAGAC
 
TCAGCTGTAGATTCTCTACTAAAGTACTGCTATAATCTTCCCCAAGGAGATCCCATCCATG

CCCATAGCATCATCACTTCTTATAATGACTTACAATCTTCAAGGAGACCTCTTTTCTAAGA

TTCCAACCCAATTGCCTGTCCTCCTACTTGATATATTCTTTAGGATTTTGAGATTGTGATA

TAACAAATAAGCTCCAGAAGCTCAGTGGCTTTACAGAATGGAAGTTTATTTCTTGGGAGCA

TTAAGTCCAATCAGCATTAACATTGGTCATTTAGAGGACTAGCCTCTTTCCTACATGCAGC

TCTACCTCAGCATAGGACCTCTAAGTCTTCCCTACTCAGCAGAGGATAGGAAAATAAAAAA

TGTGGTTTGCCTCACAGTCATAAAGGAGCATTTTATGGTTCGATGGTGGCAACAGTGCATA

TCACACACACTCATGGTCCACTGGCCCCAACTCAGTCACACGGCCACACTTATCTCCAGGG

AGACAGGGAACTATAGTCTAGCTTTGTGCCAGGAGGAAAGGCAATTGTAATTGTTAATCAA

CAAATAAGTCTCTGCAACAATTTACCCTTCCTCTTAGCAAATTTCCGTTGCATTTTTCTCT

TCACACAGAGAATATGCTTATTCCTTCTCCATTGGAGACATCTTAATGTTTTTCAAATTTT

TATTCAGCATCAGCCGCGAACTCAAAGCCCTGAATGTTAAAAGATGTGCAAGCTAAATGTT

GTGGCACCTTGTAATAGACAGGAAACCCACTGCCATCTTCCCGACACAGACACCAAACACT

AACACACCCAAACCCCCACTATAAACCTACATATAGTATCAGGGACAGCAAAACCTCAAAA

AAAAAAAAAAACTTCTATTCAAAATCAGGAAAAATGGGAGAACAATAACACACAGTGAGAT

CCTATACTATAGGCATTGTGCAGCCTCTCTGGCAGTGTGGATTTCAGTAAGGAGAACCTGA

ATCAGATATCTGTGAAGAACCACCTTCTGTGGCTCCTATTTTATCCTCTGAAAGGTTCTTC

CTTGACCGTATTTTTCCACGGCCATATCTGAAATAGGTTTGGCGGAGTACACCCTCCTCGG

GGAACATGGAATGTTCATAGCCAAATCCTGTTCATAGAGTTTGAAGGACTGAAAGTTGTTT

TAAAGTTTAACAGTCACAGACTTTTGCAAGCAGACTGGTGGCTCCTTTGGCAATATAATCT

CTTAAAAATGTGTTAGATTTCTCATGTACTTGCTTTTTATCAGTTACATGTGCCAGTATTC

ACAATAAAAAATTTTTCTTGACTTAGTTCCCAAGTTTGCCTTATTTCTTTCCTTCCTCACC

TCTGTGCCTCTCTCTCTCTCATTTAATAATTTAAGCTAATCTAAACAATAGACTGTGTAAT

AAGGTAATACTCTTAATCTGAGATTTGCTCCATCATTGAATCAATTTATTTAACTTAGAAA

TACTAGGCCTTTTATGACTGGAGATATTTTCCTTTGTTTTCTTCAACTTTTTTTTTTTAAG
 
TTCTGGGATACATGTGCAGGATATACGGTTTGTTACATAGGTAAATGCATGCCATAGTGGT

TTGCTGCACAGATCATCCCATCACCTAGGTATTAAGCCCAGCATCCATTAGCTATTCTTCC

TGATGCTCTCCCTCCTCCCACCAGCCACCTGACAGGCTGCAGCATTTGTTGTTCCCACTGT

GTGTCCATGTGTTCTCATCATTCTGCTCCCACTTATAAGTGAGCACATGCAGTGTTCGGTT

TTCTGCTCCTGTGTTAGTTTGCTGAGGATAATGGCTTCCAATTCCATCCATTTTCCTGAAA

AGGATATGATCTCATTCCTTTTTATGGCTGCATAGTATCCCATGGTATATATGTACCACAT

TTTCTTTATCCAGTCTATCATTGATGGTTATTTAGGCTGATCCCATGTCTTCACTATTGTG

AATAGTGCTGCAGTGAACATATCCATGCACATATCTTTATAATACAATGATTTATATTCCT

TTGAGTATATACCCAGTAGTGGGATTGCTGGGTCAAATGGTATCTCTGCTTCTAGATCCTT

GAGGAATTGCCACACTGTCTTCACAATGGTCGCATGTTAGTTTTGATTTGCATTTCTCTAA

TGATCAGTGATGTTAAGCTTTTTTTCATATGTTTGTTGGCCACATGAATGTCTTCTTTTGA

GAAGTGTCACTCCTTTCCTTTACCCACTTTTTAATAGTGTTGCTTGTTTTTTTTCTTTTCA

ATTTGTTTAAGTTACTTGTAGATTCTGGATATTAGATCTTTGCCAAATGGAAAGATTGCAA

AAGTTTTCTTCCACTATGTAGGTTGTCTGTTCACTCTGATGATAGTTTATTTTGCTGTGCA

AAAGGTCTTTAGTTTAGATCTCATTTGTCAATTTTTGCTTTTGTTGCAATTGCTTGTGGTG

TTTTCATCATGAAAATTTTGCCTGTGCCTATATCCTGAATGGTATTGCCTAGATTTTCTTC

TAGGATTTTCATAGTTTTGGGTTTTACATTTGTCTTTAAGCCATCTTGAGTTAATTTCTGT

ATATGGTGTAAGGAAGGGATCCAGTTTCAATTTTCTGCATATGCTAACCAGCACTCCAGGC

ATCATTTATTAAATAGGGATTCCTTTCCCCACTGCTTGTTTTTGCTAGGTTTGTCAAAGAT

CAGATGGTTGTAGCTGCGTGGTCTTATTTTTGAGCTCTCTATTCTCTTCTGTGTATCTATG

TGTATGTTCTTATACCAGTATCATACTGTTTTGGTTACTGTAGCCTCACAGTATAGTTTGA

AGTTGGGTAGCGTGATACCTCCAGATTTGTTCCTTTTGCTTAGGACTGTCTTGGCTATTTC

GCTCTTTTTTTGCTCCATATGAATTTTTAAATAGCTTTTTTCTAATTCTGTGAAGAATGTC

AATGGTAGTTTAATGGGAATAGCATTGAACATATAAATTACTTTGGGCAGAATGACCATTT

TCATGATATTGATTTTTCCTATCCATGAGCATGGAATGTTTCTTCATTTGTTTGCATCCTC
 
TCTGATTTCTCTGAGCAGTGGTTTGTAGTTCTCCTTGATGATGTCTTTCACTTCCTTTGTT

AGCTGTATTCCTAGATATTTTGTTCTTTTTGTAGCAGTTGTAAATGCGAGTTTGTTCATAA

TTTGGCTCTCTGCTTGTCTGTTGTTTGTGTATAGGAATGCTAGTGATTTTTGCACATTGAT

TTTGTATCCTGAGACTTTGCTGAAGTTGCTTATTAGCTTAAGAAGCCTTTGGGCTCAGTCA

ATGGTGTTCCCTAGATATAGGATCGTATCATCTGCCAACACAGATAGTTTGACTTCCTATT

TGAATACACTTTATTTCCTTCTCTTGCCCAATTGCTCTGGCCAAAACTTCCAATACTATGT

TGAATAGGAGTGCTAAGAAAGGGCAACCTTGTCTTGTGTCACTTTGCACATTAATCTTATA

TCCTGAGACTTTGCTGAAGTTGCTTATCAGCTTAAGATGCTTTTGGGCTAAAACAATGGGG

TTTTCTAGATACAGGATCATGTCTTCTACAAAGATAATTTGACTTCCTCTTTTCCTATCTG

AATACCCTTTATTTCGTTCCCTTGCCTGATTTCCCTGGCCAGAAATTCCAATAGTATGTTG

AATAGGAGTGGTGAGAAAGGGCATCCTTATCTTGTGCTGGTGTTCAAGGGGAATGCCTCCA

GCTTTTGCACATTCAGTATAATATTGGTTGTGGATTTGTCATAGACGGCTCTTATTATTTT

GAGGTATGTTCCTTCAATAACTAGTTTATTGAGAGTTTTTAACATGAAGCGATGTTGCATT

TTATCAAAGGCCTTTTCTGCAACTATTGAGATAATCATGTGGTTTTTGTCTTTAGTTCTGT

TTATGTAATGAATCATATTTATTGACTGGGCTGTATTGAACCAAACTTGCACCCCAGACAT

GAAGCCAACTTGATCGTGGTGGATAAGATTTTTTTTGTGCTGCTGGATTCGGTTTGCCAAT

ATTTAATTGAAGATTTTTGCATCGATGTTCATCAAGAATATTGGCCTGAAGTTTTCTTATT

TTTGTTGCATCTCTTTCAGGTTTTGGTATCAGGATGATTTTGTGCTCATAGAGTAAGTTAG

GGAGGAGTCTTTCCTTTTCAATTTTTTGGAATAGTTTCAGTAGAAATGGTACCAGCTCTTC

TTTGTACCTCTGGTAGGATTCAGGTGTTAATACCATCTTGCTTGGTAGGCTATTTATTACT

GCCTCAATTTCAGAACTCATTATTGGTCTATTTAGAGATTCAGTTTCTTCCTGGTTCAGTC

TCGGGAGGGTGAAGGTGTCCAGGAATTTGTCTATTTCTTCTGGATTTTCTAGTTTATGTGC

ATAGAGGTGTTTATAGTATTCTGTAATGTTTGTTTGTATTTCTCTGGGGTCAGTGGTGATA

TTCCCCCTTATTATTTCTGATTGTGTCTATTTGATTCTTCTTTCTTTTCTTCTTTACTAGT

CTAGCTAGCTAGCTATTTTATCTAAAAAAATGGCTACTGGATTTATTGATTTTTTGAAGGA
 
TTTTCATGTCTCTATCTTCTTCAATTTATCTTGGATCTTGGTTATTTCTTGTCTTCTGCTA
GCTTTGGGGTTTATTTGCTCTTGGATATTTTTCAATCTTATCTTTTACTATTTGGGACCTA
CAAGTAGTCGGCTTTTTCAATGTTAGGGGATCCTTATTTTAATGCCTCTAATCTTTGCTTG
ACAACCAATTTCTACCTGAGCTCATGTCTCTTTTGTAAAACCCTGCTCAAGCAGCCAAGAG
TGAGCCATTCATACTATCACTCCAGTTCTTTCAAACCACCACATGTAGTGCTACTGGCTGG
GTGGATATCTGATCTCCTTTGCAAATTGTACCAAATAATTTGCCAATTCACAATTTAAAAA
CGGTCTCCATCTTTCCAGCCCCCAATAGGCAGTTTCTCACCACCTGACTGCTAAGACAATT
CTCCATATTTTAAACTGTGGCAGCTGCCCTACTTCAAGGTGTCAATTTTTATATAGGCTAG
GCATCACTAAGCCATCACCAATAACACATCAAATACCAGTAGGCCAAAACAATAGTTAATT
TCTGACTCACATGAAATTTAATTCCAGAGGGAAGAGGTGGGATGGGTTGAGTGAGTTAGAG
GAATTTAGGTGTTATACAGCTATTTAGTGATTCCAAATGTTTCTACCGTATGGCCACACCC
TCTTCTAAGTTTACAGATGCTTCTTCATTCATCCTGCAGATTCAGAAAAAGACATTACACT
GGAGATTTTTAGGGGCCAGTCATAAGTGCATTCCACATGTACTTCTGTTTCTACTTATCTG
TGGGGAGGCTGGGAAATATAGTTTAGCTCGATACCTTGGAGGAAGAAAAGTGAATTTGGTG
AGTGGCTTGCCAGTCTTTGCCACAGTATTTCCAACAAAGCATGTAATCTGTCTCCCTAAAA
CTAGTCCTATTTAATTATCATTTGTACCACTCTATTATAGATAGATACTGAAAGATCCAAA
ATTATTTTTGAATATGGCCAGTACATATAACAGAGAACAAAGCATAAAGCAATGACTTTTG
TGCAGTAGTTTATCTGGGAGCACATGTAAAGGAGATGGGGTGTCAAGAGTCAGTAAAAAGA
TGCAATACTGAGTTGTTTTCTAATTCAGGTAACTGAAGTTTTCTTCTTTGGGGACCTTCTA
AGATGCTTTGTGAAATACATCACTGAACTTTATTCATAGGAGAAGAAAGGGGAAATCATTT
GACCTTTGGATCCTGTCCCTCACTGATTTGGGGTGACCCCACTAGCCTTGATTCCCTCAAA
CTCGCATACAGTGTATGCATGAGTGCTGAGTGGGTCCCCACATTTCGTGCTTCAGTGGCAG
AAAAGTACTGGGAAAAGAAAAATGAAAAGCATGGTACAATGTCCAAGATGAAGCACTCTTA
GGTTGCAGCTGCATAAAAAAAGATTAGGCATTCTATGAAGAATTTTGATGGTGGGGAAGGA
AGGCAAATTCATATCCAAAGTAAGTATAAATTCTAGTTGGAAAGCATCACTACCTCTTTCA
 
AGTAGAAAGGTTACGGTATTATAAATCTGCCATCAACCTGCTAAAATAAGATCTTGGTATA
GGTATCAGTTGTTGCTGGAAGGTTTGCCATTCAGCATTGGTAGAACACTAACTGATCAGCC
TTATGGAGAGGAAATCCATTCTGTCAGGCCCATGCATAGCTGCCATCCTTGCCACCATAGC
TACATTCTTCAAGCTCACTGCAAAACTATGGTCATGGAGGGTCTTCTCTGTGGTAGATACT
CTCTGCTGGCAAAAATCTGAGACAGAAAAATTTGCCCACGTCTTGCCACTACCATGGGTAC
TTCCACAAGTCTTTTTCCCTCATTCCCTTTTCTACAGTCTTCCAAGTCTGCTGTTTTTTTT
AATCCCTTTGACCAAGCAATTTACCACTGACCATGAGTCTTACTTCTAGCCACTTGTCTTT
CCATACAAAGTGAACAGTCAAGAGTACTGCTCATGTTCTGCCTCTGGGAAGGATTTCCTTT
TACCACTGGCTTCTAGAGCCACCTCTGTGAGTGTTTGTAGTCATCTGTGCATGGACAGTAA
ACACTGATATACTGGGTTCGTCTTTCTGGGAATCATTTACTTAACAGATCCGATAATATCC
AATAGGTGCTAAATTTCAAATGGCAAGGAGTATTCTACTGCAGATGGAATGGTTTTGCTTC
AGAGCCCAAGGTGTCTGTGTTGTAACTCTCCTATTAAGTTCTTGCCAGAGACTCCATATAC
CAACCTGAGAGCTTGAATGAGATTAAGTGTATAAAGTGACTATTAAATAGCAAATATTGTG
TATATGTTACACACTGTGCCTTTATTTTTATATTTTTAACTTTTATTTTAGGTTTGGGGGT
ACATGTGAAGGTTTGTTACGTAGGTAAACATGTGTCATGGGGATTTGTTGTACATATTATT
TCATCACCCAGGTATTAAGCCCAGTACCCAAGAGTTATCTTTTCTGCTCCTCTCCCTCCTC
CTCCCTCCCCCATCAGGGAGACCCCAGTGTCTGTTGTTTCCTTCTTTAACACTCTGCCTTT
AAAACCATTATGTTGATTTGGATTAAGAATTGAACCATTAGGCTTCTTTTATTTGATGCCT
TACAAAGACTATAGTGAATACTGCTACACATTTTTCAGAATTTATTTTTTATGTTTTGCTT
TTTCCCCAAATTTATATATTTTTTAAATTGATATAAAAATTATATGTATTTACCATATGCA
AGATGATGTTTTGAAATACACACACACACACACACACACAAACTGTGGAATGGTTAAATCT
AGCTAATTTACACACACATAACCTCACATAGTTATCATTTTTGTGGTGAGAACACTTTGCA
TTCACTCTCTCAGCATTTTTCAAGAATGTAATATATCATCATTAACTATAGTCACCATGTT
GTACAATACATCTCCTGAATTTTTTCTTCCTATCTAACTGTAGTTATGCATCCTTTGACCA
ATATCTCCCCAACAACCCTTGCTTTTAATAATCATCCCTCTATTCTCTGTATCTATGAGAT
 
CAACATTTTTAGATTCCGCATACAAGTGAGATCATGTGGTATTTGTCTCTCTGTGCCTGGC
ATTTTTCGTTCAGCACAATGTTATACAGTTTCATCCATGTTGTTGCAAATAAAAAAATTCA
TTTTTTTCCATGGTTGAATAGTATTGCATTGTGTATGTATATCACATTTTCTTAGGTTGAT
TCCATATCTTGGCTGTTGTGAACAGTGTTGCAATAAACATGGAATGCAGATATATCTTCAG
CACACTAATTTCACTTCCTTTGGATGTATGCCCAGCAATGGGATTGCTAGGTCATTTGGTA
GTTCTAGTTTTAATTTTTAGAGGAAACTTCATACTGTTTTCCATAATGGCTGCCCTAATTT
ATTTCCACCAACAGTGTGCTAGGGGTTTCTATATTAAGGTTTTACTAAAGCCAGCTGGTTC
CCCAACTCTATTAGTAACAATCTTTCCACTATTAATGAAGAAACATGGTCACAAAGACAAG
CCCAAACTATTGAGGAGTATTACTGGACATTAAATTTTTGGTTCTCAAATCTGGCTAATCC
TCAGAAATATATACAGAGTGTGTTGAAAATATAGGTATTTAGCCCTAACCCCAGTAATTCT
AATTCAGTAGGCCTGGGATAGAGTTCAGCAACATGTATTTAAAAAGTTATCCAGGTGGTTT
TGATTTTCAAGCAAGTCTGGGAACCACTTCTCTAATCCACATTTGGGAAAACCCAGTCACC
AGCCATAGGTCCCAGATCCTCTTTTCACTTACACCTCTCAGGGAATAAGAAAAGTCTGTGC
TCAGAAGGGAAAGACCAATAAGACTAATACCATGCAGCTCCCAAGGTGGCCTGCTTCACTC
CAGTGCCCAAAACAATTGTTGGTACACATTAGCCACTCAATAAGTAGTGAATGAATGAATG
AGTAAATGAATGAAAGTCAACCATAGTTCTATGGCAGCACTGTTCTTGAATTCAATAGCTT
TTCCTTGCTTTATTGGAAGTATCCTAATACTGCTTCTCTATTTAGAGCCTCAATTCTTTAT
GTTTCCACTCAAGCTGGAACTTTTAATTCAAATATTTGAGCAAAAGAATCAGGAATTAACA
AGACCAGGCTGCTACTGCTCACTTTAATAAAAACATTTCAAGCTCCTTGTATTTTCCAACA
CTTTTCCTTTTTATAGATCTGCTCTCAAAAAGTTTGAGTAGTAGGAAATAATTTTACAGTT
GATCCTTCAGTGGTGTCATCATTGCAAATAAGCAATTGATCCTAACACATTTTTGCTCTTA
AATGTGTGCATTCCTTTAATGACTGATATGGTTAGCCCTATCTGTGAATGACAGAAGATTG
TTCCCAACTGATTTCCAGAACCAGCTGGCTTTTGTGAAATATTAACATAAAACCTCCTCAA
AATGTATATAGTCTTTTTCAAGAAGCTTTAGAAAAGAGCCTTTCCGTACCTGTTTCTACCA
TTTCATTTCTTTTTATAGCCATACTTCTCACCCATCTGTCTATCGATCCCTCATGTTAGAT
 
GTCAAGATGCTAGTAAGATTGGTTTATTCATCTTACTTGTCTGCATTCAATGGATAGATGA
TTTATTTGGCTGCATTTCATCCTGCCTTACAACATTTCATCTGTCTACTCCCAGTGAAGGT
CTGGGTTTAAAAATAACATTCCATTAGCGAGCATTTTTATAGAAAGTACTTTGAATACACT
CATTTATATATGGAGGTGCATGATACATAGTCTTACATTTTTTTCCTTAAGTACATAAAAT
TTTAAACTTAATTTTACCAAAATAATGCAAAGACTGTGTAAAATGACTTAATAAAGGTTCG
AACTCACTTTTGTAATTACATGTTATAATTTTTACTATTATCTGATCACTGACTGTCTGAA
GAAAGATTACAGACTATAAATAAAACACTGAGCTAAGTGAATTGTGAAAGACATTTCCCCT
GCAAAAACTCCTCTCTCTCTCTCTTTCTTTCCCTGTCTTATGCACACAGATATACATACAC
GTATACATAAATGCACATACATCACCTTCTAGTATAGCCAATTCAAATAAATTAGTATTTC
AGGAGCTTTATGTTAAGTTTTGGCTTTAAATAAATCACTTCATCTAGCCAAAACCACTGAC
TTTTTCTTGGTTTCTACACAAAACTCTAATTCAAGTCTAGTTTCCTTCAAATTCAGTCCAG
GTGCTAAAAAAGCTTCTTGGTTTTCCAAGGAACATTCAACAGAATTTCTCTACTCTGTATT
CATATTTATATCATAGCCTCTAAACAGTCCAGTATTTGGTATTTTCTGGATTAAAATGGTT
CCATGGTGAATTTGTAGTCCATTGGATTCATAGACATTCACTTATAAATGTTTGTTTTTCC
TCTTAGAAATAAGTGATTATTCCTTAAGGATCTTGTTTTTTATTATTTGCATTGTTTTGAA
TTCTGTAGAGAACAGGCATATGTGTTTTAAGTTAGCAATAAAGAGAATAATAACAAATAAA
CTGTTTCTTTAAAATTACTTTAAATACCATCCAATGTAAAAATGTTGATTTATAATAAACT
CTACAACTTTTAGGTTTTATGCTATTTAATATATACACTTAGAATTCTAGATATTTCTTTG
GGTTGGGTCCTCAGGGAAAAGTCAGTGTATTTAACCATTATTAGCTTTCAAATTTAAAATA
TAAATAGTGCCCCTTCTTCCACTCTCCTTCCTCAGCCTGACTCTTACATTTTTGATTAAAA
ACAATAATCAGCAACAATGACTCTAGAAGTCAAATTCTGTGCTTGGAAATGTAAATAGACC
TTATTACAGAGCACAGCCATAGAGAGGCAACATAGCACCAATGTGTCTGGGTTCAAATAAT
TTTTCCATCATTTACAAGATAAGTAACCATAAACAAGTTACTTCTCTCACTTTCCTTCAAT
TTCCAGTCTGTGAAATGAAAATCACAATAAACATTTCTAACTAAGAATGTTGGTACAAAAA
TTATATCAGTAAAGAAATATCTGAAACATAGTAAAACTCAGTAAATACTACTTTTTATCAC
 
AGATTTTGAGGGGTTTGAAAAATCAGGCAAATCAATACTTTTTGAATAATTATGATGTTAA
ACATGTAATATGGGTAGAAAATTTCTGGAAGATCTAAGGCATATTTTCTTAGAATAAGTGA
CTTCTTAAGTATGGTGTTATCTTTGAATAATTTTAGGGTAGATAATCAACTGGGGCTATTA
GTTGCAAACTTCATTGTTCATAAGAATCACATGGTTTCCAGTTAAAATCCAGATTCTGATT
CATTAGGTCTGGGGTAGGGCCTGAGATATGCATTTCTACAAGCAACTAGGTGAGGCTGATG
CTGTTGGTCTAAGGATCACATTTTAAATAGCAATTACCTCTAAGGATCACATTTTAAATAG
CAATTACCTCAACAATTTCTTGAGGACATTTTAAGAAGACATGAATTTACTATCCAATCTC
ATATACAATTATGCACTGCATAATGATGTTTCTGTCAAAGTCAGACTGCATAATGATGGTG
GTCCCATAAAATTATAACATCGTATTTTTACTGTGTTATACTTTGTTATACTGTGTTTTAC
CTTCTCTATGTTTAGGTACACAAATACCATTGTGTTACACGTTGACTACAGTATTCAGTAT
TCAGTAACATGCTGTACGGGTTTGTAGCCTAGGAGCAACAGGCTAGACCATATAGCCTAGA
TCTGTAGTAGGCTATACCACCTAAGTTTGTGTACATTCTCTGATATTTGCACAACAAAATC
TCCTAATGATGCATTTCTCAGAATGTATTCTGGTCCTGATCATTAAGCTACACATGATTGT
ACATACCTTCCTGCCGTCTACTGGGAAATAGACTAACCTGATTGACATGATGCACTTTCTA
ATCCAATGGGCTTGAAAACTGGGGCATTTACATTTAGGACAAGTTCCCAGGTGATGCTGAA
ATTGCTTGTCCAGGTACCACACTCCATGAAACACTGGCTTAGAGTACTCATTGATTCTCTT
CGGAAAATCAGGTGGAATCTGGCAATGTTCACTTACTTAAATCATAAACATAGCATACACT
ACTGTTAACTCCATAAGGAGAACTTCAACACTAGTTCCTTTGAGATACCATCCCGATGTTT
TCGAATTTGCTTACCAAGTCAATGGAACAAAAGATCTCACTCATAAGTGAGAGTTGAACAA
TGAGAACACATGGACACAGGGAGGGGAACATCACACACTGGGGCCTGTTGGGGGGTTGGGA
GCTAGTGGAGGGATAGCATTAGGAGAAATACCTAATGTAGATGATGGGTTGATAGGTGCAG
CAAACCACCATGGCACGTGTATATCTATGTAACAAACCTGCATGTTCTGCATGTGTATCCC
AGAACTTAAAGTATATAAAAAAAACTCAAGAACTTCCATTTATCCCCACACAATAAAACAT
CCCCAGACTCAAGAGAAAGAGAAGCATTATTTTTTCTATTAATTAAAAATAAACTATTACT
ATATTCCAGAGGCATTAGTATCAAGATTATCATGTTATAAACATGAAAAAGTAGACTTGGA
 
TAACTGGAAGAATTCCCATTGGCCCCACAATTCTACTGTTTACATTTGATAACAACTGATG
GGACAGATAAAAGTCAAGAGTCAGAAAACATCTGAACACTGAGTGTCATCACTTGATTTTC
AGCATCAAGCACACTGTGTTTAGAAAGGAGTGAAGAATTTTTGCTTCGCACTGGGTAAAAT
TAGACTTGGAAGCAAAGAGCAACCAACAGATGTGTGTCAGGGAGAGATCAGCTGGATAAGG
TTAGTTGTTTAACATCTAAGTCAAACCTGAACATATTTGAGTAAGTCATATTTTCCACAGT
AACTTTTGCAACTTTAGAGGTTCTTTGGTCATGGCAGTTGGAATTTGCATCAGAACTTCGC
AAATAGGAAAATCTCCCTTAAGTTCACTCTAGATTTTCCTAGATTTCTCCAAAAAGCAGCA
AAAAAAAAAAAAAAAGTCAGACTCAGCTACGGAGTTAGCCTATGAGGAATGCTCCAACCTT
GCTGATGGTATACTTGGGACTATCCTGGAAAGCATCAGTCTTTAGAATAGAGGTGGAGCCT
ATAGATGAGATCCACCTGTGCAATTAGCTTTACATTTGCTGTCACTTCTGATGGAAAGCAC
TAAGACAATCAGGTGTTTCCCTTTGAGCACACTACTACCTTCCTCAAAGAAACTACAGAAA
TAGTGAATGACTAATGACCAGAAGCATATCAAGAAAACATTACATTAATATTAATAGTATA
AGAATCTGGACAAAGTCTTGAAAATAATTATCCCATTTGACCAGCTGTAAAGAATCTTCTC
ATGGATCTGCTTGCCACTTTATCTCCACATTGTAAAATGATGACATGGAGAAAACTGAAGC
TATAAAAGATGTATCTTAAAAATGGAGATTTAATAAACATCTAAAGATACTGCTGTTGAAG
CTACATTCTGACTACTAATGCTAGCTGACTTGTCTTTCGTTTGCTCGAGCTGCTGAGACCT
AATTGACTGCTATGTGTCAGACATTGTTCTATTCATTTTATGTGGCTTAACTAATTTAATT
TTTACAAGCAATATGTGAAGTAGGTGCTCTTTCTTTTACCCAAAAGAAAACAGAGGCGACT
AAAAATTAGTAACTTGTCCAAGTCACAAAAAAAGACAATAGAGCCAGAATTTAAACATAGC
TGTTTTTGGCACCAGAAGCTGTGATTTTATTCACCATAATATACTGCTCTCGTCTAATGAT
ATAAATCTTATAATTGTCAATTGATTATAAATGTTACATTTCACAGGGAACACAGGAATAT
AATAAGAGACTATTTAAAAAGTTTGTTTGATGAGAGGCAATTTGTTGCAATATAAAGAAGA
GCCTAAGAAGCCAGTTCTATCCCTACCTCCACCAGGTTGTGTAGAGGAAATTAGTTATCTT
CTCCCTAGAGCTCAGGTTTCTCACATATTAAATTCTAGTAAAGATTTCAAAGAAATAGATG
ATCTCTGATATTAGATGTGTTCTGATAGTCTTGGATTAGGTAACTGAGGCTAAGTTCAGGG
 
CCATTTAAGCAAACTCCAGACTGTGTATGTGGCCCCCTCTGGAGTGACTATTTATTTAATA
TTAAAAAGACAAGTAGCCCACCAATAACTTTCCTTTCCTAATACTATACTAAAATTTTAAG
AAAGCCTGTATTATTTCTTTCTTTGCCCTTGAAAAAAGTACTTATTCTTTCAGAGAGCTAA
ATTGAGATTTGGGATAAAAGAGTTTTCTCTAGTTCCAGATTACCTTTTATTGCTTTGTTTT
AAAAATTCTAAATAGCTAATAATGTGTCCATATTCAATAAACAATCATAATAAGGCACGTT
TCTTTGCTTCTAGTACTTACGTGTTTGACTCTATAAAACTGCATATTTTTTTAGCAAGGAA
CACAATAATTTTCACCTAAGGGACATTCAAAAATATGATGTGGAACTTTTGTCATCTACAA
ATCCAGTATGTTGGTCATTATTTGCCACTTTGACCAGGCACTATTGTAAAAGTGTGTGAGT
TTAAGTGAATGCGTGTCTGTTGGCTATGTTTTTGTAAGTATATGTGTATAGGGAATCTTTA
TCTGTTTTAATTTGGGCAACTTAAAATTAGATTACTGAGAGAGACCCAGCTTCCCCTCATC
TCTCTCAAGATTTGACTAAGAGAGGCATCATATCTGGCTGCAACTTCTACCTTTGTTGGCT
AAGATTTCAACCAGATACTCTAAGGAATTATCTGGAAGATTTTATCTTAGGACCTTTCAAA
ATTCAAAATTCCCTCAAGAGAAGAGACATGGAGGAGTAGTTCCTAAAGATGCAGCCCATTC
CAGTGGAGACAAATGCTGGCTTTAGTTTCACCACTAGGTTTACTTGCACTCAAAAATTCCC
AATAAAATCCAGCTGGTTTAAATTTTGTCTGCAGACCTGAAACACTGCATATTTGGCTAAG
GCTTCCTATTTTGCAGTAAATGCTTCACAGGGCTATAATCTGGAAATACTGAAGTGTACAA
ACCCTCAACAGAAACAACAGGAGAACGGCTGTCTTCTACCTTCATTTTCTCCTAACTCTAC
TCTAAAGGCATAGGTGTTGTTAAGGAAGGGGAAAAATCCTTCATTGAACATGAAATGTTGT
ATGAATCATCATTTGACTACCTTTGTTGTTTGCTCAAAGCCAAAACATAAAGATTAAAGAG
TTGCCATTGATGAGTGAGACAATTCACATTTTGGCATTACTAGAGAAGAAAGGAGCATTTA
AGATCATGGAGGGAAATAAAACAACAAAACAATACTGTTGCAATTTGAACAATAACAGCCT
TCAGTACATGAAATCATAGTATTATCTTTTCCATTTTCCACCCTTATATTTTCATTGGCTT
CATACAATCCTCTTGTTGCCTATAATTGGGGATAGGGTTAGTGCCAAAGCTGCATAAAACA
AGCAGCCTTTCCTTTATACTTTCTTTGCATCGTGATTGTGTATGTGAAGTCATAAAAATCA
TTTATTATGAGCATGTGTATTCTTGGCTTAAAGAGCCAAGTATTCTTGTGACAACAATATA
 
ATAGCATCAGATGTATTGCCATAAAACACCATATGTGGGCACTTAATAGTTACTGCCCTGG

CACCTACATGCTGGGATATCATCTTAAAGAGGGACTCTTTAGAAAAAAAGAATGTTTGGTA

CTTAGACAAATAAAGCTCCTTGGATGAGATAGGTGAGCAATGAGTAGACATTTGTGAAGAA

TCCATTGATGATGGCAACGGTTCGTCTTCCATTAGTATCAGATTTTCTCAACCCATCTGCT

GGAGTTCTTTTCATGGGTCCAAGAAAATCCTTTACAACATTGCAAACATCAATAAATATCT

CTCAGGCTAAAAGTTGCAAGAATGTTTTAATGTCATATTTACAGCTAATAAGCTATAAAAT

GAGAACAACGTAATTTTCATTCTGTGCAACCATTACTCTTTGGAAAGCATTTATTTAGGGT

ATGTTGTACAAATCATTAGTAAACAAATTAGTTCTCAAACTAGATAAAAGAATGTAGAATG

GATGAAAGCCACTGCATGGCACATGTGAGCCCTTGGTGGTGTTTCGAGCCTCTGTGACAGA

CACTGCTCCCCACTGTCTCTGTCCTTTCTTCTAGCAATGATTTCCCTTGGTTGCTGCTGAA

GCTAGAGACTAAGCTTTCCCAAGCATCAATGCACTTCAAGTTTGCCATAACTCTGGGACTG

ATGTTCTACTTCTGCATTAGCACCTGAGTCAGGTGGCCAGACATTAACTGTAGACACATGC

TAGTGTCTTTCAAAAAAAACTCAGTTGCAAAGGAGGCCAGAGTCCACTAGTTCCTGAGTGC

ATGTCTGCTTTAGTTAGGAGTGAAGGCGATCTGAAACAAGAAAAGCCACATAACTCTGTTT

GCTGAGTTTTCTCAGAGGGCGACTCATCTTAGTGATACACTGCTGTGCGTCACTGGTGTCT

GTGTAGGAAGATGCTACTCTTGATGTTTTGAGTTTCCAGGCCAGACATGATTCAATCCCTG

AATCTCTATTCACTAGCTCTTTCTAGTATAACAGACAGTCTAAAACTTACAATGGTATTTT

GTTGAAAGAACATATGGTAGCTATTACATTGAAAATGTACACTAAGCATATGTCTACAACC

CTAATAGCTACTTCCAACACTTCTACACAACTGCTTCGACTCAGTCAATTTCTTTCACTGT

TCTTTGGCCCAAACTGCCATTCCAGCACCCCTACCCTAAAATGGAAATATTATTCCTTTTA

ATTGCTTTTGTTATTTTAATACTGCCAGGTAGCTGAAAGGGAACTTTTGAGGTGGTAACAT

CTCTTACTGATCCAAATATGTTTTTCTTCAGTTGGTAAAACTGGCTTAGCAGTTACCGTCA

TTATGTCTGGTTCTTCTCTACCAGCTTATTTTTAAAGGAGTTTTCGTAAGAAACATAACTC

CCCAACCTCTCCCTCTTCCTGTTCTTCCCCCAGCTCCCCTCACTAATTAAGTATTTCATTT

CCACTAGCCCATTATAATGTTGTTTATGATCTGATTTCTCTTTCTCTTTTTCATTTCCAAA
 
ATCATGAGTTTTCCCCTTTCTACCAATGGTCTACATCCTGACTGTATAGATATATGCTTTA

CATGTTTTTGTTTTATTATTTTAAAAATTTTATGTTAAGTTCAGGGGTACATGTGCAGATT

TGTTATACAGGTAGACTTATGTCATGGGGGTTTGTTATACAGATTATTTCATCACCCAGGT

ATTAAGCCTAGTACCCATTAGTTATTTTTCCTGATCCTCTCTCTCCTTTCACCCTCCACTC

TTCAAAAGGTCCCAGCCAGCATGTGTTGTTCCCTCTATGTGTCTGACCATATATTGAAAGG

GTATAGAAAAGTATTTGAAATTATGAAAGCCTAGTACTTATCTCTACTTTTCCCTTGCCCT

ATTAAATCAAAACGTACAGCAATAGAACCCAGAAATCTCTCATTGCTCCATAGGTAATACT

AATGTGTAACCACTAGCAAAACATAATTTCTTACTTTAAAAAAAATAGCTCCTGTTGTATG

TTTACCCTCTGCAAGTTACCTTACATGCCTTGCCTCATTTAATCCTCACAAAAAAACAGTA

AATATTATTATCCTAAAATTTTCTAGGATAGAAAACTGGAGCATCAATGAGTCAGAAAAAA

GAAGGAAAAACAAAAGTACAAATTTTAGAGCAAGGTCTTCAATCCATGATTGGAAGTCAAA

GGCTGACCCCTTTCTACTATGCTACACTACTCCTCACTCCACTCTATTATCTATGTTTACT

AGTATTTTTATTTCTAATTCTCATTATGTATTTGTGTGTATATGTAAAGTGACAAATCAGC

TGGAAAATCCACATTTACAAAAGGTGCTAATACCTCATATGGTAATGCAGTACTGTAAGAT

GTTTATGAATAAAAGGAATGTGGCCCTGACTGACGTGGAATAATTTAAATCTCAGTAGAGA

TAATGATGCTGCATCCCATACTTACTTTTGAGAATATGGTGGACTTTAAAATAACACTGCT

GCATAATAAATAAGGAGTGACTGCAATTAATGCATACCTCACTGCCTAAGGTTTCCCAAAA

GAGAAGGGAAAGCCTTCCTATTTCCAAGGTGGGAATGTTCTTCAACGCTCTCCTGACCTTT

CTCCAGTTTCCTCCTTTCTCACCTTTCTTTAAACCCTTTTCACTTTTTCTTCTCTTTTCAC

CTGCAAGTCTATTGACAGGTAAGTGAGAGAAGAGAAAAGAAAAAGCTAGTATTTATTAAGC

ACCTGCAATCTGTCAAAAATTTTCATTAGTGGTATATTATAACCCCCCTGAATTAGCTTTT

GCTATGTTATACACTAACATGCAATTGTCAAATTTCAGTGGCATACAAAAACAAAGATTGA

TTTCTAGGTCATGCCACATGTAGGCAGCTGGGTTCTGCTTCCCATTTCTAGACTCAGGCTG

AAGGCACAGTTGCCATTGGGAACATGCCATTTTTATGGAAAAGATAAAAACAAGCAACAAG

ATCACTCTTAAATATCTGCATGAATATAATATATGTCCTTTCTTTCTTACATTTTTGACAA
 
ATGCAAGTCAAAGTGAGCCCAATATCAGTGGAGTAGTAAATATTGACCTCCCATAGAAAGT

AGTGCTCTAGGCCAGGCGTGGTGGCTCACACCTGTAATCCCAGCACTTTGGGAGACCAAGG

CGGGCGGATCACGAGGTCAGCAGTTTGAGACCAGCCTGACCAACATGGTGAAACCCCGTCT

CTACTAAAAATACAAAAATCAGCCAGGCATGGTGGCGAGCGCCTGTAATCCCAGCTACTCA

GGAGGCTGAGGCAGGAGAATCACTTGAACCTGGGAGGCAGAGATTGCAGTGAGCCAAGATC

GCGCCATTGCACTCCAGCCTCGGCGACAGAGCGAGACTCCATCTCACACACATACACACAC

ACACACACACACACACACACACACACACACAAGTAGTGGTCTAACTCACAGGGCATTGGAT

GGTGCATGCATATATTTTTACAGCAAAATAGAAGTGAATCTTTATAAAAAAATATAATCTA

TCGCAATACCCTTTTTATATAGGGGAAACATGAGGCTCAGAGGTTAAATTAACTGTCCAAG

AGTAGTGCATCTAGTGGACGACTCATTTAAGATTGTAGTCAAGTTTTTTCTAATTCTACAG

CCACTGTTCTTGGATTTGCATAGCCAAGTTGAAGAGAGGGGAGACAGACTTAGTTCTCTCT

TCTATTATTTTTTAATCTTTCATTCCAAATATTTTCTAAAACATCACGTCACCTTAAGGAT

CTAGGAATAATCTTTGATTTAATCCTCTTCATTTTTTCATATTAAATAAAACTCTACATTG

TCTAATGCTTAATAATGATACAATTTTTTGAGCACTTAACTTGTGCCAAACACTATTTCAA

ACTCTCTTCACATACTGTTTTATTTAATTTTGAACCATAAGTTTTGTGGTAGGCATCATAT

CACTTTAAAGACAAAGGGCTACACATAGTGGCTCATCCATGTAATCCTAGAGCTTTGGGAG

GCCAAGGTAAGAGGATTGCTTGAGGCCGGGAGTTTGAGGCCAGCCTGGGCAATAATAGCAA

GGCCTTGTCTCTAGAAAAAATAAAAATTAGTAGGGCATGGTGACATGCCCCTGTAGTCCTA

GCTACTCAGGAGGCTGAGACAGGAGGATTGCTTGAGCCCAGGAGTTCAAAGTTGCAGTGAA

GTATGATTGTGTAACCTGGGCAACAAAGCAAGAACTTATCTCAATAAAAAAGGATAAAAAC

ATTGAGGAAAAAATCTCAATAACTTGCCTAAAATGACTGACTGGGAGTGAAGTAGAGCTGG

TTTTTAATCCATATCTGATGTTAACACTTGTGCTTCTATGCTCTGCATCATTCTGCTTTCT

AATGAAAATCTCTGTTTTAAATATATCCTACACTTCTCTCTTCTCGTCTCCAGCTTCTATT

GCCTATGTTTAGTTTTTATCTTGTTAAGCCTAAATTTCTGCTGTTGCTTTCTAGTTAGATT

TCACTCATGAAACCAACCTATATACCAAGACTAGACTAATTCTTAAACTCTGCCTTGATCA
 
CTTTTGTGTGCAGAAGACATTCATTGCTCTCCCTTCTTCCTACTTTCATCCTTCCCTCATA

TAAACTCCTTCATCTGATTTCCAAGGACACCCAGAGTGCAAATTATTCTTATTTCCCACTA

GTCCTCAAAGTGTACCATTTACCTGGTTGTCCCTTATTGATTCAACCTTGCTTTATTTACA

TTTTTCCCCTTACTGGAAACTAGATCAAGAACTTTACGGGAGCAAGGTCATGCATCATTTT

ATGAATATCTCTCCATATTGATTGTCATTATGCTAGCTTTGAAGTAGGAAGTAATTACTTA

CATAGGTAACCTTTACTTGGGTCCAAATATGGTGCTTTTCATCACATTGTTATGTGGACAT

TTACAGGTAACTAGGAATAAATGATCCATGAATTCATTTCTGGTTCTGCTGTAATGGGTCT

CCTGAATAGACAGCAGATTCTAAATTCTCCATATCTCATGGGGACATGTTGGGAAATTGAA

GAATGCCAGATTAACTGGCAGCAAAAGTGGGATTGATGTTGCATCACATCGCCCTCTCTAA

GCAACAATAAGAATAGCACTTGAAAGGTCAGGCATCTTTCAAACCTCAGGGTGAAAAGTTT

GCTTGAACACATGGACAAATAGAGGGGAACAACACACTTGGGGGCCTGCTAGAGGGTGGAG

GGTGGGAGGAGGGAGAGGATTAAAAAAATAACTAATGGATACTAGGTTTAATACCTGGGCG

ATAAAATAATTTGCACAACAAACTTCCATGACACAAGTTCACCTATGTAACAAACCTACAC

ATGTACCCTTGAACTTAAAAATTAAACAAAATAGAATAACATAATATAAAATAAATAACTG

AAAAAGAAAAAAGGAAAGAAAAATTTGCTTGGCTCCCAGAAAAACATATGAAGAGAAATTG

TCGTACATAGCGTTTGCATATACGTGTGTCTAGTAGAGGTAAGGATTATAAATTAGTACTT

ACATTTATCAACACACAATGTTTTCAGTGTGGTGTTCTCTTGCCTTCCATTGACTAAGCAA

AAATAAAGATCCTCAATGAACATCTTATGCCTGCACACATTTTATAAATCCTTGCCATTTA

ATCACATATATCCATGTTTTCCAATTTGTTTTCCTGGATTCTAAAATATATCCCCTGGAGA

AAGTAAATTTAGGTAATGTATGCCACTATATTATGTCAAAAAAACTGAAAATTACTTGCAA

CTAAAATGCATATATTACATCTAACCCAAAATTTTCTAAAGTCATTTACTTATGGGATCTT

TTGTTTGCCTAACCCATAAAAGTATTCTGTAGAACCAGTGTTTTATGGAACATAATTCTAA

AAACTCTGCCTCTTCTAAAAAAGACGTTGAATTTACTGTGTCCCCTTGAAAATAGAGAATG

ACAGATGGTTCTACTTGAGTAAAATGGCCTGAGAAAATCTTTCCCAGAAGGTGGTCTTTAG

GCAGACACCTGAAGAAAGAGAAGGAGTCTGTTCTAGGAAGAGGCAATAGCAAGGGTTGAGT
 
GCTCCAGCAGTAAAGAAAATGTTTAATGTGTTTGGCAAACTAAAAGGAAGTAAGAGAAAGA

GGAAGGAAGGAAAAGAGGCAAGGGGGGAGGAAAGAAGCCTCTTGGTGGCTGGAATGAAATG

GGCAAAGACGAGATTTAATATACCAAAGAGATTGCTAGAGTTTAGATCATGTGGGACTTCC

TAGACATGATAGAGGCTTTAGGATTTCATTTAAGTTCAGTGAGAGGCTATTTAAAGATTTG

AAGTAGGGGAAATTAATGATTTCATTTGGATGTTTAATAGATAATCCTGATTGCTTTCCTT

GTAGTCAGGCAGAAGTGAAAACAGGAAAAAAGTTGAGATTACTGGGGGTATTTATTGAAGC

ACCTATGCTGGAATATGATGATCACTTTAGCTAGTTCAGTGCAGTTTGCAATGAGGAAGAA

TAAAGATACCTATTTATGTAAAATCAACAAGACAAACTGGCTAATGGGATATACGATTGAA

AGAAGGGAAAAAATCTAGGATAATTTCCAGATTACCTGAATAATATAGATGGGCCATTTCA

TTTACTGAGATGAGAAACACAGTGAGAGAAGTAAGTATAGTAAGAGGAAACCTACATTTAG

TTTAGAACTTGTTAAATATGAGATGGTTATAAGATATGCAAGCAAAAATATCAAGAAGGTA

GCTTGAAATGAATCTGTAGTTTACTTAAAATCATTGGAAAACTGAGATCACTCAGTAAGAT

TATTAGGTTTCTTCTTACTTAAGGGGAGGGGCAGGACTGAATCTTATTAAAGGGAAGGGCC

AGGACTGAACTCCAGCCTTTAGAAGTTGAAGACAGCAGAAGAAGCCAGTGAAGAAGAATAT

TTGAAATGACCAAGTAGGCCTTAGCTGATCTTGAAATAATAATTTCAGTGGACTAGTGAAG

CAAAAACCTGTTTGGAAATGTAAAGTGAAATGGGTTGAGGATCAGAGACAATTATCAAGAC

AACTATTTCAAGACATTTGGCTATTGAAGGATAGTCAGAAAAGGTGGAAGGTAGTTGTCAT

ATTGACAGTTTAATGCAGTAGTGTGGGATCTTTTCAAGATGGCTTGAAAATGTACTAATGA

GAAAGGAGGGAGAACTAGAGGTAACAAAATGGTTCCTGAGTCGTCAGCTTTTAAGTGGTCT

TTGGGTAATGACTGCAAAGACATCAAATCTGGTGTATTTTCACACCACATTCAACAATACT

GAAAAATTACACGTGGATCACTTTTTAAAAAATCTCCACTGTCTATTAATAGTAAATGCTC

ACTAAATACTTTTTGAGGAAAGATAGGAATGAGAGAAGGAGGAGAAGGAGGAGAGAAAGAA

AAATGAGAAAAGGAGCCAAAAAGAAATCCCATATTTTATCCTTGTTTTCCCTTCTACTCTC

ACTACAATTGTGCATTCTTTCTGATGGAGGTTCCCCATTTCATAACTCAGTAACATATAAA

ACTTTCTGGATTGTCTCATCATTCGAAATGACTGTGAACACTCTCCAAAAGAGTGTTCTCT
 
TTCTTTTATTTTTTCCTAACTCTCCCCTGTTGTTTTCAATCAGCCTATGGAAAAAAAAAAA

AAATATATATATATATATATAAGCCAGCTAGGTAAAATTTTACACATTTTCCAAAGGGTAG

CCATAAGTGAGTTATTCAATGTCAAATGAAGACATCAAAACCTGTCTGGACAATCAGTGGT

AATGTCATTTATATATTTTTTCATTGAAAGTATTCCATACACTGAAACCTCCACATATGAA

ATATAAAGTCTTGAAATTTATAAATTATAGTACAAACTATTTTCATTATCTTTCATTTGTC

TTATTTATTACAAACTTGCCTAGTGCTTGTGTAATATAATTCAAGGTAAAAATTACTGTTC

AATTTTGTGAACTTATTTTTTTCCCATGAAACTCATTCTAGATACAGGCATTTTTTCCCCC

AAACTGACTCTTCTAAATGTTGCTTTAATTAAAATGAAATGAAGGTTTATAAATTTGCAGT

TTGTGATATGTTAAATCCCTGTTTCAAATTATATCTCAAGTATTCTGACATTTATTTCTAA

ATCATTACTTTGTCTCTGATCAACCATAACTTTTACAATGCATTTACTGTTTCATGTGAAT

TATATAAGCAGAGTTTCAGTCCCCATGAACTCTCAACTACTGTTGCACAAGGATTTTGGAA

AAAAAAAAAAAAGAAAAAAGAAACAGTAATGCTCACCTCTCCATAGCTCATTTGGGGAGAA

TATTACCTAAATTTGTTCCTAATGCAAGATTGAAATTAATCTTGATTTCCCATCAAATGTA

CAAGGATTTTACAATATAGTTCATAATCGTGTTTAATTTCTCCAGGGTACAAGTTAAGATA

AACAAGTCACTGACAGTGTTTATCTATAAATAGAGCAGATGGTCTGGGGCTGAGGTTAGTT

ATCAACTTTTCTGACTTTAATTTCATGGTTATAAAACGCTGTAAACCTCACTGTTTATAAA

AGGCAGGGAAGGAACTCCAGTGGGCTTCAGTAGCCAAGAAACCCTCAGTAACATTAATCTT

TCTAACTGATATTCGGTATCCTCCTAGGGTGCAATATTTAAGATTGTGGAGTGATTGCTAG

ACAAATTTAAATTTTCACTGGGATAATTAGCTAACATTTCTGTCACTGATCTATTATAACG

TTGTTAATGAGTTCCATGTGAGAGAAATTTAGTAAATGAGATGATTCTTTCTAAACTCTGC

TAGGTGAGTGTTTAACAATTAAGGCAGTTACATATCTAAAAAAAGCCTTTTTTCTTTCAAA

ATAATACTTTTTTCTTCTGCAAAGATGAACTGTAATACCCAACTAGACTAAATGTACTTAG

GGATAGAATTTCATAAGGAAAAACCATAGCATCATTTAAATAATTAATAATCATCTAATGT

ATATTGTATATACTTAGGCCATGAATCCAGCTACAGCTTTTTTTTAAAAAAGGCCATAGAA

CTTAAAAAGAAGTAAAACTATTGATTCTTCATATTCTGAATTGATTTAAATACACAATTTT
 
ATTGTGATCATTTATTTTGCTAAAGGTCTGTATGATCAATGTGATTAAAATTAATTTTTTT
CAAGGACCCCTTATTAAAAAAAAGTATTATTTAAAATTTCTCATTTTTCCCCTTGCAACTT
TATAGTGAATATAAAAGTAATTTTACAATCACAAAAGTAAAAAATAATTCACAAGTTCTAC
CTCTATTACTTTTACATTCTTAAGTGGTTTATTTTGCATTCTAACAAGGGGGAAGCTTTTT
TTTTTTTTTTTTCTGGAGTCAGAGTCTTGGTGTTTTCACCCAGGCTGGAGTGTAGTGGCTT
GATCACAGCTACGTCAATCTGGAATGCCTGGGCTCAAGTGATCCTCACACCTCAGCCTCCA
GAGAAGTTTGGACTACAGGTGTGCACCACCATGCCCAGCTAATCTTTAAACTTTTTAGAGA
TGGGGTCTTGCTATGTTGGCCAGGCTGGCTTCAAACTCCTGGGCTCAAGTGATCTTCCCAA
CTTAGCCTTCCAAAGTGCTGGTTTTACAGGCATCAGCCACATTGGAGGTTTCTTAAATAGA
TGGTAATCCAATATGCAAATTGGAGACTAAAACTATTTTAAAAAAATGCCGGCCGGGCGCG
GTGGCTCACGCCTGTAATCCCAGCACTTTGGGAGGCCGAGGCGGGTGGATCATGAGGTCAG
GAGATCGAGACCATCCTGGCTAACAAGGTGAAACCCCGTCTCTACTAAAAATACAAAAAAT
TAGCCGGGCGCAGTGGCGGGCGCCTGTAGTCCCAGCTACTCGGGAGGCTGAGGCAGGAGAA
TGGCGTGAACCCGGGAAGCGGAGCTTGCAGTGAGCCGAGATTGCGCCACTGCAGTCCGCAG
TCCGGCCTGGGCGACAGAGCGAGACTCCGTCTCAAAAAAAAAAAAAAAAAAAAAAAAAATG
CCTTAACTGTATTTGAGTTCAGACTCCCTGAGAATGAGTTTTCTAATGACAAAATCCTCAC
TTTTCTAGTCTTGTGTTGGGCTGCTGCTTTCTGACCCACTCTGGGAGGTTTACTACTGGTC
TGAAACTCCAATCTATCTCTCTACTGATTATTGTTAACACAGTGCTCAAATCTTGGCTCCC
AATAAGCTGTCTTCTGAATAGCAGCAGCCCTAGGTGGAGAGATAACTGAATGGAACATTTG
CTAATAAAGTCATGTACCTCGTAAGGACATTCTGGTCAATGACAGACAGACAGACTATACA
TCAGTGGTCCCATAAGATTATAATACCGTATTTTTTATAGTATCTTTTCTATGTTTATATG
CTTAAATATGCAAATATTTACCATTGCATTAAAGTTGCCTAAAGTATTTAGTACAATAGCA
CACACTGTGCATGTTTGTAACCTAAAAACAAAGGCTATATTATATAGCCTAGGCATGTGGT
AGGCTATACCACCCAGGTTGGTGTAAGTACACTCTATGATGTTCACACAAGCACAAAATCG
ACTAAGCATGCATTTTTCAGTGCATATCCCCATCATTAAGCAATGCATGACTGTAGATTTT
 
CTTATCTCCCTTCCTGCTCAGAGTCTGTTGTCACTAGTTTTCATCGGATGTAATATCCCAC
TGTTTGATACATTCATTTTTGTAAACAACTGTCTACTCATAGTTAGTGTCAGGTCTCAAAA
ATGCAGGAAGCATCTATAACAGAAGACCGCAACCTCTCTAAAATGGGAGCTTTATCTCATT
TTTCTTACTGCCTCAAAACCAGCTTTCATTACATAAATTGGTTACTAAACTATTGGTATGT
TTGACATTCATATTTGTGTACAATTACTCAAAACTCGGAGTGATTTCACTTTAAAGTTATT
GATAAAAATCACCTAAAAGAGATCTATCAGTTAATTGTAACAATTGCATCTTTCTGTTGTT
AAAGTAGAAAAGCCAGAGAAAAGATTGTCCTCACAGGGAAAGATTTGTTTTTATGAACTGG
TCACTCTAAAGCATGATTTCTAAAATTGTGAGCTATTGGAAACTGGTGGGAATGTATTTCT
CAGAAACATGGCCAAGAAGACTAGCACCTCAGAATGGCCAGGAATAGCTCTGAAGGGAGGC
AAAAATAGGTAACAGGAGAAACTGAAGTTTTAGCTAATAGAGAGAAAAAGAGAACAAGGGA
AAAAAGCAAAACTAGGTATTCCAGTATCTGAGTGCAGAGGTACATTAAAATAATCACTTTT
GTAAAGAAAGGATCTTTTTCAATATCCCAAAGATGGCCTAAAGTAACCATCTTTTATTTTT
CTTGTCTGTATATAAGATACAATGATTTATGTTATAAAATATAATCATTGTACTAATCTTG
GTTTGCAAAAAATAAAAGCTTATACACGGAAATTACCTGATTAAAAGGGGAAAAACAGGCT
GTTAAAATTTAAGAAAATTGTTCCTAGTTTTATTCAAATCTATCAGATTAAAATAATGTAA
TGGAAATAAATTGCTGCCTTAAAAGATAAAGCAAATTGTAATCCATAAAATTAAAATAATG
TATGATATGTAACTAATCTCATTTTTATGATTTTCTTTCAGATTATCCAACCCTGTCTAAT
GTCAAAGGCATGGCATTGTATACAAGAAAACATGCAAACAAAATCACTTTAACTGGATTTC
ATTCCCCCTTCAATGGTCAGTTAAAAATCAATCCTCTATAATATTTATATTATTTATTTCC
TCTAAAAATCCAGTGACTATTATTTCTTAATCTTTTTCTTTTTCTAGGAGAAGTAATCTAT
GCTGCCATGTGCATGACAGTCACCTGTGGAATCCTTCTGTTGGTTATGGTCAAGCTTAGGT
AACTGAATATAAATTAGAAGGAAAGGTCTGAAAGGGAGTCACTTAGCTGTGTTAAAGCATG
TTGCACAATGGCTGAGCTTACAAAGAGCCTGAGAATGAAAGCTTCTCTGTTAAGACATATT
AAATCAAGTTTTATGTGGCATAGGTCCATGGAAACGTTACTTTCAGTCTTCCTACATAAGG
AAAATGTGATCATAGGAGATTTTTTATGTTGCATTTCATAAGCACATATGCATTGTATGTT
 
CCTTCCAATTAAACTCTTATCCATCTTTCCCCTTGGCTTCCCTACCAAATCCAAATTGAAC

TTTGCTGCATTTGTTATTCAAATCATTTGCATGATTGATAGGCTCACATAACTGACCTATT

GTTTACTGCATACAGTTTGTATTTACCTGAAGATGTATTCTTCACTCTAATATCTTATATG

CTAATACATTTTATTCATTTTTAATAGGCAATTATTGAAAAAGACATTTCATAAAGCCTCA

CAGAGTGCCCCTAGAAAGACATTACCATTGCCTGACATTAATTTATTCTCCAAATCTTGGC

TGCTTTTATTGCTGGAATGTAGTAATGCTCCAGCAGGCATGTGAGACATTGAGATGAGATA

TACATCAACCTCTAGATTTCTAAACGTCTAGTGTTTAAAATTTTTCCACTTATTTAGCATT

TACAACTATACATAAAATAGTGTAGACTTGCCTTTAATTAAAAAAAACTCACATTTAAACA

TCAATCATGTTCATTTGCTTATTCTTTACACATATGGGTACATGTCTATAAAATTTGAGTA

GCATAAATGTTAATGAAGAAATATACATAAGTGTGTATACAGCCATATATACATGTAAGTA

CATACACAAATTATATCAGTAAGATTGGGCCTTCAGAGGAACAGAAGTATTCACTGGAGAT

TTACCTTATGACATCTGACACCTAGAAAGATTAATTCAGTCAACTCTACAATACATATAAA

CATATCTTGCTCCTCCAGAGATCTCAGACTTAAAAAGGCCTTGACAATTAGGCAAATAAAA

GACTGGCATGAGAAGGTGTACGGTGATACCAAGGGGTCAGGTACATACGGACATCCTAATT

TATTTATTTTTAAATGCAATGCCAGCTAAACAAAATGTATCCACTGTTTGGACCCAATCCC

TCTAAGCATTTAATTTGTTGTTAACTAAAATCAGTACCAAAACACACTGCTTTTCTCCAAG

GCCTTAAAAGTTACATTTGTATGAGAAGACATTCCGTGGGTTCTAAGATATGGGTTGAAAG

CCAAATAATTAGAGTCCATTGCTAAGAGGGAAAAATAGCATTTGGAATTTATTAAAGTTTA

CTTTATTTGGTTTTAAGTACTTGAGAACACTGTTTAGTTATTGTTCCTGCCTAGATATTCC

ATGACAAACATTGTTACAACAAAATATGGACAGTTTTCTGGCCCGGCAAAAACATCTTACT

TTAATGGTTCTCAATCTTTACTTCTTAACTACATACAGAATGTATAAAATTTACACCTGTT

CCATGATCCCACTGGTTTTTAGATACTACTGTTTTGCTACAGATACAAGATATAGACTACT

CATAATTCCCTATGTATTTGCTGTAACTTCGTCTTTTTTCTCACCTGCATAGAAGGCATAT

CCCTTTTTTGGATGGCAGTGGGGGCATTATTATTATGGACAAATGTTTTGAATTTATTCTA

ATGTGTATTTTAAAGCAAATTATTCATACTATTAAATATATTTGTATTTAGTTACAAATTA
 
TTTTCATATAATTTAAAATTTATCATAATGTGTAATCGGAAAACTCCAGGCCCAGGATAAA
AGAGACAAAATTTAAAGCCATCATAAAAAGAACAAACTGAAACAAAAGCATCATTGGCAAC
AAAACCTGTGTTGCCCCACACCTGGAGATAAATAATGCTCCCTAAAGAAAGAAGAATGATT
GCTATTTCAGACATTGTTCACTCACCTGATGGTCAAGCCCAACCCATGTGAATACCTGTCT
TCTGTAAGGAATAAGATAGTTGCATTTGTTTCATGTTATGCTATATTCAAATAGCCCAGAG
CCTTCAAAGAAGTTTGCAGAGAAACAACTAAAAATTTAAATATTTACAAAAAGAAAGGACT
TTTTAAAAAATGATTACTTGGACATCATAGTTAAAGGTGTGATGCTCCCTAGAAAGAAAAT
ATGATATTTTACTATTGGGACTTTACATTCTGGAACTTGATTAGTTTTTTCCTCCAGGTGC
AGACTCCAGCAAAAATGCAAACACACACAAGCACATTTGTGAAATCCTTAAAATGGCATGT
TGTTAACTTTTACTTTTATTTTATACAGAACTTCCAGAATATCAAGTAAAGCAAGAGATCC
TTCATCGATCCAAATACCTGGAGAACTCTGATTCATTAGGAGTCATGGACCTATAACAATC
ACTCTTTTCTCTGCTTTTCTTCTTTCCTCTTTTCTTCTCTCCTCTCCTCTCCTCTCTTCTC
CTCTCCTCCCCTCCCCTCTCTGTTTCTTTTTCTTTTTCTTTTCTTTTTTGTGGCGGAGTTT
TGCTCTTGTTGCCCAGGCTGCAGTACAATGGCTCAATCTCGGTTCACTGCAACCTCTGCCT
CCAAGGTTCAAGTGATTTTCCTGCCTCAGCCTTCCCGAGTAGCTGGGATTACAGGTACCCG
CCACCACGCCCAGCTAATTTTTTTGTATTTTTAGTAGAGATGGGGTTTTGCCAAATTGGCC
AGGGTGGTCTCAAACTCCTGACCTCAGGTGATCCACCCACCTCGGCCTCCCAAAGTGCTGG
GATTACAGGCGTGAGCAACCACGTCAAGACAACAATCACTTTCTTTAAAGCAAATCCTACA
GCTGGTCAACACCCTATTCCATCTGTCATCGAGAAAGAAAATGTTAAAATAGACTTAAAAA
TATTGCTTTGTTACATATAATAATATGGCATGATGATGTTATTTTTTTCTTAATACTCAAG
AAAAAATATATGGTGGTATCTTTTACAACACTGGAACAGAAATAAAGTTTCCCTTGAAGGC
 SEQ ID NO: 2 (GenBank Homo sapiens GFRAL mRNA, NM 207410.2)

TTATTCTGGACAGTTACTCTTAAGAAAGTTGTCAGAAGAAACGCATCTGCCTTTTT

TTCCAGGTGAACTGCCGTGAGTTGTCCAGCATGATAGTGTTTATTTTCTTGGCTATGGGGT

TAAGCTTGGAAAATGAATACACTTCCCAAACCAATAATTGCACATATTTAAGAGAGCAATG

CTTACGTGATGCAAATGGATGTAAACATGCTTGGAGAGTAATGGAAGATGCCTGCAATGAT

TCAGATCCAGGTGACCCCTGCAAGATGAGGAATTCATCATACTGTAACCTGAGTATCCAGT

ACTTAGTGGAAAGCAATTTCCAATTTAAAGAGTGTCTTTGCACTGATGACTTCTATTGTAC

TGTGAACAAACTGCTTGGAAAAAAATGTATCAATAAATCAGATAACGTGAAAGAGGATAAA

TTCAAATGGAATCTAACTACACGTTCCCATCATGGATTCAAAGGGATGTGGTCCTGTTTGG

AAGTGGCAGAGGCATGTGTAGGGGATGTGGTCTGTAATGCACAGTTGGCCTCTTACCTTAA

AGCTTGCTCAGCAAATGGAAATCCGTGTGATCTGAAACAGTGCCAAGCAGCCATACGGTTC

TTCTATCAAAATATACCTTTTAACATTGCCCAGATGTTGGCTTTTTGTGACTGTGCTCAAT

CTGATATACCTTGTCAGCAGTCCAAAGAAGCTCTTCACAGCAAGACATGTGCAGTGAACAT

GGTTCCACCCCCTACTTGCCTCAGTGTAATTCGCAGCTGCCAAAATGATGAATTATGCAGG

AGGCACTATAGAACATTTCAGTCAAAATGCTGGCAGCGTGTGACTAGAAAGTGCCATGAAG

ATGAGAATTGCATTAGCACCTTAAGCAAACAGGACCTCACTTGTTCAGGAAGTGATGACTG

CAAAGCTGCTTACATAGATATCCTTGGGACGGTCCTTCAAGTGCAATGTACCTGTAGGACC

ATTACACAAAGTGAGGAATCTTTGTGTAAGATTTTCCAGCACATGCTTCATAGAAAATCAT

GTTTCAATTATCCAACCCTGTCTAATGTCAAAGGCATGGCATTGTATACAAGAAAACATGC

AAACAAAATCACTTTAACTGGATTTCATTCCCCCTTCAATGGAGAAGTAATCTATGCTGCC

ATGTGCATGACAGTCACCTGTGGAATCCTTCTGTTGGTTATGGTCAAGCTTAGAACTTCCA

GAATATCAAGTAAAGCAAGAGATCCTTCATCGATCCAAATACCTGGAGAACTCTGATTCAT

TAGGAGTCATGGACCTATAACAATCACTCTTTTCTCTGCTTTTCTTCTTTCCTCTTTTCTT

CTCTCCTCTCCTCTCCTCTCTTCTCCTCTCCTCCCCTCCCCTCTCTGTTTCTTTTTCTTTT

TCTTTTCTTTTTTGTGGCGGAGTTTTGCTCTTGTTGCCCAGGCTGCAGTACAATGGCTCAA

TCTCGGTTCACTGCAACCTCTGCCTCCAAGGTTCAAGTGATTTTCCTGCCTCAGCCTTCCC

GAGTAGCTGGGATTACAGGTACCCGCCACCACGCCCAGCTAATTTTTTTGTATTTTTAGTA

GAGATGGGGTTTTGCCAAATTGGCCAGGGTGGTCTCAAACTCCTGACCTCAGGTGATCCAC

CCACCTCGGCCTCCCAAAGTGCTGGGATTACAGGCGTGAGCAACCACGTCAAGACAACAAT

CACTTTCTTTAAAGCAAATCCTACAGCTGGTCAACACCCTATTCCATCTGTCATCGAGAAA

GAAAATGTTAAAATAGACTTAAAAATATTGCTTTGTTACATATAATAATATGGCATGATGA

TGTTATTTTTTTCTTAATACTCAAGAAAAAATATATGGTGGTATCTTTTACAACACTGGAA

CAGAAATAAAGTTTCCCTTGAAGGC

**[Table 1]**

| GFRAL gene targets and antisense oligonucleotide sequences | | | |
|---|---|---|---|
| **SEQ ID NO.** | **Target sequence (5' to 3')** | **SEQ ID NO.** | **Antisense sequence (5' to 3')** |
| 3 | UGUAGGGGAUGUGGUCUGUA | 19 | TACAGACCACATCCCCTACA |
| 4 | UGGUCUGUAAUGCACAGUUG | 20 | CAACTGTGCATTACAGACCA |
| 5 | GUCUGUAAUGCACAGUUGGC | 21 | GCCAACTGTGCATTACAGAC |
| 6 | CUUACCUUAAAGCUUGCUCA | 22 | TGAGCAAGCTTTAAGGTAAG |
| 7 | GGUUCUUCUAUCAAAAUAU | 23 | ATATTTTGATAGAAGAACC |
| 8 | GGUUCUUCUAUCAAAAUAUACC | 24 | GGTATATTTTGATAGAAGAACC |
| 9 | UUCUUCUAUCAAAAUAUACCUU | 25 | AAGGTATATTTTGATAGAAGAA |
| 10 | CCCAGAUGUUGGCUUUUUGU | 26 | ACAAAAAGCCAACATCTGGG |
| 11 | CCAGAUGUUGGCUUUUUGUG | 27 | CACAAAAAGCCAACATCTGG |
| 12 | CAGAUGUUGGCUUUUUGUGA | 28 | TCACAAAAAGCCAACATCTG |
| 13 | AGAUGUUGGCUUUUUGUGAC | 29 | GTCACAAAAAGCCAACATCT |
| 14 | AAAUGAUGAAUUAUGCAGGA | 30 | TCCTGCATAATTCATCATTT |
| 15 | GAAAGUGCCAUGAAGAUGAG | 31 | CTCATCTTCATGGCACTTTC |
| 16 | AAAGUGCCAUGAAGAUGAGA | 32 | TCTCATCTTCATGGCACTTT |
| 17 | AUUUUCCAGCACAUGCUUCA | 33 | TGAAGCATGTGCTGGAAAAT |
| 18 | UUUUCCAGCACAUGCUUCAU | 34 | ATGAAGCATGTGCTGGAAAA |

**[Table 2]**

| Antisense oligonucleotide sequences including chemical modification | | |
|---|---|---|
| **SEQ ID NO.** | **ASO ID** | **ASO sequence (5' to 3')** |
| 19 | A422 | T*ACAG*5*6*6*5*6*5*8*6*6*6*CTAC*A |
| 20 | A423 | C*AACT*7*8*7*6*5*8*8*5*6*5*GACC*A |
| 21 | A424 | G*CCAA*6*8*7*8*7*6*5*8*8*5*CAGA*C |
| 22 | A425 | T*GAGC*5*5*7*6*8*8*8*5*5*7*GTAA*G |
| 23 | A426 | A*TAT*8*8*8*7*5*8*5*7*5*5*GAAC*C |
| 24 | A427 | G*GTATA*8*8*8*8*7*5*8*5*7*5*AGAAC*C |
| 25 | A428 | A*AGGTA*8*5*8*8*8*8*7*5*8*5*GAAGA*A |
| 26 | A429 | A*CAAA*5*5*7*6*6*5*5*6*5*8*CTGG*G |
| 27 | A430 | C*ACAA*5*5*5*7*6*6*5*5*6*5*TCTG*G |
| 28 | A431 | T*CACA*5*5*5*5*7*6*6*5*5*6*ATCT*G |
| 29 | A432 | G*TCAC*5*5*5*5*5*7*6*6*5*5*CATC*T |
| 30 | A433 | T*CCTG*6*5*8*5*5*8*8*6*5*8*CATT*T |
| 31 | A434 | C*TCAT*6*8*8*6*5*8*7*7*6*5*CTTT*C |
| 32 | A435 | T*CTCA*8*6*8*8*6*5*8*7*7*6*ACTT*T |
| 33 | A436 | T*GAAG*6*5*8*7*8*7*6*8*7*7*AAAA*T |
| 34 | A437 | A*TGAA*7*6*5*8*7*8*7*6*8*7*GAAA*A |
| A=2'MOE-A, C=2'MOE-5'-methyl-C, G=2'MOE-G, T=2'MOE-T, 5=DNA-A, 6=DNA-5'-methyl-C, 7=DNA-G, 8=DNA-T, *=PS | | |

### Experimental Example 1: Primary Screening

Antisense oligonucleotide candidates targeting GFRAL mRNA were selected as described in Table 1 above and then were produced by Chemgenes (USA). The antisense oligonucleotides were transfected into HEK293 cells overexpressing GFRAL and RET cDNA using Lipofectamine (Invitrogen, USA) reagent at a concentration of 200 nM. After 24 hours, RNA was purified by treating with an RNA extraction kit (Bioneer, Korea) and DNA decomposition enzyme (NEB, USA), and reverse transcription was performed using RT-Premix (Applied Biosystems, USA). Then, qRT-PCR was performed to measure the level of GFRAL gene expression. The following primer and probe sequences were used for qRT-PCR:
GFRAL: predesigned qPCR Assay (Hs.PT.58.21179605, IDT, USA)
GAPDH:
forward: 5'-GGTGTGAACCATGAGAAGTATGA-3' (SEQ ID NO: 35)
reverse: 5'-GAGTCCTTCCACGATACCAAAG-3' (SEQ ID NO: 36)
probe: 5'-AGATCATCAGCAATGCCTCCTGCA-3' (SEQ ID NO: 37)

As can be seen from FIG. 1, the result of the first screening showed that GFRAL gene expression was inhibited by the antisense oligonucleotide.

### Experimental Example 2: Secondary Screening

100 nM of 16 antisense oligonucleotides were administered to HEK293 cell lines expressing GFRAL and RET cDNA using Lipofectamine (Invitrogen, USA) reagent and whether or not the GFRAL gene could be knocked down was repeatedly observed. First, the cells were transfected with ASO for 24 hours and were lysed with lysis and RT kits, and then reverse transcription was performed. Then, qRT-PCR was performed using Taqman probe and qRT-PCR premix (Applied Biosystems, USA) to measure the GFRAL gene expression level. As can be seen from FIG. 2a, the result showed that 8 antisense oligonucleotides had a knockdown effect at a concentration of 100 nM.

In addition, after the cells were transfected with ASO for 48 hours, western blot assay was performed to confirm GFRAL gene protein expression. As can be seen from FIG. 2b, the result showed that protein expression was reduced in 8 antisense oligonucleotide candidates. Based on the results of FIGS. 1 and 2, a total of 8 candidates including A426, A427, A428, A429, A430, A433, A436, and A437 were selected.

### Experimental Example 3: Tertiary screening

Tertiary screening was conducted using the same method as in Experimental Examples 1 and 2 by administering 50 nM of 8 antisense oligonucleotides selected in Experimental Examples 1 and 2. As can be seen from FIG. 3a, the result showed that all 8 antisense oligonucleotides exhibited excellent knockdown efficiency at a concentration of 50 nM. After the cells were transfected with ASO for 48 hours, cytotoxicity was measured using the Quantimax wst-8 cell viability assay kit (Abcam, UK), and GFRAL gene protein expression was confirmed by a western blot assay under the same conditions. As can be seen from FIG. 3b, the result showed that none of the eight antisense oligonucleotide candidates decreased cell viability.

In addition, as can be seen from FIGS. 4a and 4b, protein expression inhibition was repeatedly confirmed in A427, A428, A429, and A430. Therefore, based on the results of FIG. 1, FIG. 2, FIG. 3, and FIG. 4, A427, A428, A429, and A430 were selected as the final candidates.

### Experimental Example 4: In Vivo Toxicity Test

Based on the results of cell experiment of Experimental Examples 1, 2, and 3, animal toxicity tests were performed three times to verify the stability of four antisense oligonucleotide candidates.

2 µl (20 µg) of the antisense oligonucleotide of Example was administered to the left hemisphere of the brain of 0- to 2-day old C57BL/6J wild-type mice by intracerebroventricular (ICV) injection and the survival rate was measured after 7 days (FIG. 5a).

As can be seen from FIG. 5b, none of the 12 experimental animals used as negative controls, PBS and A114, died, and 11 out of 12 animals that were administered A386, which was used as a toxicity positive control, died within 6 hours, which means that A386 has significant toxicity. Of the 7 animals that were administered A427 as an experimental group, one died, and all animals that were administered A428, A429, and A430 survived, which means that A428, A429, and A430 are relatively safe antisense oligonucleotide candidates when administered *in vivo.*

### Experimental Example 5: Analysis of GFRAL protein expression regulation in vivo

The following animal experiments were performed to verify the pharmacological effects of antisense oligonucleotide candidates.

8.5 µl (500 µg) of antisense oligonucleotides (A114, A427) were administered to 8-week-old male C57BL/6N wild-type mice by intracerebroventricular (ICV) injection. After 1 week, the brain tissue was primarily fixed by sequentially perfusing saline (sodium chloride, perfusion agent; JW Pharmaceutical corporation, Korea) and 4% paraformaldehyde (FUJIFILM, Japan) through the aorta. The brain tissue was extracted and secondarily fixed in the same fixative for 24 hours. The brain tissue was washed twice with phosphate buffer containing 30% sucrose at 4°C, and then the brain tissue was frozen and cut into 30 µm sections in a cryostat. The sections were blocked with 0.1% PBST (triton-x100) containing 5% donkey serum (Jackson immunoresearch, USA) for 1 hour and then were reacted with purified primary antibodies for 24 hours. The sections were reacted with secondary antibodies for 2 hours and the expression level of GFRAL gene protein was measured using immunohistochemical (IHC) fluorescence staining.

As can be seen from FIG. 6, the result showed that, when A427 antisense oligonucleotide was administered, the fluorescence signal intensity of GFRAL gene protein decreased, and the proportion of GFRAL gene-expressing neurons with respect to the total neuron decreased.

### Experimental Example 6: Analysis of ASO distribution in vivo

The following animal experiments were performed to verify the pharmacological effects of antisense oligonucleotide candidates.

8.5 µl (500 µg) of antisense oligonucleotides (A114, A427, A428) were administered to 8-week-old male C57BL/6N wild-type mice by intracerebroventricular (ICV) injection. After 2 weeks, the brain tissue was primarily fixed by sequentially perfusing saline (sodium chloride, perfusion agent) and 4% paraformaldehyde through the aorta. The brain tissue was extracted and secondarily fixed in the same fixative for 24 hours. The brain tissue was washed twice with phosphate buffer containing 30% sucrose at 4°C, and then was frozen and cut into 10 µm sections in a cryostat. The ASO probe was stained with the RNAscope^{™} Plus smRNA-RNA HD Reagent kit (ACD bio, USA), and the in vivo distribution of the ASO probe was observed using Fluorescence in situ hybridization (FISH).

As can be seen from FIGS. 7a and 7b, the result showed that A427 was distributed in the hindbrain including the AP (area postrema) and NTS (nucleus tractus solitarius).

### Experimental Example 7: Analysis of in vivo GFRAL RNA expression regulation

The following animal experiments were performed to verify the pharmacological effects of antisense oligonucleotide candidates.

8.5 µl (500 µg) of antisense oligonucleotides (A114, A427, A428, A429, A430) were administered to 8-week-old male C57BL/6N wild-type mice by intracerebroventricular (ICV) injection. After 2 weeks, the brain tissue was primarily fixed by sequentially perfusing saline (sodium chloride, perfusion agent) and 4% paraformaldehyde through the aorta. The brain tissue was extracted and secondarily fixed in the same fixative for 24 hours. The brain tissue was washed twice with phosphate buffer containing 30% sucrose at 4°C and then was frozen and cut into 10 µm sections in a cryostat. The GFRAL RNA was stained with RNAscope^{™} Multiplex Fluorescent Reagent Kit v2 Kit (ACD bio, USA) and the change in GFRAL RNA expression level was measured using Fluorescence in situ hybridization (FISH).

As can be seen from FIGS. 8a and 8b, the result showed that, when A427, A428, A429, and A430 antisense oligonucleotides were administered, the fluorescence signal intensity of GFRAL RNA decreased and the proportion of GFRAL gene-expressing neurons with respect to the total neurons decreased.

### Experimental Example 8: Confirmation of efficacy of ASO in cancer cachexia model

Colon cancer cells (MC38) were injected into 8-week-old male C57BL/6J wild-type mice by intraperitoneal injection (IP injection). After 2 weeks, 500 µg of antisense oligonucleotides (A427, A430) was administered thereto by intracerebroventricular injection. One week after antisense oligonucleotide administration, the serum concentration of GDF15, which has diagnostic utility in cancer cachexia, was measured using a Quantikine ELISA kit (R&D Systems, USA) (FIG. 9a).

As can be seen from FIG. 9b, the serum concentration of GDF15 in the group administered MC38 increased by more than 1000 times, which supports that the cancer cachexia model was well constructed.

As can be seen from FIG. 10a, in the cancer cachexia animal model, the group administered A427 had an increase in the weight of muscle tissue (gastrocnemius) compared to the negative control group (PBS). In addition, the expression of Atrogin-1 and MuRF1 proteins, which are major molecular markers of muscular dystrophy, was measured in the muscle tissue (gastrocnemius) using western bolt assay. It can be seen that the protein expression of Atrogin-1 was significantly reduced by ASO (FIG. 10b).

### INDUSTRIAL APPLICABILITY

In the present invention, antisense oligonucleotide candidates that regulate GFRAL expression were discovered, and it was confirmed that the candidates effectively inhibit GFRAL expression *in vivo* and have an improvement effect in a cancer cachexia animal model. Therefore, the antisense oligonucleotide of the present invention is expected to have a remarkably high applicability as a therapeutic agent for obesity or cancer cachexia by inhibiting GFRAL expression.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### SEQUENCE LISTING FREE TEXT

An electronic file is attached.

## Claims

1. An oligomer capable of hybridizing with at least 13 consecutive nucleobases in an whole pre-mRNA of human GFRAL (GDNF family receptor alpha like) gene represented by SEQ ID NO: 1 through Watson-Crick base pairing A:T or G:C or wobble base pairing G:U, I:A, I:C or I:U, and having a length of 13 to 35 nt.

2. The oligomer according to claim 1, wherein the oligomer is capable of hybridizing with at least 13 consecutive nucleobases comprised in any one nucleic acid sequence selected from the group consisting of SEQ ID NO: 3 to SEQ ID NO: 18 through Watson-Crick base pairing A:T or G:C or wobble pairing G:U, I:A, I:C or I:U, and has a length of 13 to 35 nt.

3. The oligomer according to claim 1, wherein the oligomer is capable of hybridizing with at least 13 consecutive nucleobases comprised in any one nucleic acid sequence selected from the group consisting of SEQ ID NO: 8 to SEQ ID NO: 11, SEQ ID NO: 14, SEQ ID NO: 17, and SEQ ID NO: 18 through Watson-Crick base pairing A:T or G:C or wobble base pairing G:U, I:A, I:C, or I:U, and has a length of 13 to 35 nt.

4. The oligomer according to claim 1, wherein the oligomer is capable of hybridizing with at least 13 consecutive nucleobases comprised in any one nucleic acid sequence selected from the group consisting of SEQ ID NO: 8 to SEQ ID NO: 11 through Watson-Crick base pairing A:T or G:C, or wobble pairing G:U, I:A, I:C or I:U, and has a length of 13 to 35 nt.

5. The oligomer according to claim 1, wherein the oligomer is capable of hybridizing with at least 13 consecutive nucleobases comprised in the nucleic acid sequence of SEQ ID NO: 8 or SEQ ID NO: 11 through Watson-Crick base pairing A:T or G:C, or wobble pairing G:U, I:A, I:C or I:U, and has a length of 13 to 35 nt.

6. The oligomer according to claim 1, wherein the oligomer comprises a chemical modification of a modified internucleoside linker, a modified nucleobase, or a modified nucleoside.

7. The oligomer according to claim 6, wherein the modified nucleobase is 5'-methyl-cytosine.

8. The oligomer according to claim 6, wherein the modified nucleoside is a nucleoside comprising a sugar moiety into which one or more substituents selected from the group consisting of 2'-O-methyl, 2'-O-methoxy, 2'-O-methoxyethyl, 2'-amino, 2'-allyl, 2'-fluoro, 2'-arabino-fluoro, 2'-OCH₂C (=O) -NHCH₃ (NMA), 2'-O-benzyl, 2'-O-methyl-4-pyridine, 4'-O-methyl, 5'-methyl, 5'-vinyl, and 5'-methoxy are introduced; or a bicyclic nucleoside comprising one or more modified nucleosides of LNA (locked nucleic acid) or cEt (constrained ethyl).

9. The oligomer according to claim 6, wherein the modified internucleoside linker comprises at least one modified internucleoside linker selected from the group consisting of phosphorothioate, phosphorodithioate, phosphotriester, phosphoramidate, mesyl phosphoramidate, methylphosphonate, methoxypropyl-phosphonate, and boranophosphate.

10. The oligomer according to claim 6, wherein the oligomer comprises a gap segment comprising linked deoxynucleosides, a 5' wing segment comprising linked nucleosides, and a 3' wing segment comprising linked nucleosides,
wherein the gap segment is disposed between the 5' wing segment and the 3' wing segment, and the nucleoside of each wing segment comprises a modified sugar moiety or sugar surrogate.

11. The oligomer according to claim 10, wherein the oligomer comprises: a gap segment comprising 8 to 10 linked deoxynucleosides; a 5' wing segment comprising 3 to 7 linked nucleosides; and a 3' wing segment comprising 3 to 7 linked nucleosides,
wherein each nucleoside of each wing segment comprises a modified sugar moiety or sugar surrogate.

12. The oligomer according to claim 1, wherein the oligomer comprises a structure of the following Formula (I) or Formula (II) :
G*GTATA*8*8*8*8*7*5*8*5*7*5*5*AGAAC*C Formula (I);
C*ACAA*5*5*5*7*6*6*5*5*6*5*TCTG*G Formula (II),
wherein A represents 2'MOE-A, C represents 2'MOE-5'-methyl-C, G represents 2'MOE-G, T represents 2'MOE-T, 5 represents DNA-A, 6 represents DNA-5'-methyl-C, 7 represents DNA-G, 8 represents DNA-T, * represents PS (phosphorothioate), and 2'-MOE represents 2'-O-methoxyethyl.

13. A pharmaceutical composition for preventing or treating a disease related to activation of GFRAL due to an increase in GDF15 comprising the oligomer according to any one of claims 1 to 12.

14. The pharmaceutical composition according to claim 13, wherein the disease related to activation of GFRAL due to an increase in GDF15 is obesity, diabetes, anorexia or cachexia.

15. The pharmaceutical composition according to claim 13, wherein the disease related to activation of GFRAL due to an increase in GDF15 is cancer cachexia.
